# EUROPEAN PATENT APPLICATION

(11) **EP 4 796 530 A1**
(43) Date of publication of application: **26.08.2026**
(21) Application number: 24896771.3
(22) Date of filing: 29.11.2024
(51) Int. Cl.: C07C 311/60, C07C 311/59, A61P 29/00, A61P 3/00, A61P 9/00, A61P 35/00, A61K 31/64

(54) **SULFONYLUREA DERIVATIVE AND USE THEREOF**

(30) Priority: 01.12.2023 CN 202311640461
(71) Applicant: Biocells (Beijing) Biotech Co., Ltd., Beijing 100070 (CN)
(72) Inventor: HAN, Huamin, Beijing 100070 (CN); JI, Qi, Beijing 100070 (CN)
(74) Representative: WSL Patentanwälte Partnerschaft mbB
(86) International application number: PCT/CN2024/135909
(87) International publication number: WO 2025/113692

(57) **Abstract**

The present application relates to a sulfonylurea derivative or a pharmaceutically acceptable salt thereof and a use thereof. In particular, the present application relates to the use of the sulfonylurea derivative or the pharmaceutically acceptable salt thereof as an NLRP3 inflammasome inhibitor, wherein the structure of the sulfonylurea derivative is represented by formula I, wherein the definition of each substituent is detailed in the description.

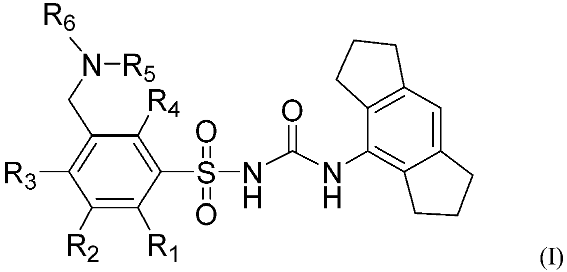

## Description

This application claims priority to Chinese patent application filed on December 1, 2023, with Application No. 202311640461.0 and invention title "Sulfonylurea Derivative and Use Thereof", the contents of which should be construed as being incorporated herein by reference.

### Technical Field

This application relates to, but is not limited to, the field of medicine, and in particular to a sulfonylurea derivative or a pharmaceutically acceptable salt thereof, and use thereof.

### Background

Nucleotide-binding oligomerization domain-like receptor protein 3 (NLRP3) inflammasome is a multimeric protein complex, which is capable of activating cysteine asparate protease-1 (caspase-1), thereby further inducing maturation of interleukin (IL)-1β and IL-18.

An NLRP3 inhibitor directly targets NLRP3 and interacts directly with NACHT or PYD domain of NLRP3, resulting in potential effects including the inhibition of enzymatic activity of NLRP3 and the inhibition of interaction between NLRP3s, which in turn affects the NLRP3 inflammasome assembly process (i.e. activation process).

Initially, the sulfonylurea compound, Glibenclamide, was found to be a potent NLRP3 inhibitor during phenotypic screening of a library of diarylsulfonylureas against interleukin 1β (IL-1β) secreted by human monocytes (J. Pharmacol. Experimental Therapeutics 2001, 299 (1), 187-197.), albeit with very weak activity. These compounds were then further optimized and their mechanism was initially studied, with the initial assumption that they act by inhibiting GST omega 1-1. Further work found that these compounds act through NLRP3 inhibition (J. Biol. Chem. 2003, 278, 16567-16578.). One of the Glibenclamide derivatives, CRID3 or CP456,773, was renamed MCC950 and was found to be a potent inhibitor of NLRP3 inflammasome (Nat. Med. 2015, 21 (3), 248-255.). MCC950 was tested clinically for rheumatoid arthritis, which, however, was terminated likely due to elevated serum liver enzyme levels in the patient. MCC950 acts by binding to the Walker B motif in the NACHT domain of NLRP3 and blocking NLRP3-mediated ATP hydrolysis. Since then, MCC950 has been used as a tool compound and applied extensively in a range of in vitro and in vivo studies involving NLRP3-related diseases. This tool compound, used together with a series of target validation techniques, has demonstrated that NLRP3 exhibits significant activity in many diseases, including cryopyrin associated periodic syndrome (CAPS), inflammatory bowel disease (IBD), nonalcoholic steatohepatitis (NASH), gout, multiple sclerosis, stroke, Alzheimer's disease, and Parkinson's disease.

MCC950 is a compound with clear mechanism of action, high cellular activity, high oral bioavailability and blood drug concentration, and demonstrated efficacy in animal models. However, MCC950 cannot effectively penetrate the blood-brain barrier (BBB), and therefore has limited efficacy for various diseases (such as multiple sclerosis, stroke, Alzheimer's disease, and Parkinson's disease) that require BBB penetration to exert therapeutic effects. Therefore, it has always been a major challenge and an important research direction in the development of NLRP3 inhibitors to obtain, by improving the structure, NLRP3 inhibitors that possess good cellular activity, excellent pharmacokinetic properties and the ability to efficiently penetrate the blood-brain barrier.

### Summary

The following is a summary of the subject matter described in detail herein. This summary is not intended to limit the protection scope of the claims.

The present application provides a sulfonylurea derivative or a pharmaceutically acceptable salt thereof. The structure of the sulfonylurea derivative is as shown in Formula I, wherein each substituent is as defined herein. The sulfonylurea derivative or a pharmaceutically acceptable salt thereof provided herein can be used as an NLRP3 inflammasome inhibitor.

Specifically, the present application provides a sulfonylurea derivative or a pharmaceutically acceptable salt thereof, wherein the sulfonylurea derivative has a structure shown in Formula I, 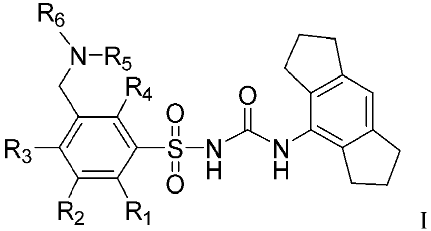 wherein:
R₁ is a non-alkyl substituent, e.g., selected from H, halogen, nitro, amino, hydroxyl, C₁₋₆ alkoxy, C₂₋₆ alkenyl, C₂₋₆ alkynyl, C₁₋₆ alkylamino, aryl, aryloxy, heteroaryl, carboxyl, and heteroalkyl;
R₂ is selected from H, halogen, nitro, amino, hydroxyl, C₁₋₆ alkoxy, C₂₋₆ alkenyl, C₂₋₆ alkynyl, C₂₋₆ alkylamino, aryl, aryloxy, heteroaryl, carboxyl, and heteroalkyl;
R₃ is a non-alkyl substituent, e.g., selected from H, halogen, nitro, amino, hydroxyl, C₁₋₆ alkoxy, halo C₁₋₆ alkoxy, C₂₋₆ alkenyl, C₂₋₆ alkynyl, C₁₋₆ alkylamino, aryl, aryloxy, aryl C₁₋₆ alkoxy, heteroaryl, carboxyl, and heteroalkyl;
R₄ is selected from H, halogen, nitro, amino, hydroxyl, C₁₋₆ alkoxy, C₁₋₆ alkyl, C₂₋₆ alkenyl, C₂₋₆ alkynyl, C₂₋₆ alkylamino, aryl, aryloxy, heteroaryl, carboxyl, and heteroalkyl;
wherein the four substituents R₁, R₂, R₃, and R₄ are not simultaneously H;
R₅ is C₁₋₆ alkyl;
R₆ is C₁₋₆ alkyl;
when R₄ is C₁₋₆ alkyl, R₆ together with R₄ forms a heterocyclic hydrocarbon group;
or when R₃ is C₁₋₆ alkyl, R₆ together with R₃ forms a heterocyclic hydrocarbon group;
or when R₄ is C₁₋₆ alkoxy, methylene linked to an N atom, together with R₄, forms a heterocyclic hydrocarbon group.

In some embodiments, the halogen is F, Cl, Br, or I, preferably F or Cl.

In some embodiments, R₁ is selected from H, halogen, nitro, or amino, preferably H, F, Cl, -NO₂, or -NH₂.

In some embodiments, R₂ is H or halogen, preferably H or F.

In some embodiments, R₃ is selected from H, C₁₋₃ alkoxy, halo C₁₋₃ alkoxy, aryl C₁₋₃ alkoxy, hydroxyl, halogen, or amino, preferably H, -OCH₃, -OBn, F, Cl, -NH₂, -OCF₃, -OCH₂CH₃, - OCH(CH₃)₂, or -OH.

In some embodiments, R₄ is selected from H, halogen, C₁₋₃ alkyl, C₁₋₃ alkoxy, amino, hydroxyl, or nitro, preferably H, -NO₂, -OCH₂CH₃, -NH₂, F, Cl, -CH₃, or -OH; when R₄ is - OCH₂CH₃, methylene linked to an N atom, together with R₄, forms a six-membered heterocyclic hydrocarbon group.

In some embodiments, R₅ and R₆ are each independently methyl or ethyl; preferably, said R₅ and R₆ are simultaneously methyl or simultaneously ethyl.

In some embodiments, R₆ together with R₄ forms a six-membered heterocyclic hydrocarbon group.

In some embodiments, R₆ together with R₃ forms a six-membered heterocyclic hydrocarbon group.

In some embodiments, the sulfonylurea derivative is selected from:

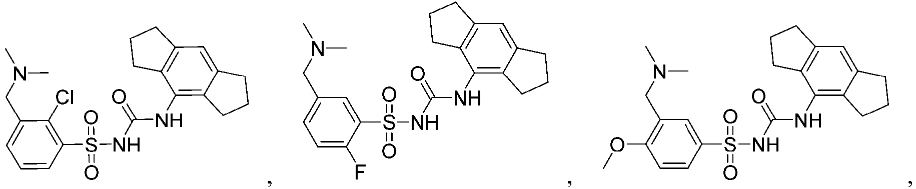

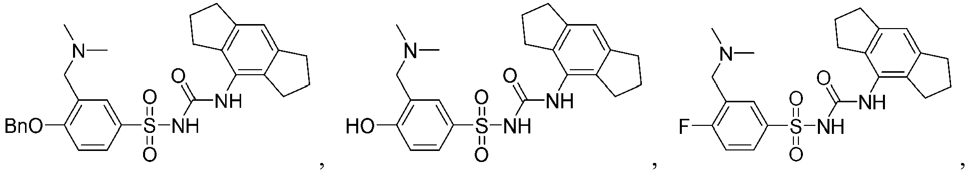

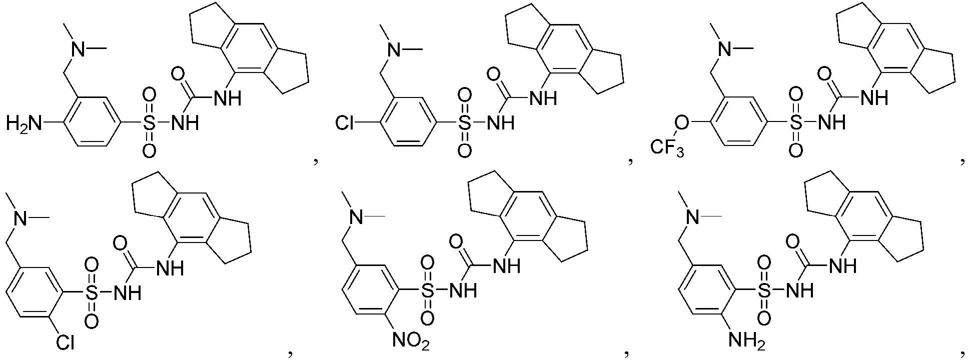

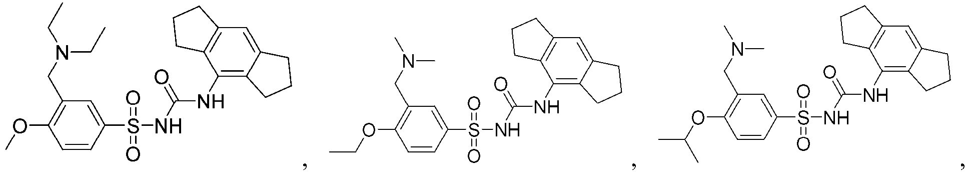

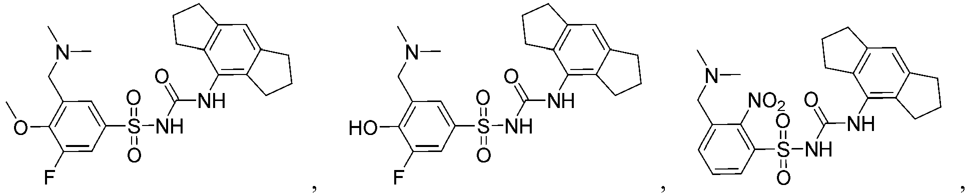

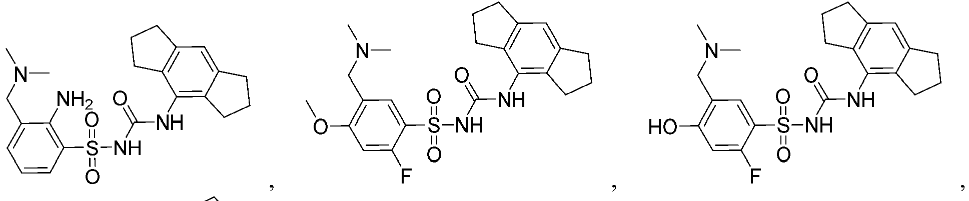

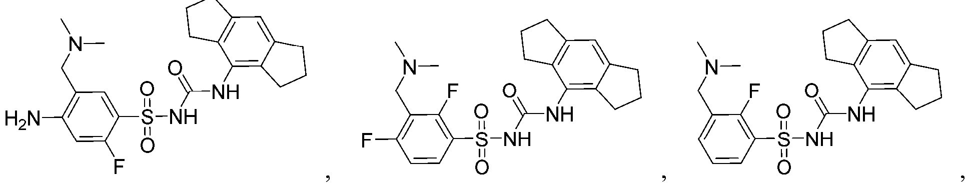

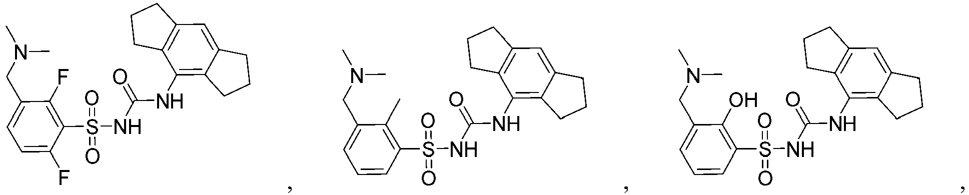

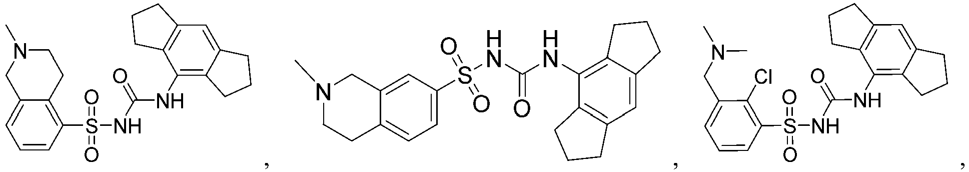

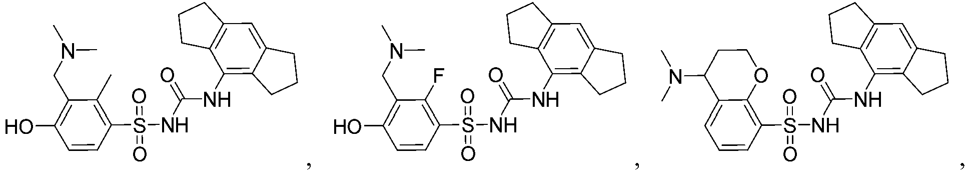

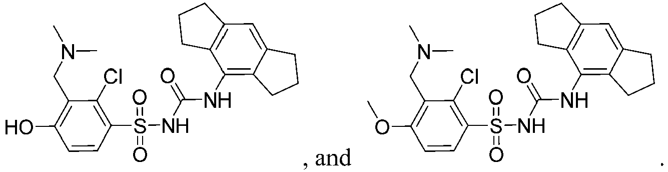

In some embodiments, the sulfonylurea derivative is a compound having a structure shown in Formula I, wherein:
R₁ is selected from H, halogen (preferably F), and nitro;
R₂ is selected from H and halogen (preferably F);
R₃ is selected from H, halogen (preferably F), amino, hydroxyl, C₁₋₆ alkoxy, and aryl C₁₋₆ alkoxy;
R₄ is selected from H, halogen (preferably F), nitro, amino, C₁₋₆ alkyl;
wherein the four substituents R₁, R₂, R₃, and R₄ are not simultaneously H; and
R₅ and R₆ are each independently C₁₋₆ alkyl; preferably, R₅ and R₆ are simultaneously methyl or ethyl.

In some embodiments, the sulfonylurea derivative is selected from: 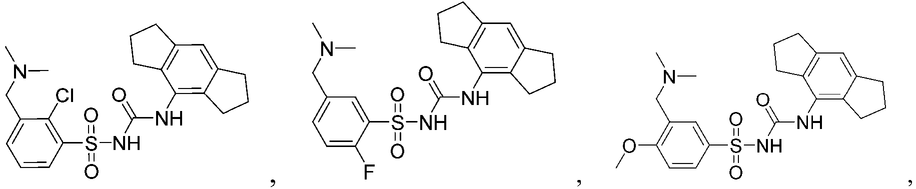 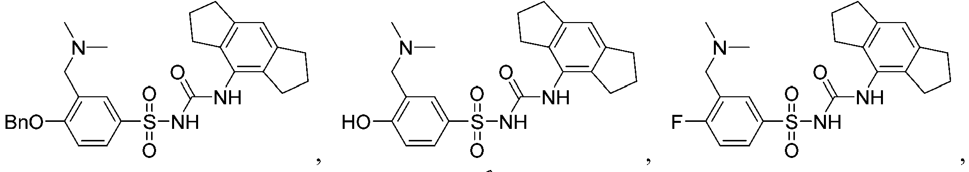 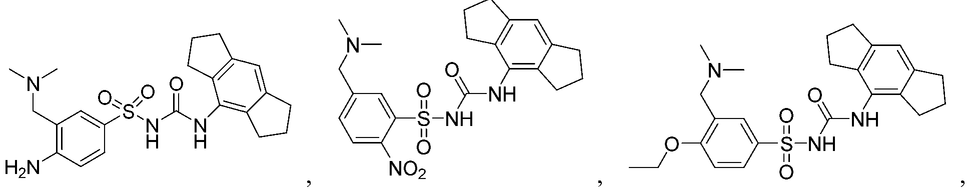 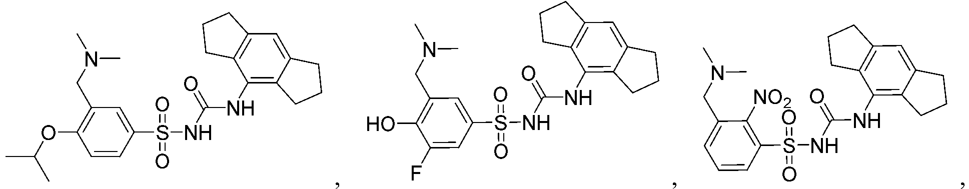 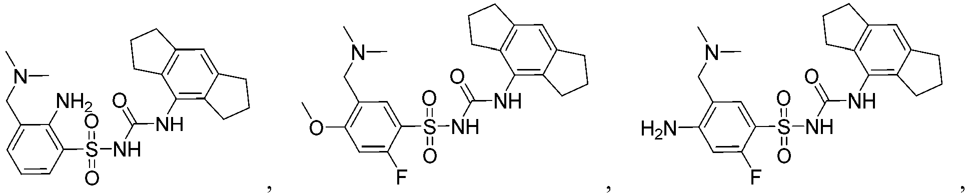 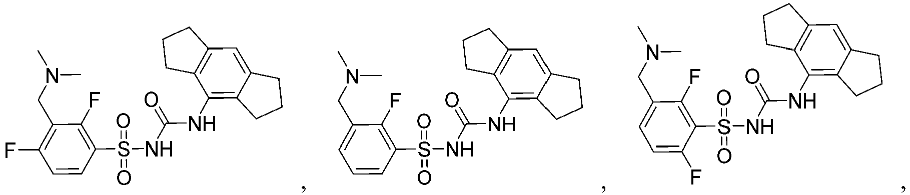 and

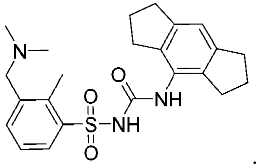

In some embodiments, the sulfonylurea derivative is selected from: 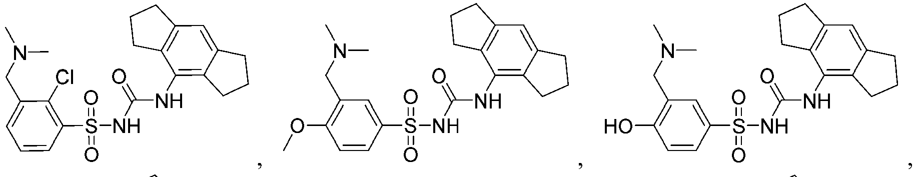 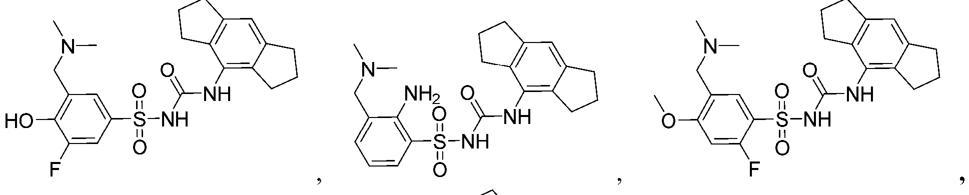 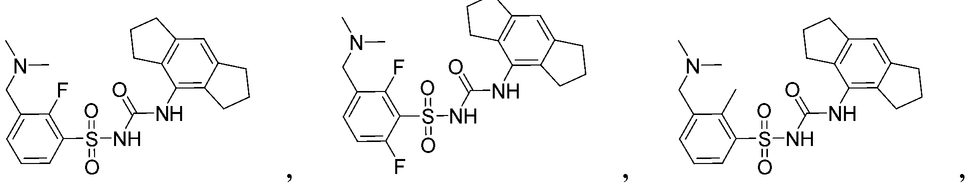 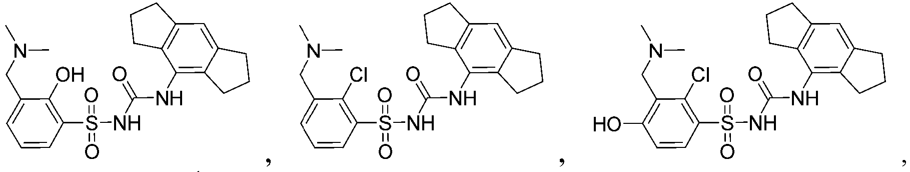 and

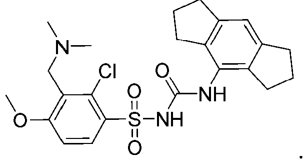

The present application also provides the above sulfonylurea derivative or the pharmaceutically acceptable salt thereof for use as:
(i) an NLRP3 inflammasome inhibitor; and/or
(ii) a modulator for one or more of IL-1β, IL-17, IL-18, IL-1α, IL-37, IL-33, and Th17 cells.

The present application also provides a composition comprising the above sulfonylurea derivative or the pharmaceutically acceptable salt thereof, and a pharmaceutically acceptable excipient.

The present application also provides use of the above sulfonylurea derivative or the pharmaceutically acceptable salt thereof or the above composition in the preparation of a medicament for treating or preventing a disease, disorder, or condition.

The present application also provides the above sulfonylurea derivative or a pharmaceutically acceptable salt thereof or the above composition for use in the treatment or prevention of a disease, disorder, or condition.

The present application also provides a method for treating or preventing a disease, disorder, or condition, comprising administering the above sulfonylurea derivative or the pharmaceutically acceptable salt thereof or the above composition to a subject in need thereof.

The disease, disorder, or condition that can be treated or prevented by using the above sulfonylurea derivative or the pharmaceutically acceptable salt thereof or the above composition is:
(i) an immune system disease, disorder, or condition;
(ii) an inflammatory disease, disorder, or condition, or an autoimmune disease, disorder, or condition;
(iii) a skin disease, disorder, or condition;
(iv) a cardiovascular system disease, disorder, or condition;
(v) tumor;
(vi) a renal system disease, disorder, or condition;
(vii) a gastrointestinal disease, disorder, or condition;
(viii) a respiratory system disease, disorder, or condition;
(ix) an endocrine system disease, disorder, or condition;
(x) a central nervous system (CNS) disease, disorder, or condition; and/or
(xi) local or systemic infection.

In some embodiments, the disease, disorder, or condition is selected from the group consisting of: constitutive inflammation, including cryopyrin associated periodic syndrome (CAPS), Muckle-Wells syndrome (MWS), familial cold autoinflammatory syndrome (FCAS) and neonatal-onset multisystem inflammatory disease (NOMID), autoinflammatory disease, familial Mediterranean fever (FMF), TNF receptor-associated periodic syndrome (TRAPS), mevalonate kinase deficiency (MKD), hyperimmunoglobulin D with periodic fever syndrome (HIDS), deficiency of interleukin-1-receptor antagonist (DIRA), Majeed syndrome, pyogenic arthritis, pyoderma gangrenosum and acne syndrome (PAPA), haploinsufficiency of A20 (HA20), pediatric granulomatous arthritis (PGA), PLCG2-associated antibody deficiency and immune dysregulation (PLAID), autoinflammation and PLCG2-associated antibody deficiency and immune dysregulation (APLAID), and sideroblastic anemia with B-cell immunodeficiency, periodic fevers, and developmental delay (SIFD); autoimmune diseases, including multiple sclerosis (MS), type 1 diabetes, psoriasis, rheumatoid arthritis, Behçet's disease, Sjögren's syndrome, and Schnitzler syndrome; macrophage activation syndrome; Blau syndrome; respiratory system diseases, including chronic obstructive pulmonary disease (COPD), asthma such as allergic asthma and steroid-resistant asthma, asbestosis, silicosis, and cystic fibrosis; dermatitis, including contact dermatitis; central nervous system diseases, including Parkinson's disease, Alzheimer's disease, motor neuron disease, Huntington's disease, cerebral malaria and pneumococcal meningitis-induced brain injuries; metabolic diseases, including type 2 diabetes, atherosclerosis, obesity, gout, pseudogout; eye diseases, including those of ocular epithelium, age-related macular degeneration (AMD), uveitis, corneal infections, and dry eye syndrome; kidney diseases, including chronic kidney disease, oxalate nephropathy, nephrocalcinosis, and diabetic kidney disease; liver diseases, including nonalcoholic steatohepatitis (NASH) and alcoholic liver disease; skin inflammatory responses, including contact hypersensitivity and sunburn; joint inflammatory responses, including osteoarthritis, systemic juvenile idiopathic arthritis, adult-onset Still's disease, and relapsing polychondritis; viral infections, including alphavirus (Chikungunya virus, Ross River virus) and flavivirus (dengue virus, Zika virus), influenza virus, and HIV; hidradenitis suppurativa (HS) and other skin diseases causing cysts; cancers, including lung cancer metastasis, pancreatic cancer, gastric cancer, myelodysplastic syndromes, and leukemia; polymyositis; stroke, including ischemic stroke; myocardial infarction, including recurrent myocardial infarction; congestive heart failure; embolism; cardiovascular diseases; graft-versus-host disease; hypertension; colitis; helminth infection; bacterial infection; abdominal aortic aneurysm; wound healing; depression, psychological stress; sepsis; septic shock; ischemia-reperfusion injury; and any disease in which an individual has been determined to carry a germline or somatic non-silent mutation in NLRP3.

In some embodiments, the disease, disorder, or condition is:
(i) an autoinflammatory disease such as cryopyrin associated periodic syndrome (CAPS), Muckle-Wells syndrome (MWS), familial cold autoinflammatory syndrome (FCAS), familial Mediterranean fever (FMF), neonatal-onset multisystem inflammatory disease (NOMID), tumor necrosis factor (TNF) receptor-associated periodic syndrome (TRAPS), hyperimmunoglobulin D with periodic fever syndrome (HIDS), deficiency of interleukin-1-receptor antagonist (DIRA), Majeed syndrome, or pyogenic arthritis, pyoderma gangrenosum and acne (PAPA);
(ii) Parkinson's disease or Huntington's disease;
(iii) gout or juvenile idiopathic arthritis;
(iv) nonalcoholic steatohepatitis (NASH);
(v) oxalate nephropathy or nephrocalcinosis;
(vi) uveitis;
(vii) hidradenitis suppurativa (HS);
(viii) myelodysplastic syndrome, macrophage activation syndrome, Schnitzler syndrome, adult-onset Still's disease, or Behçet's disease; or
(ix) sepsis or septic shock.

Compared with the prior art, the present application has achieved the following beneficial effects: the compounds in the present invention possess good NLRP3 inhibitory activity, excellent pharmacokinetic properties and the ability to efficiently penetrate the blood-brain barrier, and therefore can be used in both non-nervous system diseases and nervous system diseases that require penetration of the blood-brain barrier.

Additional features and advantages of the present application will be set forth in the description which follows, and in part will become apparent from the description, or may be learned by practice of the application. Other advantages of the present application may be realized and obtained by solutions described in the description and the drawings. Other aspects will be apparent upon reading and understanding the drawings and detailed description.

### Brief Description of Drawings

The accompanying drawings are used to provide an understanding of the technical solutions of the present application, and constitute a part of the specification. They are used to explain the technical solutions of the present application together with the embodiments of the present application, and do not constitute a limitation to the technical solutions of the present application. Different NLRP3 activation models correspond to different pathological conditions, and thus the same examples may reflect different inhibitory effects.
FIG. 1 shows that in Experimental Example 1, Example 1, Example 5, Example 17, Example 20, Example 24, Example 25, Example 26, Example 27, and Example 28 can effectively inhibit the release of IL-1β at the concentration of 1 µM (the release amount is reduced by 80% or more compared with the positive control); while Example 16 and Example 21 have a weaker inhibitory effect on IL-1β release at the concentration of 1 µM (the release amount is reduced by 20%-80% compared with the positive control).
FIG. 2 shows that in Experimental Example 1, Example 2, Example 3, Example 4, and Example 6 can effectively inhibit the release of IL-1β at the concentration of 1 µM (the release amount is reduced by 80% or more compared with the positive control); while Example 8 and Example 9 only have a weaker inhibitory effect at 1 µM (the release amount is reduced by 20%-80% compared with the positive control).
FIG. 3 shows that in Experimental Example 1, Example 7, Example 10, Example 14, Example 15, Example 18, Example 19, Example 22, and Example 23 can effectively inhibit the release of IL-1β at the concentration of 1 µM (the release amount is reduced by 80% or more compared with the positive control); while Example 10 has no inhibitory effect at the concentration of 1 µM.
FIG. 4 shows that in Experimental Example 1, Example 11 can effectively inhibit the release of IL-1β at the concentration of 1 µM (the release amount is reduced by 80% or more compared with the positive control). Example 12 has a weaker inhibitory effect on IL-1β release at the concentration of 1 µM (the release amount is reduced by 20%-80% compared with the positive control), and Example 13 has no inhibitory effect at the concentration of 1 µM.
FIG. 5 shows that in Experimental Example 2, Example 3, Example 5, Example 14, Example 17, Example 18, Example 19, Example 20, Example 22, Example 23, Example 24, Example 25, and Example 26 have an effective inhibitory effect at the concentration of 10 µM (with a reduction of 50% or more compared with the positive control).
FIG. 6 shows that in Experimental Example 4, Example 17 and Example 27 have an effective inhibitory effect at the concentration of 1 µM (with a reduction of 75% or more in release amount compared with that of the positive control minus the LPS-alone control).
FIG. 7 shows that in Experimental Example 4, Example 29 has an effective inhibitory effect at the concentration of 1 µM (with a reduction of 50% or more compared with the positive control).
FIG. 8 shows that in Experimental Example 4, Example 31 has an effective inhibitory effect at the concentration of 1 µM (with a reduction of 50% or more compared with the positive control).
FIG. 9 shows that in Experimental Example 4, Example 32 has an effective inhibitory effect at the concentration of 1 µM (with a reduction of 50% or more compared with the positive control).
FIG. 10 shows that in Experimental Example 7, Example 19 can significantly inhibit the release of IL-1β in a mouse sepsis model, has a faster onset than MCC950, and is better than MCC950 in improving the mouse survival rate.
FIG. 11 shows that in Experimental Example 8, Example 19 can significantly inhibit the release of IL-1β in a mouse gout-related peritonitis model, with an inhibitory effect stronger than that of MCC950.
FIG. 12 shows the results of mouse plasma pharmacokinetic analysis of Example 3.
FIG. 13 shows the results of mouse plasma pharmacokinetic analysis of Example 5.
FIG. 14 shows the results of mouse plasma pharmacokinetic analysis of Example 19.
FIG. 15 shows the results of mouse plasma pharmacokinetic analysis of the control drug MCC950.
FIG. 16 shows the results of mouse plasma pharmacokinetic analysis of Example 17.
FIG. 17 shows the results of mouse plasma pharmacokinetic analysis of Example 27.
FIG. 18 shows the results of mouse plasma pharmacokinetic analysis of Example 29.
FIG. 19 shows the results of rat plasma pharmacokinetic analysis of Example 5.
FIG. 20 shows the results of rat plasma pharmacokinetic analysis of Example 19.
FIG. 21 shows the results of rat plasma pharmacokinetic analysis of MCC950.

### Detailed Description

The present invention is further illustrated by the following examples, but any of the examples or combinations thereof should not be construed as limiting the scope or embodiments of the present invention. The scope of the present invention is defined by the appended claims, and those skilled in the art, in light of this specification and common general knowledge in the art, can clearly understand the scope defined by the claims. Without departing from the spirit and scope of the present invention, those skilled in the art can make any modifications or changes to the technical solutions of the present invention, and such modifications and changes are also included in the scope of the present invention.

### Terminology

As used herein, halogen refers to a fluorine, chlorine, bromine, or iodine atom.

Cₘ₋ₙ as used herein refers to a moiety having an integer (in a given range) number of carbon atoms. For example, "C₁₋₆" means that the group may have 1 carbon atom, 2 carbon atoms, 3 carbon atoms, 4 carbon atoms, 5 carbon atoms, or 6 carbon atoms.

The term "alkyl" refers to a hydrocarbon group having the general formula CₙH_{2n +1}. Alkyl may be linear or branched. For example, the term "C₁₋₆ alkyl" refers to an alkyl group containing 1-6 (e.g., 1, 2, 3, 4, 5, 6) carbon atoms (e.g., methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, etc.). Similarly, the alkyl portion (i.e., alkyl) of alkoxy, alkylamino, and heteroalkyl has the same definition as described above.

The term "alkenyl" refers to a linear or branched unsaturated aliphatic hydrocarbon group consisting of carbon atoms and hydrogen atoms and having at least one double bond. For example, the term "C_{2¬6} alkenyl" shall be understood as preferably representing a linear or branched, monovalent hydrocarbon group which contains one or more double bonds and has 2, 3, 4, 5, or 6 carbon atoms. It shall be understood that where alkenyl contains more than one double bond, the double bonds may be mutually isolated or conjugated. The alkenyl includes, but is not limited to, vinyl, propenyl, (E)-2-methylvinyl, (Z)-2-methylvinyl, (E)-but-2-enyl, (Z)-but-2-enyl, (E)-but-1-enyl, (Z)-but-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, etc.. The above description about alkenyl may apply to alkenylene, but alkenylene is a divalent group.

The term "alkynyl" refers to a linear or branched, unsaturated aliphatic hydrocarbon group consisting of carbon atoms and hydrogen atoms and having at least one triple bond. For example, the term "C_{2¬6} alkynyl" shall be understood as preferably representing a linear or branched hydrocarbon group which contains one or more triple bonds and has 2, 3, 4, 5, or 6 carbon atoms. Alkynyl includes, but is not limited to, ethynyl and propynyl.

The term "alkoxy" refers to -O-alkyl.

The term "alkylamino" refers to -NH-alkyl.

The term "heteroalkyl" refers to an alkyl group containing 1 to 5 heteroatoms (e.g., 1, 2, 3, 4, or 5 heteroatoms) independently selected from sulfur, oxygen, and/or nitrogen.

The term "hydroxyl" refers to -OH group.

The term "nitro" refers to -NO₂ group.

The term "amino" refers to -NH₂ group.

The term "carboxyl" refers to -COOH group.

The term "membered" refers to the number of skeletal atoms forming a ring. For example, "3-12-membered" means that the number of skeleton atoms forming a ring is 3, 4, 5, 6, 7, 8, 9, 10, 11, or 12.

The term "aryl" refers to a monocyclic or fused polycyclic system consisting only of carbon atoms as ring-forming atoms and having at least one aromatic ring. The term "C₆₋₁₈ aryl" refers to an aryl group as defined above containing 6 to 18 (e.g., 6 to 15, 6 to 12, 6 to 10, 6 to 8) carbon atoms. Aryl includes ring systems formed by fusion of aromatic rings with aromatic rings, or aromatic rings with non-aromatic carbocyclic rings (e.g., cycloalkanes, cycloalkenes, or cycloalkynes). Non-limiting examples of aryl include, but are not limited to, phenyl, naphthyl, anthryl, 1,2,3,4-tetrahydronaphthyl, indanyl, etc..

The term "heteroaryl" refers to a monocyclic or fused polycyclic system containing at least one (e.g., 1 to 5, e.g., 1, 2, 3, 4, or 5) ring atom selected from N, O, and S, the remaining ring atoms being carbon, and having at least one aromatic ring. Ring-forming carbon atoms and heteroatoms may be substituted with oxo- or thio- groups. The term "C₂₋₁₈ heteroaryl" refers to a heteroaryl group as defined above having 2 to 18 (e.g., 2 to 15, 2 to 12, 2 to 10, 2 to 8) carbon atoms. The heteroaryl includes ring systems formed by fusion of heteroaromatic rings with aromatic carbocyclic rings, heteroaromatic rings with heteroaromatic rings, or heteroaromatic rings with non-aromatic carbocyclic rings (e.g., cycloalkanes, cycloalkenes, or cycloalkynes) or heterocycles (e.g., heterocycloalkanes, heterocycloalkenes, or heterocycloalkynes), and in the foregoing fused heteroaryl, the link site at which the heteroaryl is linked to the rest of the molecule can be either on the heteroaromatic rings or on other rings fused with the heteroaromatic rings. Non-limiting examples of heteroaryl include, but are not limited to, pyrrolyl, furyl, thienyl, imidazolyl, oxazolyl, pyrazolyl, pyridyl, pyrimidinyl, pyrazinyl, quinolyl, isoquinolyl, tetrazolyl, triazolyl, triazinyl, benzofuryl, benzothienyl, indolyl, and isoindolyl, etc..

The term "aryloxy" refers to -O-aryl.

The term "heterocyclic hydrocarbon group" refers to a fully saturated or partially saturated, cyclic group which may be present as a single ring, a fused ring, a bridged ring, or a spiro ring. Heterocycle may be a 3-12-membered ring, such as a 3-10-membered ring, 3-8-membered ring, a 3-7-membered ring, and a 5-7-membered ring, containing 1 to 5 heteroatoms (e.g., 1, 2, 3, 4, or 5 heteroatoms) independently selected from sulfur, oxygen, and/or nitrogen. Examples of 3-membered heterocyclic hydrocarbon group include, but are not limited to, oxiranyl, thiiranyl, and aziridinyl; non-limiting examples of 4-membered heterocyclic hydrocarbon group include, but are not limited to, azetidinyl, oxetanyl, thietanyl, and azetidinyl; examples of 5-membered heterocyclic hydrocarbon group include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocyclic hydrocarbon group include, but are not limited to, piperidinyl, tetrahydropyranyl, tetrahydrothiopyranyl, morpholinyl, piperazinyl, 1,4-thioxanyl, 1,4-dioxanyl, thiomorpholinyl, 1,3-dithianyl, and 1,4-dithianyl; and examples of 7-membered heterocyclic hydrocarbon group include, but are not limited to, azepanyl, oxepanyl, thiepanyl, and 2-oxa-6-aza-spiro[3,3]heptanyl. Examples of 8-membered heterocyclic hydrocarbon group include, but are not limited to, 8-azabicyclo[3.2.1]octanyl, 3,8-diazabicyclo[3.2.1]octanyl, and hexahydropyrrolizinyl. Examples of other heterocyclic hydrocarbon groups include, but are not limited to, octahydroquinolizinyl, 1,1-dioxothiomorpholinyl, etc..

As used herein, the sulfonylurea compounds of the present application include all stereoisomers, geometric isomers, tautomers, and isotopic variants of the structures depicted. Compounds herein identified by structure or name as a particular tautomer also include other tautomers unless otherwise specifically indicated. Unless otherwise specifically stated, the sulfonylurea compounds of the present application may be present as enantiomers, epimers, or racemates or mixtures thereof. Stereoisomers of the sulfonylurea compounds of the present application include cis and trans isomers, optical isomers (such as enantiomers), epimers, geometric isomers, rotamers, atropisomers, conformational isomers, and tautomers of the sulfonylurea compounds of the present application, and also include mixtures of various isomeric forms, as well as racemates and a mixture of epimer pairs.

As used herein, the atoms in the sulfonylurea compounds of the present application may be present in natural isotopic abundance, or one or more of the atoms are present as specific isotopes that are artificially enriched. The isotopes have the same atomic number as the atom predominately present in nature, but the atomic mass thereof is different from the atomic mass predominately present in nature. The present application encompasses all suitable isotopic variations of the sulfonylurea compounds described herein, such as deuterated compounds, and deuterium enrichment may provide certain therapeutic advantages, such as increasing in vivo half-life or reducing dosage requirement, or may provide compounds that can be used as standards for characterization of biological samples. Those skilled in the art can prepare isotope-enriched compounds by using appropriate isotope-enriched reagents or intermediates, according to known conventional techniques or by referring to the preparation methods in the examples herein. Examples of isotopes that may be incorporated into the sulfonylurea compounds of the present application include ²H, ³H, ¹³C, ¹⁴C, ¹⁵N, ¹⁸O, ¹⁷O, ³¹P, ³²P, ³⁵S, ¹⁸F, ³⁶Cl, ⁸²Br, ¹²³I, ¹²⁴I, ¹²⁹I, and ¹³¹I.

As used herein, the sulfonylurea compounds of the present application may be present as solvates or non-solvates. The solvates described here include hydrates, and the solvents or water in the solvates or hydrates and the sulfonylurea compounds of the present application may be either in a strong binding relationship (i.e., stoichiometric), or in a weak binding relationship (e.g., adsorbed solvents or water). The solvate refers to a complex formed by the sulfonylurea compound of the present application and one or more pharmaceutically acceptable solvent molecules, and the pharmaceutically acceptable solvent can be methanol, ethanol, acetone, dimethylformamide, or water, etc.. When the solvent is water, the solvate is a hydrate.

As used herein, the sulfonylurea compounds of the present invention can be present in the form of their pharmaceutically acceptable salts, for example, acid addition salts or base addition salts of the sulfonylurea compounds of the present invention. Here, the acid can be an inorganic acid, for example, hydrochloric acid, hydrobromic acid, hydroiodic acid, nitric acid, sulfuric acid, hydrogen sulfate, sulfurous acid, hydrogen sulfite, phosphoric acid, acid phosphate, carbonic acid, and hydrogen carbonate; or an organic acid, for example, formic acid, acetic acid, propionic acid, pantothenic acid, lactic acid, oxalic acid, salicylic acid, citric acid, hydrogen citrate, tartaric acid, hydrogen tartrate, succinic acid, maleic acid, fumaric acid, glucuronic acid, gluconic acid, benzoic acid, methanesulfonic acid, ethanesulfonic acid, benzenesulfonic acid, p-toluenesulfonic acid, ascorbic acid, gentisic acid, pamoic acid, camphorsulfonic acid, mandelic acid, saccharic acid, amino acids (such as natural amino acids, L-glycine, L-aspartic acid, L-glutamic acid, L-valine, etc.). The base can be an inorganic base, for example, hydroxides of sodium, potassium, calcium, magnesium, manganese, iron, zinc, or aluminum; or an organic base, including basic amino acids (such as arginine, lysine, histidine), ammonia, primary amine, secondary amine, tertiary amine, cyclic amines (such as piperidine, morpholine, piperazine).

As used herein, the term "pharmaceutically acceptable" refers to those compounds, materials, compositions, and/or dosage forms which are, within the scope of sound medical judgment, suitable for use in contact with human and animal tissue, without undue toxicity, irritation, allergic response, or other problems or complications, commensurate with a reasonable benefit/risk ratio.

As used herein, the term "pharmaceutically acceptable excipient" refers to those auxiliary materials that do not cause significant irritation to an organism and do not impair the biological activity and properties of the active compounds. Suitable auxiliary materials are well known to those skilled in the art, e.g., carbohydrates, waxes, water-soluble and/or water-swellable polymers, hydrophilic or hydrophobic materials, gelatin, oils, solvents, water, etc..

As used herein, the words "comprise" or "comprise" and variations thereof, such as "comprises" or "comprising" shall be construed in an open-ended, non-exclusive sense, i.e., as "including, but not limited to".

As used herein, the term "pharmaceutical composition" refers to a mixture of one or more of the compounds of the present application or salts thereof, and pharmaceutically acceptable auxiliary materials. The purpose of the pharmaceutical composition is to facilitate administration of the compound of the present application to an organism.

As used herein, the effective amount refers to any one or more beneficial or desired amounts of the sulfonylurea compound or pharmaceutical composition of the present application that is sufficient to affect a disease, including the biochemical, histological, and/or behavioral symptoms of the disease, its complications, and intermediate pathological phenotypes presented during the development of the disease. The therapeutically effective amount refers to an amount of the compound that, to some extent, alleviates one or more symptoms of the disorder being treated.

As used herein, the term "treating" means administering the compound or formulation of the present application to ameliorate or eliminate a disease or one or more symptoms associated with the disease, and includes:
(i) inhibiting the disease or disease state, i.e., arresting its development; and
(ii) relieving the disease or disease state, i.e., causing regression of the disease or disease state.

As used herein, the term "preventing" means administering the compound or formulation of the present application to prevent a disease or one or more symptoms associated with the disease, and includes:
preventing the occurrence of the disease or disease state in a subject (e.g., a mammal), in particular, when such subject is predisposed to the disease state but has not yet been diagnosed as having it.

The compounds of the present application can be prepared by a variety of synthetic methods, including the specific embodiments listed below, embodiments formed by their combination with other chemical synthetic methods, and equivalents well known to those skilled in the art. Preferred embodiments include, but are not limited to, the examples of the present application.

The chemical reactions of the specific embodiments of the present application are carried out in suitable solvents, which must be appropriate for the chemical transformations of the present application and the reagents and materials required thereby. In order to obtain the compounds of the present application, those skilled in the art sometimes need to modify or select the synthetic steps or reaction schemes based on existing embodiments.

**The following abbreviations are used in the present application:**
Toluene: toluene; NCS: N-chlorosuccinimide; HCl: hydrochloric acid; ACN or MeCN: acetonitrile; H₂O: water; NH₃: ammonia; BTC or triphosgene: triphosgene; TFA: trifluoroacetic acid; NH₄HCO₃: ammonium bicarbonate; DMSO: dimethyl sulfoxide; EA: ethyl acetate; THF: tetrahydrofuran; DIEA: N,N-diisopropylethylamine; DMF: N,N-dimethylformamide; DCM: dichloromethane; Pd₂(dba)₃: trisdibenzylideneacetone dipalladium; LCMS: liquid chromatography-mass spectrometry; TLC: thin layer chromatography; HPLC: high performance liquid chromatography; RP-HPLC: reverse phase high performance liquid chromatography; STAB or NaBH(AcO)₃: sodium triacetoxyborohydride; AcOH: acetic acid; MeOH: methanol; EtOH: ethanol; PPh₃: triphenylphosphine; Pd(PPh₃)₄: tetrakis(triphenylphosphine)palladium; TEA or Et₃N: triethylamine; Boc₂O: di-tert-butyl dicarbonate; DMAP: 4-dimethylaminopyridine; XantPhos: 4,5-bisdiphenylphosphino-9,9-dimethylxanthene; HSBn: benzyl mercaptan; PE: petroleum ether; t-BuXphos: 2-di-tert-butylphosphino-2',4',6'-triisopropylbiphenyl; TTC: 2,3,5-triphenyltetrazolium chloride; HEPES: 4-(2-hydroxyethyl)piperazine-1-ethanesulfonic acid; EGTA: ethylene glycol-bis(2-aminoethylether)-N,N,N',N'-tetraacetic acid; Na₂-ATP: disodium adenosine triphosphate; -OBn: benzyloxy; flash: medium-pressure preparative, flash column chromatography; 2M in THF: 2 mol/L tetrahydrofuran solution; 1M in THF: 1 mol/L tetrahydrofuran solution. Unless otherwise specified, all chemical reagents were purchased from Shanghai Acmec Biochemical Technology Co., Ltd.

**The information of model of the instruments involved in the experiments is as follows:**
LCMS: Agilent 1260-G6125 liquid chromatography-mass spectrometry, using the default ESI mode for reaction monitoring and compound identification, and SIM mode for Drug Metabolism and Pharmacokinetics (DMPK) testing.
Detection HPLC: Agilent 1260 high performance liquid chromatography
Preparative-HPLC: Xiaohe LC5100 preparative high performance liquid chromatography
NMR instrument: Zhongke-Niujin Quantum-1 plus AS400 NMR spectrometer
Medium-pressure preparative instrument (flash, flash column chromatography): ALSE AP-200 medium-pressure preparative chromatography system

Unless otherwise specified, preparative HPLC uses a gradient elution of from 10% acetonitrile/water to 100% acetonitrile/water, with 0.05% trifluoroacetic acid added to the mobile phase; LCMS uses a gradient elution of from 10% acetonitrile/water to 100% acetonitrile, with 0.1% formic acid added to the mobile phase; for medium-pressure preparation, if the mobile phase is not specified, acetonitrile/water is used by default, with a gradient elution of from 10% acetonitrile/water to 100% acetonitrile, otherwise, the first (I) and second (II) solvents will be listed, with a default gradient elution of from 100% of the first solvent to 100% of the second solvent, if trifluoroacetic acid is added, the ratio of trifluoroacetic acid is fixed at 0.05%, and if ammonium bicarbonate is added, the ratio of ammonium bicarbonate is fixed at 0.05%.

Unless otherwise specified, ratios of solvents or liquid reagents mentioned herein are volume ratios.

### Example 1: 2-chloro-3-((dimethylamino)methyl)-N-((1,2,3,5,6,7-hexahydro-s-indacen-4-yl)carbamoyl)benzenesulfonamide

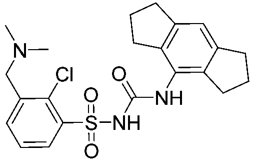

### Synthetic route:

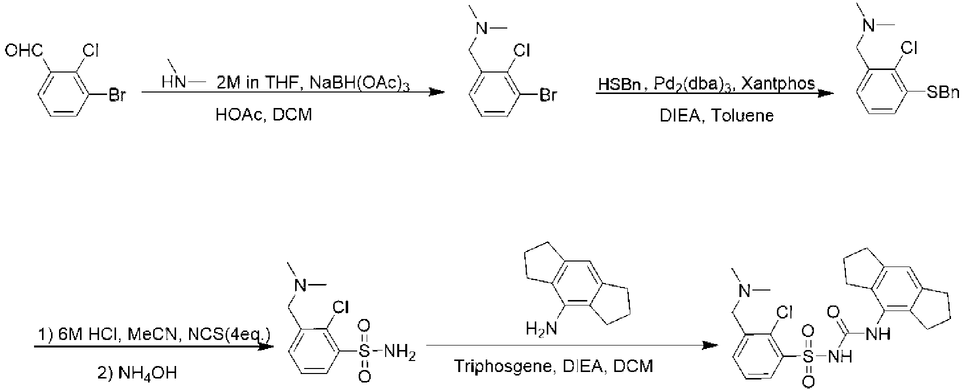

### Step 1: 1-(3-bromo-2-chlorophenyl)-N,N-dimethylmethanamine

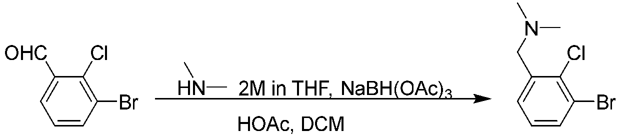

3-bromo-2-chlorobenzaldehyde (0.6 g) was weighed out and placed into a 100 mL single-neck flask, then DCM (15 mL) was added. Dimethylamine (2 M in THF solution) (2 eq.) and HOAc (0.2 eq.) were added while stirring, and the mixture was stirred at room temperature for 2 h. 2 h later, NaBH(OAc)₃ (3 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 v/v (50 mL). The organic phases were combined, washed with saturated brine, dried and rotary evaporated, and passed through flash column chromatography (DCM/MeOH) to yield the target compound, 520 mg of yellow oily liquid.

### Step 2: 1-(3-(benzylmercapto)-2-chlorophenyl)-N,N-dimethylmethanamine

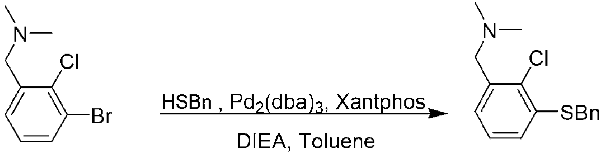

1-(3-bromo-2-chlorophenyl)-N,N-dimethylmethylamine (520 mg) was weighed out and placed into a 40 mL microwave tube, toluene (15 mL) and benzyl mercaptan (2 eq.) were added. Under a nitrogen atmosphere, DIEA (3 eq.), Pd₂(dba)₃ (0.1 eq.), and Xantphos (0.2 eq.) were added. The temperature was raised to 115 °C to react for 12 h. LCMS showed the formation of the target compound. Work-up and purification: The reaction solution was mixed with silica gel, rotary evaporated, and passed through flash column chromatography (DCM/MeOH) to yield the target compound, 400 mg of crude yellow oily liquid.

### Step 3: 3-((dimethylamino)methyl)-2-chlorobenzenesulfonamide

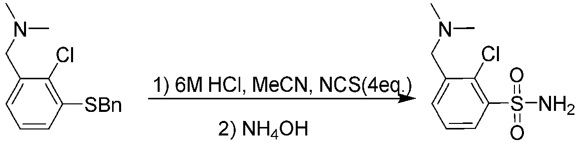

6M HCl (0.3 mL) was measured out and placed into a 50 mL single-neck flask, and MeCN (5 mL) was added. The mixture was stirred and cooled down, and NCS (3 eq.) was added at 0 °C to form a reaction solution. 1-(3-(benzylmercapto)-2-chlorophenyl)-N,N-dimethylmethylamine (200 mg) was dissolved in 5 mL MeCN, and then added dropwise into the above reaction solution at 0 °C. The mixture was stirred for 0.5 h. NH₄OH (10 mL) was placed into another 100 mL single-neck flask, and the above reaction solution was added dropwise into NH₄OH via a constant pressure dropping funnel while stirring at room temperature. The mixture was stirred for 0.5 h. LCMS confirmed the formation of the target compound. Work-up and purification: The reaction solution was rotary evaporated to remove MeCN, and extracted twice with DCM:MeOH=10:1 (50 mL), followed by drying and concentration under reduced pressure to yield the target compound, 210 mg of yellow oily crude product.

### Step 4: 2-chloro-3-((dimethylamino)methyl)-N-((1,2,3,5,6,7-hexahydro-s-indacen-4-yl)carbamoyl)benzenesulfonamide

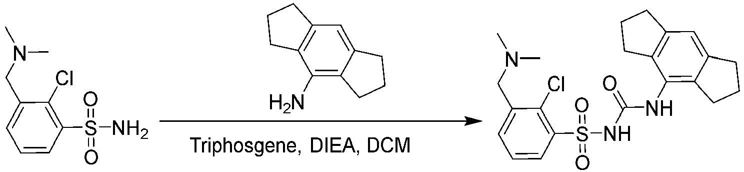

1,2,3,5,6,7-hexahydro-s-indacen-4-amine (100 mg) was weighed out and placed into a 40 mL single-neck flask. DCM (8 mL) and DIEA (5 eq.) were added, and triphosgene (0.4 eq.) was added batchwise while stirring at room temperature. The reaction solution was heated to 50 °C to react for 1 h. 210 mg of crude 3-((dimethylamino)methyl)-2-chlorobenzenesulfonamide was dissolved in DCM (4 mL) and DIEA (3 eq.). Once fully dissolved, the solution was added dropwise into the above reaction solution at 50 °C. The reaction was carried out at 50 °C for 2 h. LCMS confirmed the formation of the target compound. Work-up and purification: The reaction solution was directly mixed with silica gel, and subjected to flash chromatography (DCM/MeOH) to yield a crude product. The crude product was purified by flash chromatography (0.05% NH₄HCO₃) and then lyophilized to yield the target compound, 20 mg of white solid. MS: 448 [M+H]⁺. ¹H NMR (400 MHz, DMSO-*d*₆) δ 8.15 (s, 1H), 7.92 (t, *J* = 7.2 Hz, 1H), 7.56 (s, 1H), 7.40 - 7.32 (m, 1H), 6.80 (s, 1H), 3.56 (s, 2H), 2.74 (t, *J* = 7.2 Hz, 4H), 2.64 - 2.54 (m, 4H), 2.22 (s, 6H), 1.93 - 1.85 (m, 4H).

### Example 2: 5-((dimethylamino)methyl)-2-fluoro-N-((1,2,3,5,6,7-hexahydro-s-indacen-4-yl)carbamoyl)benzenesulfonamide

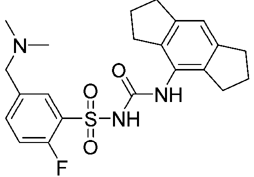

### Synthetic route:

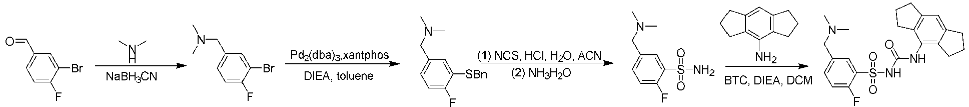

### Step 1: l-(3-bromo-4-fluorophenyl)-N,N-dimethylmethanamine

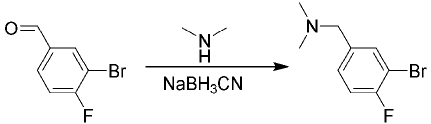

3-bromo-4-fluorobenzaldehyde (1.0 g) was weighed out and placed into a single-neck flask, and then anhydrous methanol (15 mL) and dimethylamine tetrahydrofuran solution (2 eq.) were added. The mixture was stirred at room temperature overnight. The mixture was added with sodium cyanoborohydride (2 eq.) and stirred at room temperature for 1 h. Work-up and purification: LCMS showed the formation of the target compound, and showed that the raw materials were reacted completely. The system was mixed with silica gel and purified by normal-phase flash column chromatography (dichloromethane/methanol, gradient elution of from 0 to 100% methanol) to yield 0.61 g of the target compound.

### Step 2: l-(3-(benzylmercapto)-4-fluorophenyl)-N,N-dimethylmethanamine

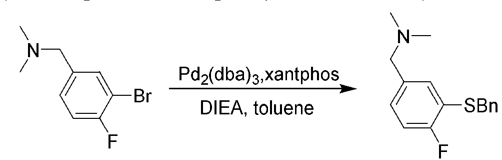

1-(3-bromo-4-fluorophenyl)-N,N-dimethylmethylamine (450 mg) was weighed out and placed into a single-neck flask, and then Pd₂(dba)₃ (0.1 eq.), Xantphos (0.2 eq.), diisopropylethylamine (2 eq.), toluene (10 mL), and benzyl mercaptan (1.5 eq.) were added. The mixture was stirred at 110 °C overnight under nitrogen protection. Work-up and purification: LCMS confirmed the formation of the target compound, and confirmed that the raw materials were reacted completely. The reaction system was filtered, the filter cake was rinsed with dichloromethane, and the filtrate was concentrated to yield a crude product. The crude product was purified by flash column chromatography (dichloromethane/methanol, gradient elution of from 0 to 100% methanol) to yield 420 mg of pure target compound.

### Step 3: 5-((dimethylamino)methyl)-2-fluorobenzenesulfonamide

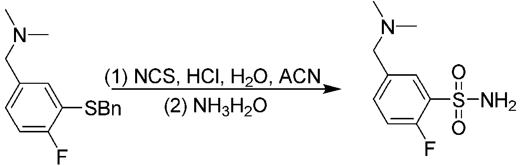

NCS (4 eq.) was weighed out and placed into a single-neck flask, and then acetonitrile (6 mL) and 6 M HCl (3 mL) were added. The mixture was stirred at 0 °C for 15 min. 1-(3-(benzylmercapto)-4-fluorophenyl)-N,N-dimethylmethylamine (400 mg) was added and the mixture was stirred at 0 °C for 1 h. The reaction system was added dropwise to aqueous ammonia (25 mL) slowly, and stirred at room temperature for 1 h. Work-up and purification: LCMS confirmed the formation of the target compound. An appropriate amount of DCM was added to extract the reaction system three times. The organic phases were combined, washed with saturated brine once, and concentrated to yield 420 mg of crude product.

### Step 4: 5-((dimethylamino)methyl)-2-fluoro-N-((1,2,3,5,6,7-hexahydro-s-indacen-4-yl)carbamoyl)benzenesulfonamide

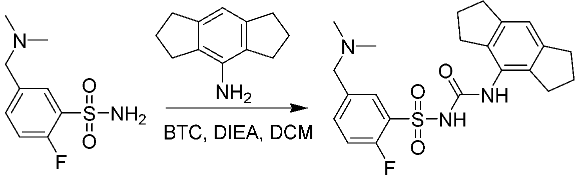

1,2,3,5,6,7-hexahydro-s-indacen-4-amine (1 eq.) was weighed out and placed into a single-neck flask. Then DCM (5 mL) and DIEA (3 eq.) were added, and triphosgene (0.5 eq.) was added at 0 °C. The mixture was stirred at 50 °C for 1 h (called system A). 5-((dimethylamino)methyl)-2-fluorobenzenesulfonamide (400 mg) was weighed out and placed into a single-neck flask, and then DCM (5 mL) and DIEA (3 eq.) were added. The mixture was stirred at room temperature for 1 h (called system B). System B was slowly added to system A at room temperature, and the mixture was stirred at 50 °C for 3 h. Work-up and purification: LCMS confirmed the formation of the target compound, and confirmed that the raw materials were reacted completely. The reaction system was concentrated and purified by flash column chromatography (TFA.H₂O/MeCN) to yield a crude product. The crude product was purified by flash column chromatography (NH₄HCO₃.H₂O/MeCN) and lyophilized to yield 191 mg of white solid product. MS: 432 [M+H]⁺. ¹H NMR (400 MHz, DMSO-*d*₆) δ 9.97 (s, 1H), 8.56 (s, 1H), 8.16 (dd, *J* = 6.9, 2.1 Hz, 1H), 7.88 (dddd, *J* = 8.3, 6.9, 4.5, 2.2 Hz, 1H), 7.65 (dd, *J* = 10.1, 8.7 Hz, 1H), 6.94 (s, 1H), 4.39 (d, *J* = 7.4 Hz, 2H), 2.82 - 2.69 (m, 13H), 2.55 (d, *J* = 7.2 Hz, 2H), 1.93 (p, *J* = 7.4 Hz, 4H).

### Example 3: 3-((dimethylamino)methyl)-N-((1,2,3,5,6,7-hexahydro-s-indacen-4-yl)carbamoyl)-4-methoxybenzenesulfonamide

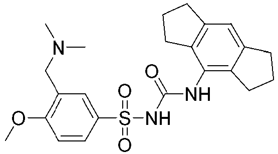

### Synthetic route:

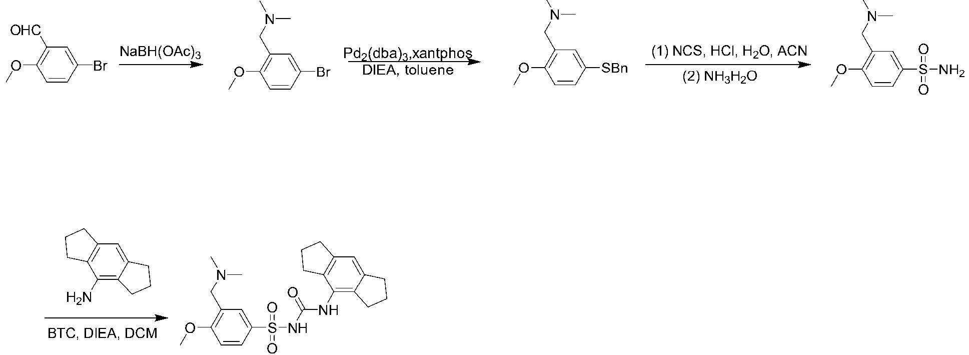

### Step 1: (5-bromo-2-methoxyphenyl)-N,N-dimethylmethylamine

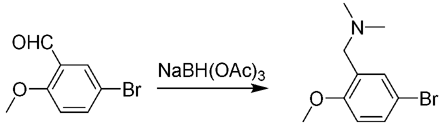

5-bromo-2-methoxybenzaldehyde (3.0 g) was weighed out and placed into a single-neck flask, and then dichloromethane (50 mL), a solution of dimethylamine in tetrahydrofuran (2 eq.), and acetic acid (1 drop) were added. The mixture was stirred at room temperature for 15 min. The mixture was added with sodium triacetoxyborohydride (4 eq.) and stirred at room temperature for 2 h. Work-up and purification: LCMS showed the formation of the target compound, and showed that the raw materials were reacted completely. The reaction system was diluted by adding water, adjusted to a pH>7 with saturated sodium bicarbonate solution, extracted with dichloromethane, dried, and concentrated to yield 3.6 g of crude target compound.

### Step 2: 1-(5-(benzylmercapto)-2-methoxyphenyl)-N,N-dimethylmethanamine

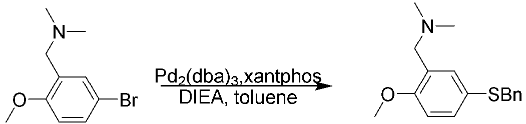

(5-bromo-2-methoxyphenyl)-N,N-dimethylmethylamine (3.6 g) was weighed out and placed into a single-neck flask, and then Pd₂(dba)₃ (0.1 eq.), Xantphos (0.2 eq.), diisopropylethylamine (2 eq.), toluene (50 mL), and benzyl mercaptan (1.5 eq.) were added. The mixture was stirred at 110 °C overnight under nitrogen protection. Work-up and purification: LCMS confirmed the formation of the target compound, and confirmed that the raw materials were reacted completely. The reaction system was filtered, the filter cake was rinsed with dichloromethane, and the filtrate was concentrated to yield a crude product. The crude product was purified by flash column chromatography (dichloromethane/methanol) to yield 4.1 g of pure target compound.

### Step 3: 3-((dimethylamino)methyl)-4-methoxybenzenesulfonamide

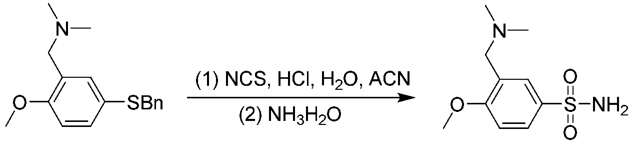

NCS (4 eq.) was weighed out and placed into a single-neck flask, and then acetonitrile (40 mL) and 6M HCl (12 mL) were added. The mixture was stirred at 0 °C for 15min. 1-(5-(benzylmercapto)-2-methoxyphenyl)-N,N-dimethylmethylamine (4 g) was added and then the mixture was stirred at 0 °C for 1 h. The reaction system was added dropwise to aqueous ammonia (200 mL) slowly, and stirred at room temperature for 1 h. Work-up and purification: LCMS confirmed the formation of the target compound. An appropriate amount of DCM was added to extract the reaction system three times, and the organic phases were combined, washed with saturated brine once, and concentrated to yield 2 g of crude product.

### Step 4: ((dimethylamino)methyl)-N-((1,2,3,5,6,7-hexahydro-s-indacen-4-yl)carbamoyl)-4-methoxybenzenesulfonamide

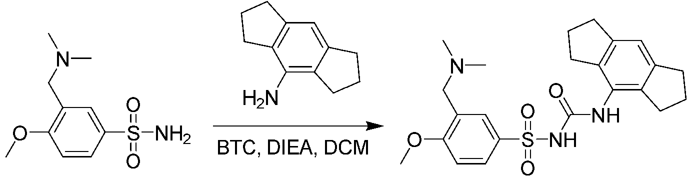

1,2,3,5,6,7-hexahydro-s-indacen-4-amine (1 eq.) was weighed out and placed into a single-neck flask, then DCM (20 mL) and DIEA (3 eq.) were added, and triphosgene (0.5 eq.) was added at 0 °C. The mixture was stirred at 50 °C for 1 h (called system A). 3-((dimethylamino)methyl)-4-methoxybenzenesulfonamide (2 g) was weighed out and placed into a single-neck flask, and then DCM (20 mL) and DIEA (3 eq.) were added. The mixture was stirred at room temperature for 1 h (called system B). System B was slowly added to system A at room temperature, and the mixture was stirred at 50 °C for 3 h. Work-up and purification: LCMS confirmed the formation of the target compound, and confirmed that the raw materials were reacted completely. The reaction system was concentrated and purified by flash column chromatography (TFA.H₂O/MeCN) to yield a crude product. The crude product was purified by flash column chromatography (NH₄HCO₃.H₂O/MeCN) and lyophilized to yield 1.08 g of white solid product. MS: 444 [M+H]⁺. ¹H NMR (400 MHz, DMSO-*d*₆) δ 9.50 (s, 1H),8.20 (s, 1H), 8.08 - 7.98 (m, 2H), 7.35 (d, *J* = 8.8 Hz, 1H), 6.92 (s, 1H), 4.28 (s, 2H), 3.94 (s, 3H), 2.77 (t, *J* = 7.3 Hz, 4H), 2.70 (s, 6H), 2.54 (d, *J* = 7.3 Hz, 4H), 1.96 - 1.89 (m, 4H).

### Example 4: 4-(benzyloxy-3-((dimethylamino)methyl)-N-((1,2,3,5,6,7-hexahydro-s-indacen-4-yl)carbamoyl)benzenesulfonamide

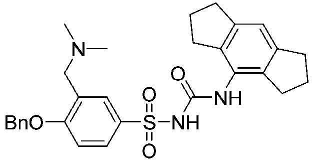

### Synthetic route:

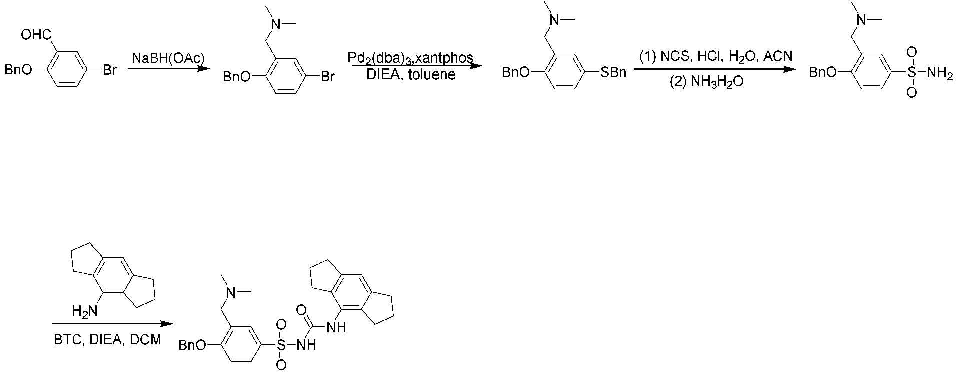

### Step 1: (2-(benzyloxy)-5-bromophenyl)-N,N-dimethylmethanamine

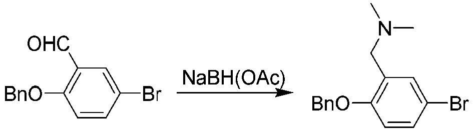

2-(benzyloxy)-5-bromobenzaldehyde (800 mg) was weighed out and placed into a single-neck flask, and then dichloromethane (20 mL), a solution of dimethylamine in tetrahydrofuran (2 eq.), and acetic acid (1 drop) were added. The mixture was stirred at room temperature for 15 min. The mixture was added with sodium triacetoxyborohydride (4 eq.) and stirred at room temperature for 2 h. Work-up and purification: LCMS showed the formation of the target compound, and showed that the raw materials were reacted completely. The reaction system was diluted by adding water, adjusted to a pH>7 with saturated sodium bicarbonate solution, extracted with dichloromethane, dried, and concentrated to yield 950 mg of crude target compound.

### Step 2: l-(2-(benzyloxy)-5-(benzylmercapto)phenyl)-N,N-dimethylmethanamine

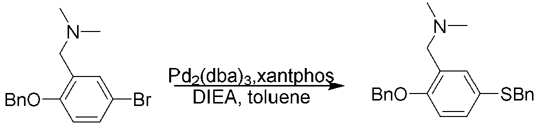

(2-(benzyloxy)-5-bromophenyl)-N,N-dimethylmethylamine (950 mg) was weighed out and placed into a single-neck flask, and then Pd₂(dba)₃ (0.1 eq.), Xantphos (0.2 eq.), diisopropylethylamine (2 eq.), toluene (15 mL), and benzyl mercaptan (1.5 eq.) were added. The mixture was stirred at 110 °C overnight under nitrogen protection. Work-up and purification: LCMS confirmed the formation of the target compound, and confirmed that the raw materials were reacted completely. The reaction system was filtered, the filter cake was rinsed with dichloromethane, and the filtrate was concentrated to yield a crude product. The crude product was purified by flash column chromatography (dichloromethane/methanol) to yield 790 mg of pure target compound.

### Step 3: 4-(benzyloxy)-3-((dimethylamino)methyl)benzenesulfonamide

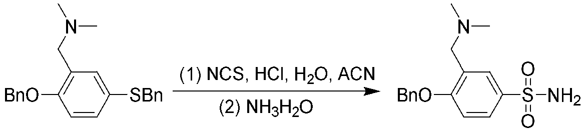

NCS (4 eq.) was weighed out and placed into a single-neck flask, and then acetonitrile (10mL) and 6M HCl (5 mL) were added. The mixture was stirred at 0 °C for 15 min. 1-(2-(benzyloxy)-5-(benzylmercapto)phenyl)-N,N-dimethylmethylamine (790 mg) was added and the mixture was stirred at 0 °C for 1 h. The reaction system was added dropwise to aqueous ammonia (40 mL) slowly, and stirred at room temperature for 1 h. Work-up and purification: LCMS confirmed the formation of the target compound. An appropriate amount of DCM was added to extract the reaction system three times, and the organic phases were combined, washed with saturated brine once, and concentrated to yield 666 mg of crude product.

### Step 4: 4-(benzyloxy)-3-((dimethylamino)methyl)-N-((1,2,3,5,6,7-hexahydro-s-indacen-4-yl)carbamoyl)benzenesulfonamide

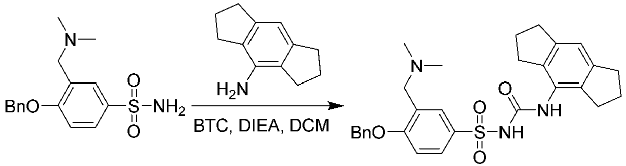

1,2,3,5,6,7-hexahydro-s-indacen-4-amine (1 eq.) was weighed out and placed into a single-neck flask. Then DCM (6mL) and DIEA (3 eq.) were added, and BTC (0.5 eq.) was added at 0°C. The mixture was stirred at 50 °C for 1 h (called system A). 4-(benzyloxy)-3-((dimethylamino)methyl)benzenesulfonamide (666 mg) was weighed out and placed into a single-neck flask, and then DCM (6 mL) and DIEA (3 eq.) were added. The mixture was stirred at room temperature for 1h (called system B). System B was slowly added to system A at room temperature, and the mixture was stirred at 50 °C for 3 h. Work-up and purification: LCMS confirmed the formation of the target compound, and confirmed that the raw materials were reacted completely. The reaction system was concentrated and purified by flash column chromatography (TFA.H₂O/MeCN) to yield a crude product. The crude product was purified by flash column chromatography (NH₄HCO₃.H₂O/MeCN) and lyophilized to yield 313 mg of white solid product. MS: 520 [M+H]⁺. ¹H NMR (400 MHz, DMSO-*d*₆) δ 9.52 (s, 1H), 8.42 (s, 1H), 8.11 (d, *J* = 2.1 Hz, 1H), 8.01 (dd, *J* = 8.8, 2.1 Hz, 1H), 7.52 (d, *J* = 7.4 Hz, 2H), 7.44 - 7.35 (m, 4H), 6.92 (s, 1H), 5.32 (s, 2H), 4.38 (s, 2H), 2.76 (d, *J* = 7.3 Hz, 10H), 2.53 (d, *J* = 7.3 Hz, 4H), 1.94 - 1.88 (m, 4H).

### Example 5: 3-((dimethylamino)methyl)-N-((1,2,3,5,6,7-hexahydro-s-indacen-4-yl)carbamoyl)-4-hydroxybenzenesulfonamide

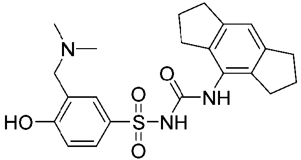

### Synthetic route:

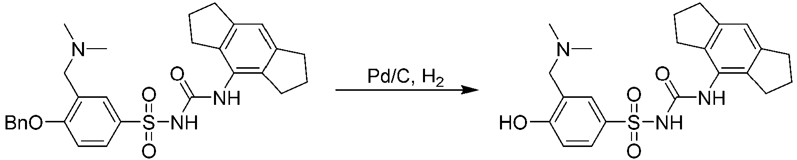

5-bromo-2-methoxybenzaldehyde (3.5 g) was weighed out and placed into a single-neck flask, and then anhydrous ethanol (100 mL) and palladium on carbon (0.2 eq.) were added. The mixture was stirred at room temperature for 1 h under hydrogen protection. Work-up and purification: LCMS showed the formation of the target compound, and showed that the raw materials were reacted completely. The reaction system was filtered, and the filter cake was rinsed three times with ethanol and concentrated to yield a crude product. The crude product was purified by flash column chromatography (NH₄HCO₃.H₂O/MeCN) and lyophilized to yield 512 mg of white solid product. MS: 430 [M+H]⁺. ¹H NMR (400 MHz, DMSO-*d*₆) δ 7.87 (s, 1H), 7.69 - 7.61 (m, 2H), 6.90 - 6.81 (m, 2H), 3.65 (s, 2H), 2.76 (t, *J* = 7.3 Hz, 4H), 2.54 (t, *J* = 7.2 Hz, 4H), 2.28 (s, 6H), 1.89 (dd, *J* = 14.4, 7.0 Hz, 4H).

### Example 6: 3-((dimethylamino)methyl)-4-fluoro-N-((1,2,3,5,6,7-hexahydro-s-indacen-4-yl)carbamoyl)benzenesulfonamide

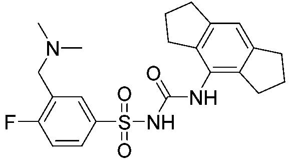

### Synthetic route:

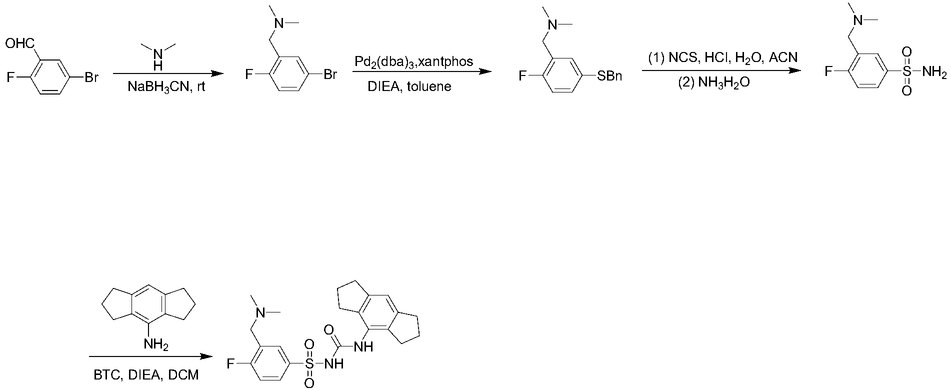

### Step 1: (5-bromo-2-fluorobenzene)-N,N-dimethylmethylamine

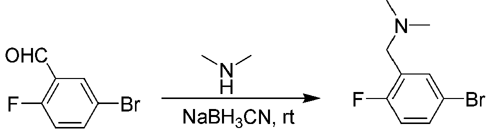

5-bromo-2-fluorobenzaldehyde (2.0 g) was weighed out and placed into a single-neck flask, and then anhydrous methanol (30 mL) and a solution of dimethylamine in tetrahydrofuran (2 eq.) were added. The mixture was stirred at room temperature overnight. The mixture was added with sodium cyanoborohydride (2 eq.) and stirred at room temperature for 1 h. Work-up and purification: LCMS showed the formation of the target compound, and showed that the raw materials were reacted completely. The system was mixed with silica gel and purified by normal-phase flash column chromatography (dichloromethane/methanol) to yield 0.88 g of the target compound.

### Step 2: 1-(5-(benzylmercapto)-2-fluorobenzene)-N,N-dimethylmethanamine

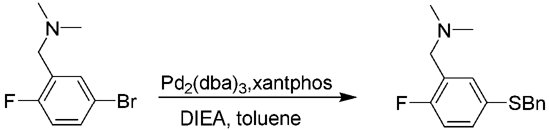

1-(5-bromo-2-fluorophenyl)-N,N-dimethylmethylamine (600 mg) was weighed out and placed into a single-neck flask, and then Pd₂(dba)₃ (0.1 eq.), Xantphos (0.2 eq.), diisopropylethylamine (2 eq.), toluene (10 mL), and benzyl mercaptan (1.5 eq.) were added. The mixture was stirred at 110 °C overnight under nitrogen protection. Work-up and purification: LCMS confirmed the formation of the target compound, and confirmed that the raw materials were reacted completely. The reaction system was filtered, the filter cake was rinsed with dichloromethane, and the filtrate was concentrated to yield a crude product. The crude product was purified by flash column chromatography (dichloromethane/methanol) to yield 560 mg of pure target compound.

### Step 3: 3-((dimethylamino)methyl)-4-fluorobenzenesulfonamide

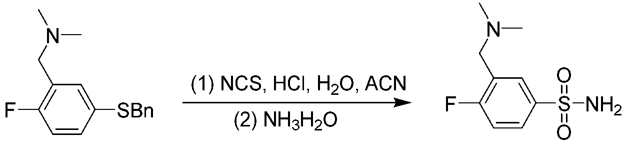

NCS (4 eq.) was weighed out and placed into a single-neck flask, and then acetonitrile (6 mL) and 6 M HCl (3 mL) were added. The mixture was stirred at 0 °C for 15 min. 1-(5-(benzylmercapto)-2-fluorobenzene)-N,N-dimethylmethylamine (540 mg) was added and the mixture was stirred at 0 °C for 1 h. The reaction system was added dropwise to aqueous ammonia (25 mL) slowly, and stirred at room temperature for 1 h. Work-up and purification: LCMS confirmed the formation of the target compound. An appropriate amount of DCM was added to extract the reaction system three times, and the organic phases were combined, washed with saturated brine once, and concentrated to yield 600 mg of crude product.

### Step 4: 3-((dimethylamino)methyl)-4-fluoro-N-((1,2,3,5,6,7-hexahydro-s-indacen-4-yl)carbamoyl)benzenesulfonamide

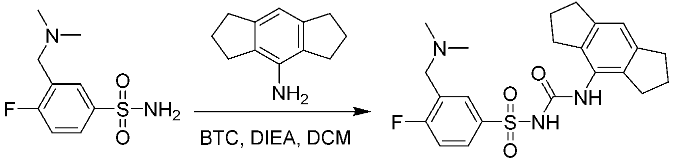

1,2,3,5,6,7-hexahydro-s-indacen-4-amine (1 eq.) was weighed out and placed into a single-neck flask, then DCM (10mL) and DIEA (3 eq.) were added, and triphosgene (0.5 eq.) was added at 0 °C. The mixture was stirred at 50 °C for 1 h (called system A). 3-((dimethylamino)methyl)-4-fluorobenzenesulfonamide (580 mg) was weighed out and placed into a single-neck flask, and then DCM (10 mL) and DIEA (3 eq.) were added. The mixture was stirred at room temperature for 1 h (called system B). System B was slowly added to system A at room temperature, and the mixture was stirred at 50°C for 3h. Work-up and purification: LCMS confirmed the formation of the target compound, and confirmed that the raw materials were reacted completely. The reaction system was concentrated and purified by flash column chromatography (TFA.H₂O/MeCN) to yield a crude product. The crude product was purified by flash column chromatography (NH₄HCO₃.H₂O/MeCN) and lyophilized to yield 336 mg of white solid product. MS: 432 [M+H]⁺. ¹H NMR (400 MHz, DMSO-*d*₆) δ 10.23 (s, 1H), 8.74 (s, 1H), 8.28 (dd, *J =* 6.7, 2.4 Hz, 1H), 8.12 (ddd, *J* = 8.7, 4.7, 2.4 Hz, 1H), 7.67 - 7.56 (m, 1H), 6.92 (s, 1H), 4.44 (d, *J =* 9.8 Hz, 2H), 2.77 (d, *J =* 5.0 Hz, 10H), 2.54 (d, *J =* 7.2 Hz, 3H), 1.93 (tt, *J =* 14.8, 7.5 Hz, 5H).

### Example 7: 4-amino-3-((dimethylamino)methyl)-N-((1,2,3,5,6,7-hexahydro-s-indacen-4-yl)carbamoyl)benzenesulfonamide

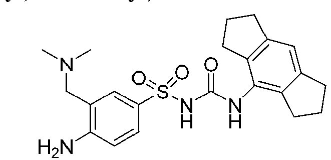

### Synthetic route:

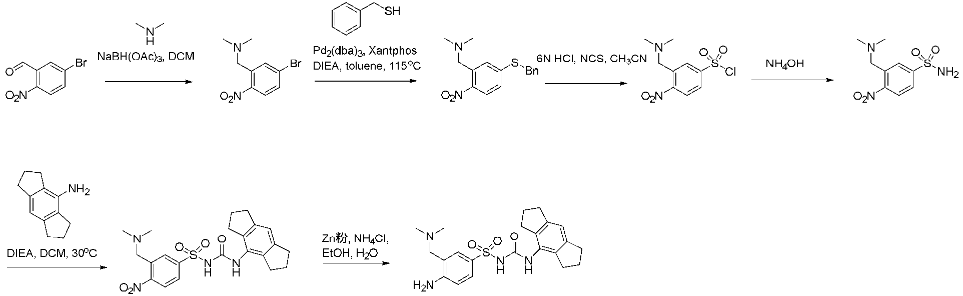

### Step 1: 1-(5-bromo-2-nitrophenyl)-N,N-dimethylmethylamine

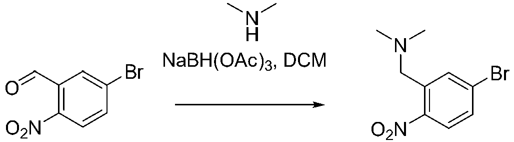

5-bromo-2-nitrobenzaldehyde (1.5 g) was weighed out and placed into a 100 mL single-neck flask, then a THF solution of dimethylamine (6.5 mL, 2 M) and DCM (50 mL) were added. NaBH(OAc)₃ (6.9 g) was added batchwise, and two drops of acetic acid was added. The mixture was stirred at room temperature for 5 h. Work-up and purification: LCMS showed the formation of the target compound, and showed that the raw materials were reacted completely. The system was diluted by adding an appropriate amount of water, extracted three times with DCM, and washed with saturated brine. The organic phase was dried with anhydrous sodium sulfate, and concentrated. Crude target compound, 1.3 g of white solid, was yielded, which was directly used in the next step without further purification.

### Step 2: 1-(5-(benzylmercapto)-2-nitrophenyl)-N,N-dimethylmethanamine

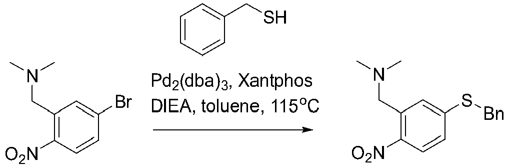

1-(5-bromo-2-nitrophenyl)-N,N-dimethylmethylamine (1.3 g) was weighed out and placed into a 100 mL single-neck flask, and then benzyl mercaptan (750 mg), Pd₂(dba)₃ (231 mg), XantPhos (175 mg), DTEA (1.3 g), and toluene (50 mL) were added. The mixture was heated to 115 °C and then stirred overnight. Work-up and purification: TLC confirmed that the raw materials were reacted completely. The reaction system was diluted by adding an appropriate amount of water, the aqueous layer was extracted three times with ethyl acetate, and the organic phases were combined, followed by washing twice with saturated brine, drying with anhydrous Na₂SO₄, concentration, and purification by column chromatography to yield the target compound, 730 mg of yellow solid.

### Step 3: 3-((dimethylamino)methyl)-4-nitrobenzenesulfonamide

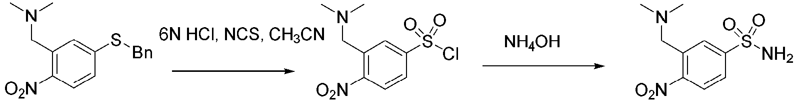

6 mL acetonitrile and 1.9 mL 6 N HCl aqueous solution were placed into a 25 mL single-neck flask respectively. After cooling down to below 0 °C, NCS (1.28 g) was added batchwise, and then a solution of 1-(5-(benzylmercapto)-2-nitrophenyl)-N,N-dimethylmethylamine (730 mg) in acetonitrile was added dropwise. After stirring at room temperature for 1 h, TLC confirmed that the raw materials were reacted completely. The resultant mixture was added dropwise to aqueous ammonia (30 mL) slowly, and stirring was continued overnight. Work-up and purification: TLC confirmed that the raw materials were reacted completely. After concentration and purification by column chromatography (dichloromethane/methanol), 510 mg of white solid compound was obtained as the target product.

### Step 4: 3-((dimethylamino)methyl)-N-((1,2,3,5,6,7-hexahydro-s-indacene-yl)carbamoyl)-4-nitrobenzenesulfonamide

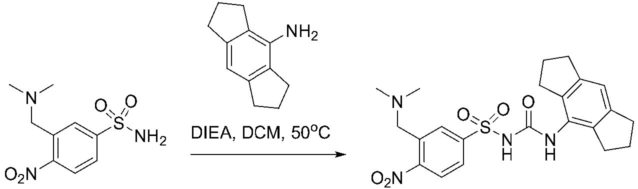

3-((dimethylamino)methyl)-4-nitrobenzenesulfonamide (510 mg), 1,2,3,5,6,7-hexahydro-s-indacen-4-amine (341 mg), DIEA (508 mg), and DCM (50 mL) were weighed out and placed into a 100 mL single-neck flask, and triphosgene (583 mg) was added batchwise at room temperature. The mixture was heated to 50 °C and stirred for 12 h. Work-up and purification: TLC confirmed that the raw materials were reacted completely. After concentration and purification by column chromatography (petroleum ether/ethyl acetate, gradient of 0-100% ethyl acetate), 173 mg of yellow solid compound was obtained as the target product.

### Step 5: 4-amino-3-((dimethylamino)methyl)-N-((1,2,3,5,6,7-hexahydro-s-indacen-4-yl)carbamoyl)benzenesulfonamide

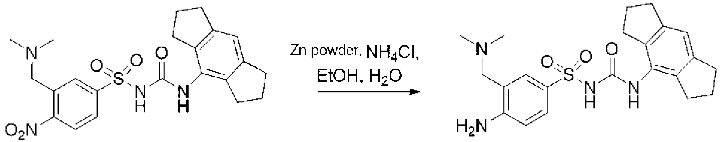

3-((dimethylamino)methyl)-N-((1,2,3,5,6,7-hexahydro-s-indacen-yl)carbamoyl)-4-nitrobenzenesulfonamide (120 mg), zinc powder (95 mg), saturated NH₄Cl solution (5 mL), and ethanol (15 mL) were weighed out and placed into a 50 mL single-neck flask, and stirred under reflux overnight. Work-up and purification: LCMS confirmed the formation of the target compound, and confirmed that the raw materials were reacted completely. The mixture was concentrated, the aqueous layer was extracted three times with ethyl acetate, and the organic phases were combined, dried, and concentrated. After preparative purification by prep-HPLC and lyophilization, 85 mg of white solid product, trifluoroacetate, was obtained. MS: 429 [M+H]⁺. ¹H NMR (400 MHz, DMSO) δ 8.82 (s, 1H), 7.66 - 7.45 (m, 2H), 6.89 (s, 1H), 6.72 (d, *J =* 8.6 Hz, 1H), 3.34 (s, 6H), 2.76 (t, *J =* 7.3 Hz, 4H), 2.55 (t, *J =* 7.3 Hz, 4H), 2.50 (s, 2H), 2.06 - 1.82 (m, 6H).

### Example 8: 4-chloro-3-((dimethylamino)methyl)-N-((1,2,3,5,6,7-hexahydro-s-indacen-4-yl)carbamoyl)benzenesulfonamide

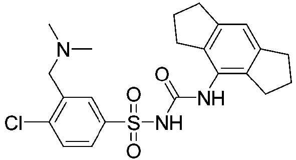

### Synthetic route:

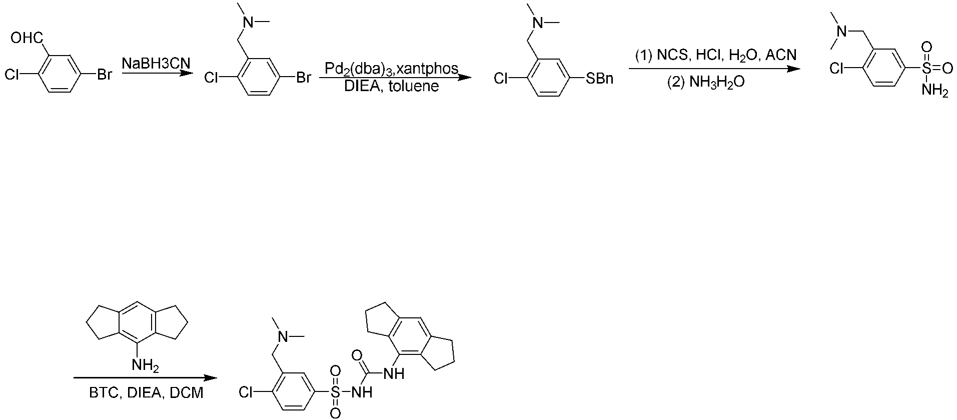

### Step 1: (5-bromo-2-chlorophenyl)-N,N-dimethylmethanamine

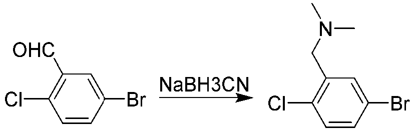

5-bromo-2-chlorobenzaldehyde (2.0 g) was weighed out and placed into a single-neck flask, and then anhydrous methanol (30 mL) and a solution of dimethylamine in tetrahydrofuran (2 eq.) were added. The mixture was stirred at room temperature overnight. The mixture was added with sodium cyanoborohydride (2 eq.) and stirred at room temperature for 1h. Work-up and purification: LCMS showed the formation of the target compound, and showed that the raw materials were reacted completely. The system was mixed with silica gel and purified by normal-phase flash column chromatography (dichloromethane/methanol) to yield 1.3 g of the target compound.

### Step 2: l-(5-(benzylmercapto)-2-chlorophenyl)-N,N-dimethylmethanamine

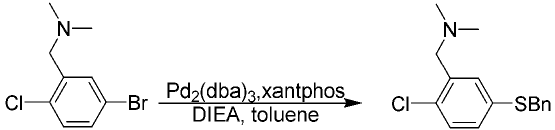

1-(5-bromo-2-chlorophenyl)-N,N-dimethylmethylamine (800 mg) was weighed out and placed into a single-neck flask, and then Pd₂(dba)₃ (0.1 eq.), Xantphos (0.2 eq.), diisopropylethylamine (2 eq.), toluene (10 mL), and benzyl mercaptan (1.5eq.) were added. The mixture was stirred at 110 °C overnight under nitrogen protection. Work-up and purification: LCMS confirmed the formation of the target compound, and confirmed that the raw materials were reacted completely. The reaction system was filtered, the filter cake was rinsed with dichloromethane, and the filtrate was concentrated to yield a crude product. The crude product was purified by flash column chromatography (dichloromethane/methanol) to yield 840 mg of pure target compound.

### Step 3: 4-chloro-3-((dimethylamino)methyl)benzenesulfonamide

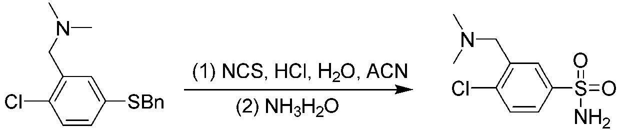

NCS (4 eq.) was weighed out and placed into a single-neck flask, and then acetonitrile (8 mL) and 6 M HCl (4 mL) were added. The mixture was stirred at 0 °C for 15 min. 1-(5-(benzylmercapto)-2-chlorophenyl)-N,N-dimethylmethylamine (840 mg) was added and the mixture was stirred at 0 °C for 1 h. The reaction system was added dropwise to aqueous ammonia (35 mL) slowly, and stirred at room temperature for 1 h. Work-up and purification: LCMS confirmed the formation of the target compound. An appropriate amount of DCM was added to extract the reaction system three times, and the organic phases were combined, washed with saturated brine once, and concentrated to yield 810 mg of crude product.

### Step 4: 4-chloro-3-((dimethylamino)methyl)-N-((1,2,3,5,6,7-hexahydro-s-indacen-4-yl)carbamoyl)benzenesulfonamide

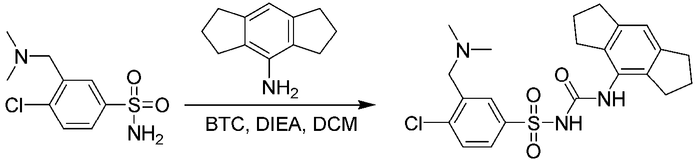

1,2,3,5,6,7-hexahydro-s-indacen-4-amine (1 eq.) was weighed out and placed into a single-neck flask, then DCM (10 mL) and DIEA (3 eq.) were added, and triphosgene (0.5 eq.) was added at 0 °C. The mixture was stirred at 50 °C for 1 h (called system A). 4-chloro-3-((dimethylamino)methyl)benzenesulfonamide (800 mg) was weighed out and placed into a single-neck flask, and then DCM (10 mL) and DIEA (3 eq.) were added. The mixture was stirred at room temperature for 1 h (called system B). System B was slowly added to system A at room temperature, and the mixture was stirred at 50 °C for 3 h. Work-up and purification: LCMS confirmed the formation of the target compound, and confirmed that the raw materials were reacted completely. The reaction system was concentrated and purified by flash column chromatography (TFA.H₂O/MeCN) to yield a crude product. The crude product was purified by flash column chromatography (NH₄HCO₃.H₂O/MeCN) and lyophilized to yield 89 mg of white solid product. MS: 448 [M+H]⁺. ¹H NMR (400 MHz, DMSO-*d*₆) δ10.02 (s, 1H), 8.77 (s, 1H), 8.31 (d, *J* = 2.1 Hz, 1H), 8.03 (dd, *J* = 8.5, 2.2 Hz, 1H), 7.88 (d, *J* = 8.5 Hz, 1H), 6.92 (s, 1H), 4.53 (s, 2H), 2.78 (d, *J* = 12.8 Hz, 10H), 2.54 (d, *J* = 7.2 Hz, 3H), 1.92 (h, *J* = 8.1, 7.5 Hz, 4H).

### Example 9: 3-((dimethylamino)methyl)-N-((1,2,3,5,6,7-hexahydro-s-indacen-4-yl)carbamoyl)-4-(trifluoromethoxy)benzenesulfonamide

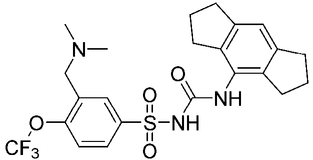

### Synthetic route:

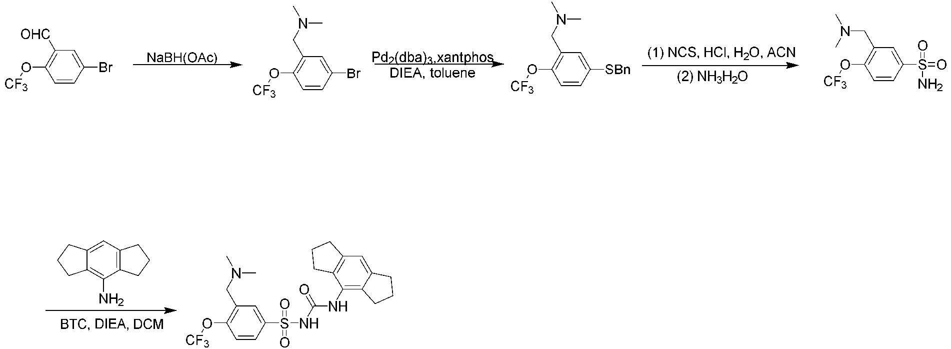

### Step 1: l-(5-bromo-2-(trifluoromethoxy)phenyl)-N,N-dimethylmethanamine

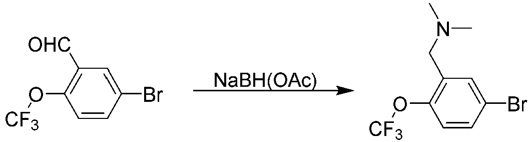

5-bromo-2-(trifluoromethoxy)benzaldehyde (200 mg) was weighed out and placed into a single-neck flask, and then DCM (10 mL), a solution of dimethylamine in tetrahydrofuran (2 eq.), and acetic acid (1 drop) were added. The mixture was stirred at room temperature for 15 min. STAB (4 eq.) was added, and the mixture was stirred at room temperature for 2 h. Work-up and purification: LCMS showed the formation of the target compound, and showed that the raw materials were reacted completely. The system was diluted by adding an appropriate amount of DCM, and adjusted to a pH>7 with saturated sodium bicarbonate solution, followed by washing three times with water and once with saturated brine. The organic phase was dried and concentrated. 220 mg of crude target compound was obtained.

### Step 2: l-(5-(benzylmercapto)-2-(trifluoromethoxy)phenyl)-N,N-dimethylmethanamine

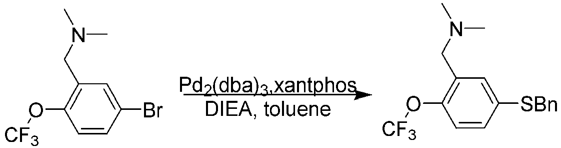

1-(5-bromo-2-(trifluoromethoxy)phenyl)-N,N-dimethylmethylamine (200 mg) was weighed out and placed into a single-neck flask, and then Pd₂(dba)₃ (0.1 eq.), Xantphos (0.2 eq.), diisopropylethylamine (2 eq.), toluene (10 mL), and benzyl mercaptan (1.5 eq.) were added. The mixture was stirred at 110 °C overnight under nitrogen protection. Work-up and purification: LCMS confirmed the formation of the target compound, and confirmed that the raw materials were reacted completely. The reaction system was filtered, the filter cake was rinsed with dichloromethane, and the filtrate was concentrated to yield a crude product. The crude product was purified by flash column chromatography (dichloromethane/methanol) to yield 193 mg of pure target compound.

### Step 3: 3-((dimethylamino)methyl)-4-(trifluoromethoxy)benzenesulfonamide

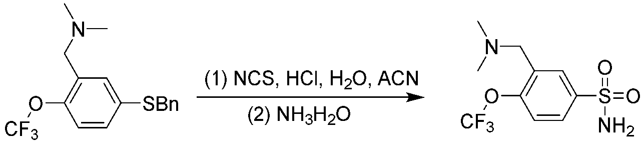

NCS (4 eq.) was weighed out and placed into a single-neck flask, and then acetonitrile (5 mL) and 6 M HCl (2 mL) were added. The mixture was stirred at 0 °C for 15 min. 1-(5-(benzylmercapto)-2-(trifluoromethoxy)phenyl)-N,N-dimethylmethylamine (160 mg) was added and the mixture was stirred at 0 °C for 1 h. The reaction system was added dropwise to aqueous ammonia (10 mL) slowly, and stirred at room temperature for 1 h. Work-up and purification: LCMS confirmed the formation of the target compound. An appropriate amount of DCM was added to extract the reaction system three times, the organic phases were combined, washed with saturated brine once, and concentrated to yield 150 mg of crude product.

### Step 4: 3-((dimethylamino)methyl)-N-((1,2,3,5,6,7-hexahydro-s-indacen-4-yl)carbamoyl)-4-(trifluoromethoxy)benzenesulfonamide

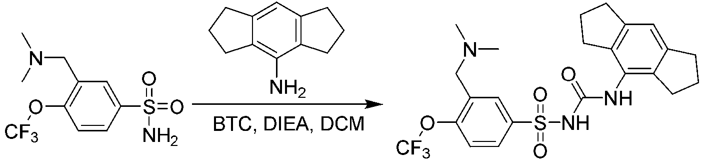

1,2,3,5,6,7-hexahydro-s-indacen-4-amine (1 eq.) was weighed out and placed into a single-neck flask, then DCM (5 mL) and DIEA (3 eq.) were added, and triphosgene (0.5 eq.) was added at 0 °C. The mixture was stirred at 50 °C for 1 h (called system A). 3-((dimethylamino)methyl)-4-(trifluoromethoxy)benzenesulfonamide (120 mg) was weighed out and placed into a 250 mL single-neck flask, and then DCM (5mL) and DIEA (3 eq.) were added. The mixture was stirred at room temperature for 1 h (called system B). System B was slowly added to system A at room temperature, and the mixture was stirred at 50 °C for 3 h. Work-up and purification: LCMS confirmed the formation of the target compound, and confirmed that the raw materials were reacted completely. The reaction system was concentrated and purified by flash column chromatography (TFA.H₂O/MeCN) to yield a crude product. The crude product was purified by flash column chromatography (NH₄HCO₃.H₂O/MeCN) and lyophilized to yield 61 mg of white solid product. MS: 498 [M+H]⁺. ¹H NMR (400 MHz, DMSO-*d*₆) δ 10.02 (s, 1H), 8.57 (s, 1H), 8.36 (d, *J* = 2.1 Hz, 1H), 8.17 (dd, *J =* 8.8, 2.1 Hz, 1H), 7.81 - 7.74 (m, 1H), 6.93 (s, 1H), 4.46 (s, 2H), 2.76 (d, *J* = 9.4 Hz, 11H), 2.52 - 2.51 (m, 3H), 1.90 (p, *J* = 7.4 Hz, 4H).

### Example 10: 2-chloro-5-((dimethylamino)methyl)-N-((1,2,3,5,6,7-hexahydro-s-indacen-4-yl)carbamoyl)benzenesulfonamide

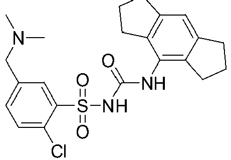

### Synthetic route:

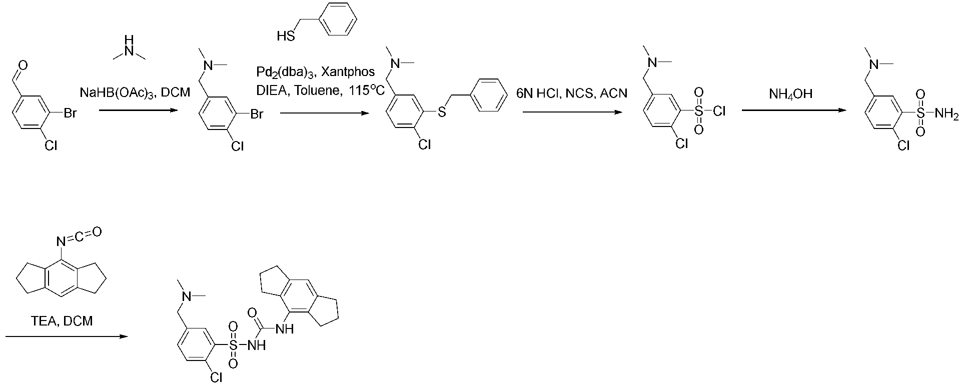

### Step 1: 1-(3-bromo-4-chlorophenyl)-N,N-dimethylmethanamine

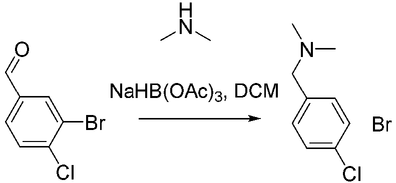

3-bromo-4-chlorobenzaldehyde (1.0 g) was weighed out and placed into a 100 mL single-neck flask, then a solution of dimethylamine in THF (2.3 mL, 2 M) and DCM (30 mL) were added, NaBH(OAc)₃ (4.84 g) was added batchwise, and two drops of acetic acid was added. The mixture was stirred at room temperature for 5 h. Work-up and purification: LCMS showed the formation of the target compound, and showed that the raw materials were reacted completely. The system was diluted by adding an appropriate amount of water, extracted three times with DCM, and washed with saturated brine. The organic phase was dried with anhydrous sodium sulfate, and concentrated. Crude target compound, 920 mg of white solid, was yielded, which was directly used in the next step without further purification.

### Step 2: 1-(5-(benzylmercapto)-2-nitrophenyl)-N,N-dimethylmethanamine

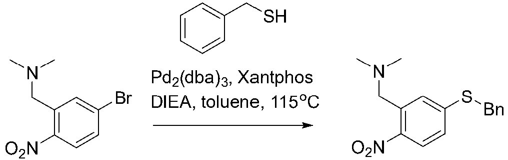

1-(3-bromo-4-chlorophenyl)-N,N-dimethylmethylamine (920 mg) was weighed out and placed into a 100 mL single-neck flask, and then benzyl mercaptan (750 mg), Pd₂(dba)₃ (231 mg), XantPhos (175 mg), DTEA (1.3 g), and toluene (50 mL) were added respectively. The mixture was heated to 115 °C and then stirred overnight. Work-up and purification: TLC confirmed that the raw materials were reacted completely. The reaction system was diluted by adding an appropriate amount of water, the aqueous layer was extracted three times with ethyl acetate, and the organic phases were combined, followed by washing twice with saturated brine, drying with anhydrous Na₂SO₄, concentration, and purification by column chromatography to yield the target compound, 380 mg of yellowish-brown solid.

### Step 3: 2-chloro-5-((dimethylamino)methyl)benzenesulfonamide

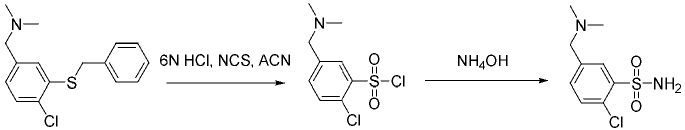

6 mL acetonitrile and 1 mL 6 N HCl aqueous solution were placed into a 25 mL single-neck flask. After cooling down to below 0 °C, NCS (694.7 mg) was added batchwise, and then a solution of 1-(5-(benzylmercapto)-2-nitrophenyl)-N,N-dimethylmethylamine (380 mg) in acetonitrile was added dropwise. After stirring at room temperature for 1h, TLC confirmed that raw materials were reacted completely. The resultant mixture was added dropwise to aqueous ammonia (20 mL) slowly, and stirring was continued overnight. Work-up and purification: TLC confirmed that the raw materials were reacted completely. After concentration and purification by column chromatography (dichloromethane/methanol), 160 mg of white solid compound was obtained as the target product.

### Step 4: 2-chloro-5-((dimethylamino)methyl)-N-((1,2,3,5,6,7-hexahydro-s-indacen-4-yl)carbamoyl)benzenesulfonamide

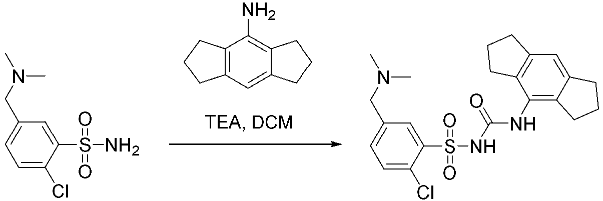

2-chloro-5-((dimethylamino)methyl)benzenesulfonamide (150 mg), 1,2,3,5,6,7-hexahydro-s-indacen-4-amine (105 mg), DIEA (195 mg), and DCM (30 mL) were weighed out and placed into a 100 mL single-neck flask, and triphosgene (174 mg) was added batchwise at room temperature. The mixture was heated to 50 °C and stirred for 12 h. Work-up and purification: LCMS confirmed the formation of the target compound, and confirmed that the raw materials were reacted completely. The mixture was concentrated, the aqueous layer was extracted three times with ethyl acetate, and the organic phases were combined, dried, and concentrated. After preparative purification by prep-HPLC (acetonitrile/water binary mobile phase, gradient of 10-100% acetonitrile, the mobile phase was added with 0.05% trifluoroacetic acid) and lyophilization, 160 mg of white solid product, trifluoroacetate, was obtained. MS: 448 [M+H]⁺. ¹H NMR (400 MHz, DMSO) δ 8.59 (s, 1H), 8.13 (s, 1H), 8.08 (s, 1H), 7.62 - 7.55 (m, 2H), 6.85 (s, 1H), 3.90 (s, 2H), 2.74 (t, *J* = 7.3 Hz, 4H), 2.58 (t, *J* = 7.0 Hz, 4H), 2.42 (s, 6H), 1.92 - 1.85 (m, 4H).

### Example 11: 5-((dimethylamino)methyl)-N-((1,2,3,5,6,7-hexahydro-s-indacen-4-yl)carbamoyl)-2-nitrobenzenesulfonamide

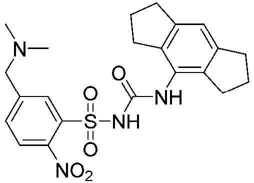

### Synthetic route:

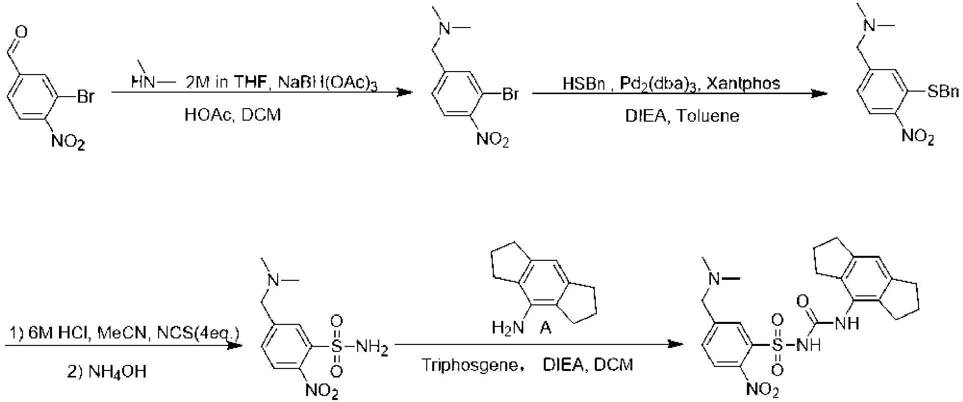

### Step 1: 1-(3-bromo-4-nitrophenyl)-N,N-dimethylmethylamine

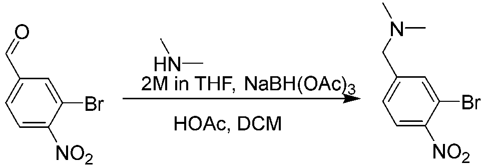

3-bromo-4-nitrobenzaldehyde (1.0 g) was weighed out and placed into a 100 mL single-neck flask, then DCM (30 mL) was added, and dimethylamine (2 M in THF) (2 eq.) and HOAc (0.2 eq.) were added while stirring. The mixture was stirred at room temperature for 2 h. 2 h later, NaBH(OAc)₃ (3 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 v/v (30 mL), the organic phases were combined, washed with saturated brine, dried and rotary evaporated, and passed through flash column chromatography (DCM/MeOH) to yield a crude target compound, 1.0 g of yellow oily liquid.

### Step 2: 1-(3-(benzylmercapto)-4-nitrophenyl)-N,N-dimethylmethanamine

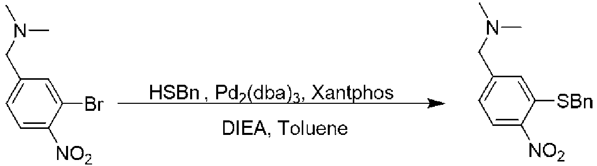

1-(3-bromo-4-nitrophenyl)-N,N-dimethylmethylamine (1.0 g) was weighed out and placed into a 40 mL microwave tube, and then toluene (15 mL), benzyl mercaptan (2 eq.), DIEA (3eq.), Pd₂(dba)₃ (0.1 eq.), and Xantphos (0.2 eq.) were added under a nitrogen atmosphere. The mixture was heated to 115 °C to react for 12 h. LCMS showed the formation of the target compound. Work-up and purification: The reaction solution was mixed with silica gel, rotary evaporated, and passed through flash column chromatography (DCM/MeOH) to yield a crude target compound, 1.0 g of yellow oily liquid.

### Step 3: 5-((dimethylamino)methyl)-2-nitrobenzenesulfonamide

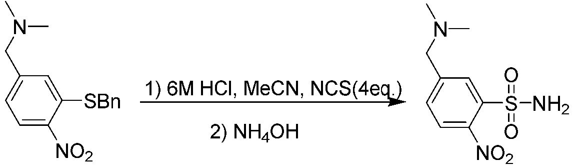

6 M HCl (2.8 mL) was measured out and placed into a 50 mL single-neck flask, and MeCN (15 mL) was added. The mixture was stirred and cooled to 0 °C, and added with NCS (4 eq.). 1-(3-(benzylmercapto)-4-nitrophenyl)-N,N-dimethylmethylamine (1.0 g) was dissolved in 10 mL MeCN, and then added dropwise into the above reaction solution at 0 °C. The mixture was stirred for 0.5 h. NH₄OH (50 mL) was placed into another 100 mL single-neck flask, and the above reaction solution was added dropwise into NH₄OH via a constant-pressure dropping funnel while stirring at room temperature. The mixture was stirred for 0.5 h. LCMS confirmed the formation of the target compound. Work-up and purification: The reaction solution was rotary evaporated to remove MeCN, and extracted twice with DCM:MeOH=10:1 v/v (50 mL), followed by drying and concentration under reduced pressure to yield the target compound, 1.2 g of yellow oily crude product.

### Step 4: 5-((dimethylamino)methyl)-N-((1,2,3,5,6,7-hexahydro-s-indacen-4-yl)carbamoyl)-2-nitrobenzenesulfonamide

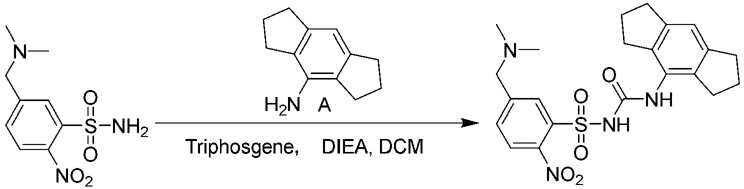

1,2,3,5,6,7-hexahydro-s-indacen-4-amine (400 mg) was weighed out and placed into a 40 mL single-neck flask. DCM (10 mL) and DIEA (5 eq.) were added, and triphosgene (0.4 eq.) was added batchwise while stirring at room temperature. The reaction solution was heated to 50 °C to react for 1 h. 800 mg of crude 5-((dimethylamino)methyl)-2-nitrobenzenesulfonamide was dissolved in DCM (5 mL) and DIEA (3 eq.). Once fully dissolved, the solution was added dropwise into the above reaction solution at 50 °C. The reaction was carried out at 50 °C for 2 h. LCMS confirmed the formation of the target compound. Work-up and purification: The reaction solution was directly mixed with silica gel and passed through flash column chromatography (DCM/MeOH) to yield a crude product. The crude product was subjected to preparative purification by flash column chromatography (acetonitrile/water gradient elution, 0-100% acetonitrile, the mobile phase was added with 0.05% NH₄HCO₃) and lyophilization to yield the target compound, 240 mg of white solid. MS: 459 [M+H]⁺. ¹H NMR (400 MHz, DMSO-*d*₆) δ 9.70 (s, 1H), 8.17 (s, 1H), 7.77 (d, *J* = 8.2 Hz, 1H), 7.66 (d, *J* = 8.2 Hz, 2H), 6.80 (s, 1H), 4.13 (s, 2H), 2.74 (t, *J* = 7.6 Hz, 4H), 2.63 (t, *J* = 7.4 Hz, 4H), 2.57 (s, 6H), 1.93~1.83 (m, 4H).

### Example 12: 2-amino-5-((dimethylamino)methyl)-N-((1,2,3,5,6,7-hexahydro-s-indacen-4-yl)carbamoyl)benzenesulfonamide

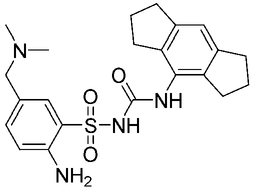

### Synthetic route:

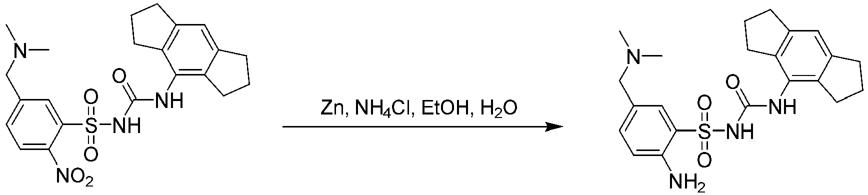

5-((dimethylamino)methyl)-N-((1,2,3,5,6,7-hexahydro-s-indacen-4-yl)carbamoyl)-2-nitrobenzenesulfonamide (140 mg) was weighed out and placed into a 40 mL single-neck flask. EtOH (6 mL) and H₂O (3 mL) were added, and zinc powder (15 eq.) and ammonium chloride (5 eq.) were added while stirring at room temperature. After the addition, the reaction solution was heated to 60 °C to react for 4 h. LCMS confirmed the formation of the target compound, and confirmed that the raw materials were reacted completely. Work-up and purification: The reaction solution was filtered with celite. The filter cake was rinsed with 100 mL of DCM/MeOH=10/1 v/v, the filtrate was concentrated, and when a small amount remained, the crude product was passed through flash column chromatography. The crude product was subjected to preparative purification by flash column chromatography (0.05% NH₄HCO₃) and lyophilization to yield the target compound, 240 mg of white solid. MS: 429 [M+H]⁺. ¹H NMR (400 MHz, DMSO-*d*₆) δ 9.52 (s, 1H), 8.20 - 7.96 (m, 1H), 7.65 - 7.55 (m, 1H), 7.34 - 7.19 (m, 1H), 7.00 - 6.71 (m, 2H), 6.33 (s, 1H), 3.94 (s, 1H), 3.85 (s, 1H), 2.79 - 2.68 (m, 4H), 2.60 - 2.53 (m, 4H), 2.50 (s, 6H), 1.99 - 1.86 (m, 4H).

### Example 13: 3-((dimethylamino)methyl)-N-((1,2,3,5,6,7-hexahydro-s-indacen-4-yl)carbamoyl)-4-methoxybenzenesulfonamide

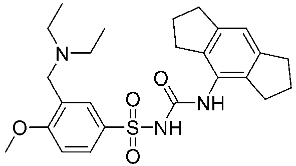

### Synthetic route:

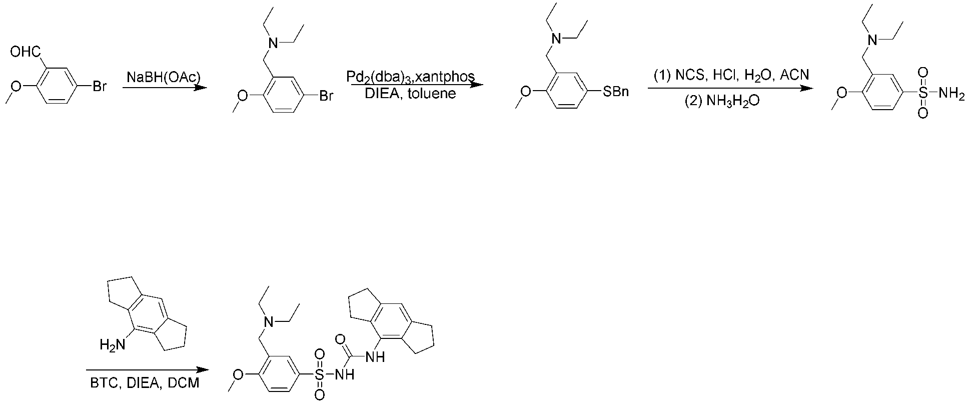

### Step 1: N-(5-bromo-2-methoxybenzyl)-N-ethylethanamine

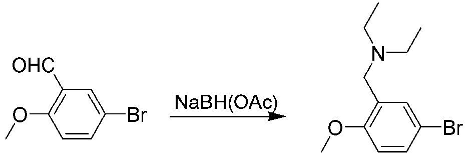

5-bromo-2-methoxybenzaldehyde (1 g) was weighed out and placed into a single-neck flask, and then dichloromethane (30 mL), diethylamine (5 eq.), and acetic acid (1 drop) were added. The mixture was stirred at room temperature for 15 min. The mixture was added with sodium triacetoxyborohydride (5 eq.) and stirred at room temperature overnight. Work-up and purification: LCMS showed the formation of the target compound, and showed that the raw materials were reacted completely. The reaction system was diluted by adding water, adjusted to a pH>7 with saturated sodium bicarbonate solution. The system was extracted with dichloromethane, dried, and concentrated to yield 1.3 g of crude target compound.

### Step 2: N-(5-(benzylmercapto)-2-methoxybenzyl)-N-ethylethanamine

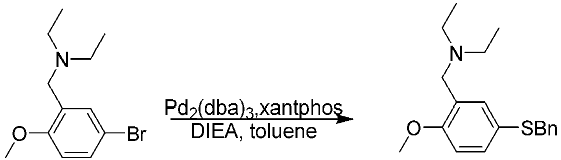

N-(5-bromo-2-methoxybenzyl)-N-ethylethylamine (1 g) was weighed out and placed into a single-neck flask, and then Pd₂(dba)₃ (0.1 eq.), Xantphos (0.2 eq.), diisopropylethylamine (2 eq.), toluene (15 mL), and benzyl mercaptan (1.5 eq.) were added. The mixture was stirred at 110 °C overnight under nitrogen protection. Work-up and purification: LCMS confirmed the formation of the target compound, and confirmed that the raw materials were reacted completely. The reaction system was filtered, the filter cake was rinsed with dichloromethane, and the filtrate was concentrated to yield a crude product. The crude product was purified by flash column chromatography (dichloromethane/methanol) to yield 900 mg of pure target compound.

### Step 3: 3-((diethylamino)methyl)-4-methoxybenzenesulfonamide

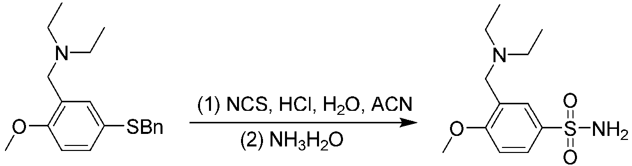

NCS (4 eq.) was weighed out and placed into a single-neck flask, and then acetonitrile (10 mL) and 6M HCl (5 mL) were added. The mixture was stirred at 0 °C for 15 min. N-(5-(benzylmercapto)-2-methoxybenzyl)-N-ethylethylamine (800 mg) was added and the mixture was stirred at 0 °C for 1 h. The reaction system was added dropwise to aqueous ammonia (40 mL) slowly, and stirred at room temperature for 1 h. Work-up and purification: LCMS confirmed the formation of the target compound. An appropriate amount of DCM was added to extract the reaction system three times, and the organic phases were combined, washed once with saturated brine, and concentrated to yield 621 mg of crude product.

### Step 4: ((diethylamino)methyl)-N-((1,2,3,5,6,7-hexahydro-s-indacen-4-yl)carbamoyl)-4-methoxybenzenesulfonamide

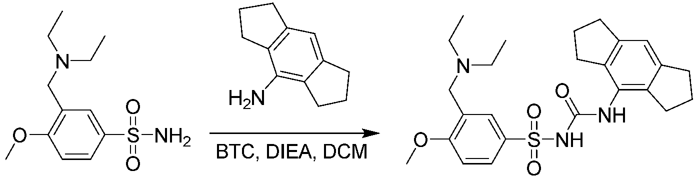

1,2,3,5,6,7-hexahydro-s-indacen-4-amine (1 eq.) was weighed out and placed into a single-neck flask, then DCM (6 mL) and DIEA (3 eq.) were added, and BTC (0.5 eq.) was added at 0 °C. The mixture was stirred at 50 °C for 1h (called system A). 3-((diethylamino)methyl)-4-methoxybenzenesulfonamide (500 mg) was weighed out and placed into a single-neck flask, and then DCM (6 mL) and DIEA (3 eq.) were added. The mixture was stirred at room temperature for 1 h (called system B). System B was slowly added to system A at room temperature, and the mixture was stirred at 50 °C for 3 h. Work-up and purification: LCMS confirmed the formation of the target compound, and confirmed that the raw materials were reacted completely. The reaction system was concentrated and purified by flash column chromatography (TFA.H₂O/MeCN) to yield a crude product. The crude product was purified by flash column chromatography (NH₄HCO₃.H₂O/MeCN) and lyophilized to yield 40 mg of white solid product. MS: 472 [M+H]⁺. ¹H NMR (400 MHz, DMSO-*d*₆) δ 9.05 (s, 1H), 8.48 (s, 1H), 8.12 - 7.89 (m, 2H), 7.40 - 7.29 (m, 1H), 6.92 (s, 1H), 4.33 (dd, *J* = 11.0, 5.0 Hz, 2H), 3.94 (d, *J* = 5.8 Hz, 3H), 3.09 (dd, *J* = 11.4, 6.1 Hz, 4H), 2.76 (d, *J* = 7.4 Hz, 4H), 2.55 (d, *J* = 7.3 Hz, 2H), 1.91 (p, *J* = 7.4 Hz, 4H), 1.23 (dt, *J* = 14.4, 7.2 Hz, 8H).

### Example 14: 3-((dimethylamino)methyl)-4-ethoxy-N-((1,2,3,5,6,7-hexahydro-s-indacen-4-yl)carbamoyl)benzenesulfonamide

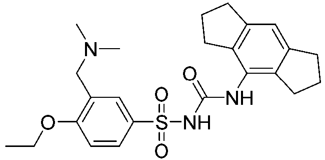

### Synthetic route:

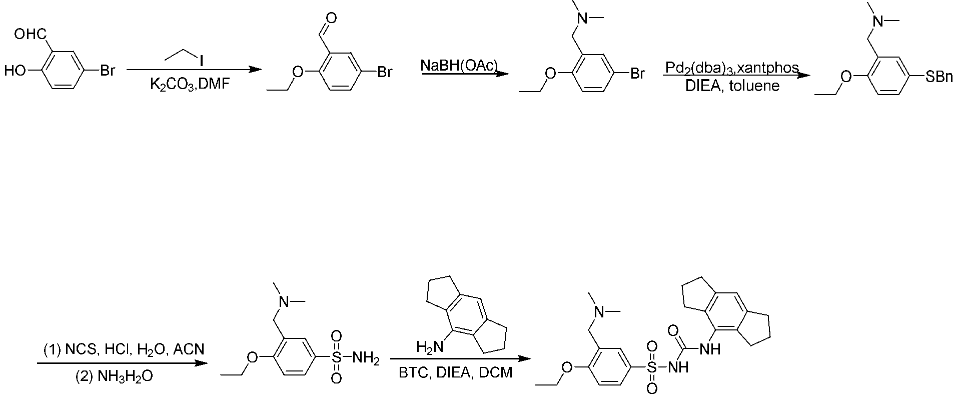

### Step 1: 5-bromo-2-ethoxybenzaldehyde

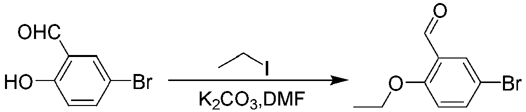

5-bromo-2-hydroxybenzaldehyde (1.0 g) was weighed out and placed into a single-neck flask, and then DMF (20 mL), potassium carbonate (2 eq.), and ethyl iodide (1.2 eq.) were added. The mixture was stirred at 60 °C for 2 h. Work-up and purification: LCMS confirmed the formation of the target compound. The system was mixed with silica gel and purified by normal-phase flash column chromatography (dichloromethane/methanol) to yield 1.1 g of the target compound.

### Step 2: (5-bromo-2-ethoxyphenyl)-N,N-dimethylmethanamine

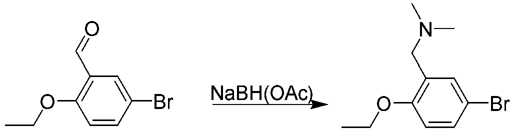

5-bromo-2-ethoxybenzaldehyde (1.0 g) was weighed out and placed into a single-neck flask, and then dichloromethane (20 mL), a solution of dimethylamine in tetrahydrofuran (2 eq.), and acetic acid (1 drop) were added. The mixture was stirred at room temperature for 15 min. The mixture was added with sodium triacetoxyborohydride (4 eq.) and stirred at room temperature for 2 h. Work-up and purification: LCMS showed the formation of the target compound, and showed that the raw materials were reacted completely. The reaction system was diluted by adding water, adjusted to a pH>7 with saturated sodium bicarbonate solution. The system was extracted with dichloromethane, dried, and concentrated to yield 1.3 g of crude target compound.

### Step 3: l-(5-(benzylmercapto)-2-ethoxyphenyl)-N,N-dimethylmethanamine

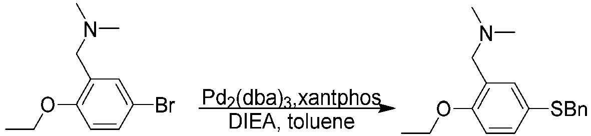

(5-bromo-2-ethoxyphenyl)-N,N-dimethylmethylamine (600 mg) was weighed out and placed into a single-neck flask, and then Pd₂(dba)₃ (0.1 eq.), Xantphos (0.2 eq.), diisopropylethylamine (2 eq.), toluene (10 mL), and benzyl mercaptan (1.5 eq.) were added. The mixture was stirred at 110 °C overnight under nitrogen protection. Work-up and purification: LCMS confirmed the formation of the target compound, and confirmed that the raw materials were reacted completely. The reaction system was filtered, the filter cake was rinsed with dichloromethane, and the filtrate was concentrated to yield a crude product. The crude product was purified by flash column chromatography (dichloromethane/methanol, ratio) to yield 660 mg of pure target compound.

### Step 4: 3-((dimethylamino)methyl)-4-ethoxybenzenesulfonamide

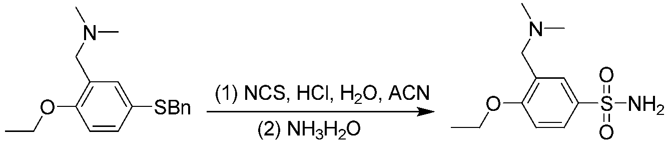

NCS (4 eq.) was weighed out and placed into a single-neck flask, and then acetonitrile (10 mL) and 6 M HCl (5 mL) were added. The mixture was stirred at 0 °C for 15 min. 1-(5-(benzylmercapto)-2-ethoxyphenyl)-N,N-dimethylmethylamine (600 mg) was added and the mixture was stirred at 0 °C for 1 h. The reaction system was added dropwise to aqueous ammonia (40 mL) slowly, and stirred at room temperature for 1 h. Work-up and purification: LCMS confirmed the formation of the target compound. An appropriate amount of DCM was added to extract the reaction system three times, and the organic phases were combined, washed once with saturated brine, and concentrated to yield 350 mg of crude product.

### Step 5: 3-((dimethylamino)methyl)-4-ethoxy-N-((1,2,3,5,6,7-hexahydro-s-indacen-4-yl)carbamoyl)benzenesulfonamide

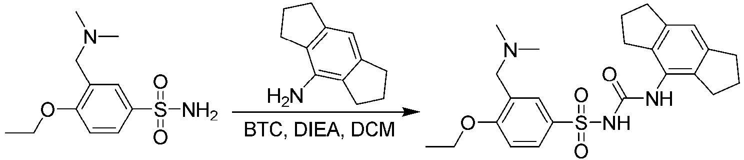

1,2,3,5,6,7-hexahydro-s-indacen-4-amine (1 eq.) was weighed out and placed into a single-neck flask. Then DCM (8 mL) and DIEA (3 eq.) were added, and BTC (0.5 eq.) was added at 0 °C. The mixture was stirred at 50 °C for 1h (called system A). 3-((dimethylamino)methyl)-4-ethoxybenzenesulfonamide (300 mg) was weighed out and placed into a single-neck flask, and then DCM (8 mL) and DIEA (3 eq.) were added. The mixture was stirred at room temperature for 1 h (called system B). System B was slowly added to system A at room temperature, and the mixture was stirred at 50 °C for 3 h. Work-up and purification: LCMS confirmed the formation of the target compound, and confirmed that the raw materials were reacted completely. The reaction system was concentrated and purified by flash column chromatography (TFA.H₂O/MeCN) to yield a crude product. The crude product was purified by flash column chromatography (NH₄HCO₃.H₂O/MeCN) and lyophilized to yield 189 mg of white solid product. MS: 458 [M+H]⁺. ¹H NMR (400 MHz, DMSO-*d*₆) δ 9.57 (s, 1H), 8.50 (s, 1H), 8.10 (d, *J* = 2.3 Hz, 1H), 8.03 (dd, *J* = 8.8, 2.3 Hz, 1H), 7.36 (d, *J* = 8.9 Hz, 1H), 6.94 (s, 1H), 4.35 (s, 2H), 4.24 (q, *J* = 7.0 Hz, 2H), 2.78 (d, *J* = 6.5 Hz, 10H), 2.56 (d, *J* = 7.3 Hz, 4H), 1.95 (dp, *J* = 14.8, 7.4 Hz, 4H), 1.42 (t, *J* = 6.9 Hz, 3H).

### Example 15: 3-((dimethylamino)methyl)-N-((1,2,3,5,6,7-hexahydro-s-indacen-4-yl)carbamoyl)-4-isopropoxybenzenesulfonamide

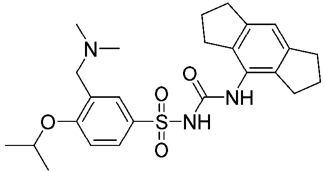

### Synthetic route:

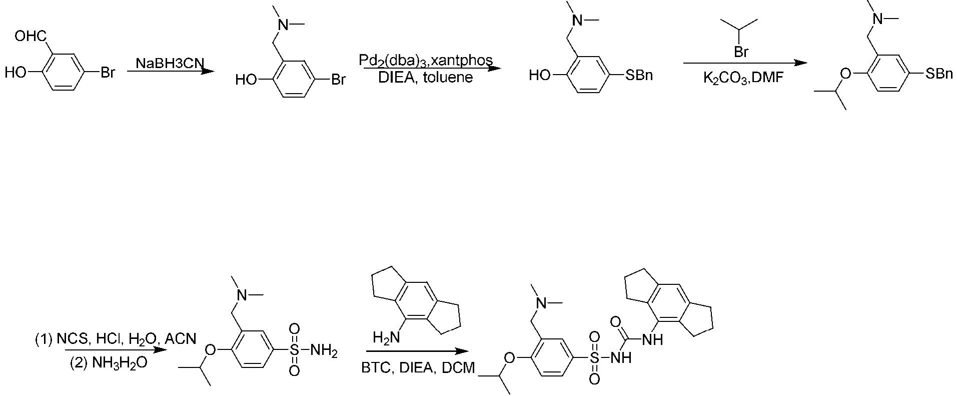

### Step 1: 4-bromo-2-((dimethylamino)methyl)phenol

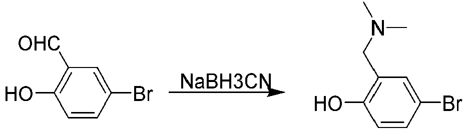

5-bromo-2-hydroxybenzaldehyde (3.0 g) was weighed out and placed into a single-neck flask, and then anhydrous methanol (50 mL) and a solution of dimethylamine in tetrahydrofuran (2 eq.) were added. The mixture was stirred at room temperature overnight. The mixture was added with sodium cyanoborohydride (2 eq.) and stirred at room temperature for 1 h. Work-up and purification: LCMS showed the formation of the target compound, and showed that the raw materials were reacted completely. The system was mixed with silica gel and purified by normal-phase flash column chromatography (dichloromethane/methanol) to yield 2.3 g of the target compound.

### Step 2: 4-(benzylmercapto)-2-((dimethylamino)methyl)phenol

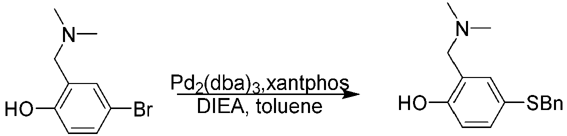

4-bromo-2-((dimethylamino)methyl)phenol (2.1 g) was weighed out and placed into a single-neck flask, and then Pd₂(dba)₃ (0.1 eq.), Xantphos (0.2 eq.), diisopropylethylamine (2 eq.), toluene (25 mL), and benzyl mercaptan (1.5 eq.) were added. The mixture was stirred at 110 °C overnight under nitrogen protection. Work-up and purification: LCMS confirmed the formation of the target compound, and confirmed that the raw materials were reacted completely. The reaction system was filtered, the filter cake was rinsed with dichloromethane, and the filtrate was concentrated to yield a crude product. The crude product was purified by flash column chromatography (dichloromethane/methanol) to yield 2.5 g of pure target compound.

### Step 3: l-(5-(benzylmercapto)-2-isopropoxyphenyl)-N,N-dimethylmethanamine

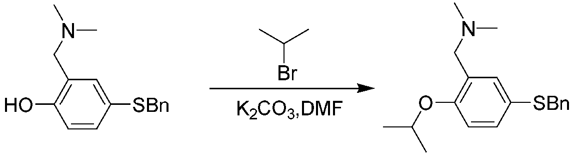

4-(benzylmercapto)-2-((dimethylamino)methyl)phenol (500 mg) was weighed out and placed into a single-neck flask, and then DMF (5mL), potassium carbonate (2 eq.), and 2-bromopropane (1.1 eq.) were added. The mixture was stirred at 60 °C for 2 h. Work-up and purification: LCMS confirmed the formation of the target compound. The system was mixed with silica gel and purified by normal-phase flash column chromatography (dichloromethane/methanol) to yield 200 mg of the target compound.

### Step 4: 3-((dimethylamino)methyl)-4-isopropoxybenzenesulfonamide

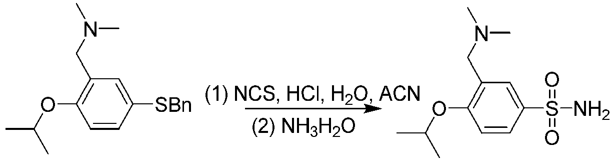

NCS (4 eq.) was weighed out and placed into a single-neck flask, and then acetonitrile (4 mL) and 6 M HCl (1.5 mL) were added. The mixture was stirred at 0 °C for 15 min. 1-(5-(benzylmercapto)-2-isopropoxyphenyl)-N,N-dimethylmethylamine (170 mg) was added and the mixture was stirred at 0 °C for 1 h. The reaction system was added dropwise to aqueous ammonia (15 mL) slowly, and stirred at room temperature for 1 h. Work-up and purification: LCMS confirmed the formation of the target compound. An appropriate amount of DCM was added to extract the reaction system three times, and the organic phases were combined, washed once with saturated brine, and concentrated to yield 95 mg of crude product.

### Step 5: 3-((dimethylamino)methyl)-N-((1,2,3,5,6,7-hexahydro-s-indacen-4-yl)carbamoyl)-4-isopropoxybenzenesulfonamide

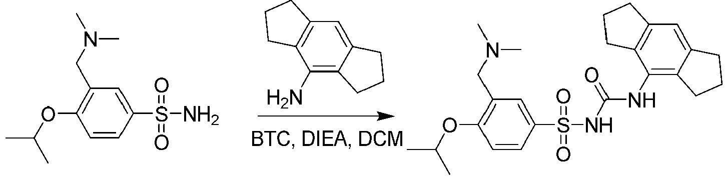

1,2,3,5,6,7-hexahydro-s-indacen-4-amine (1 eq.) was weighed out and placed into a single-neck flask. Then DCM (3 mL) and DIEA (3 eq.) were added, and triphosgene (0.5 eq.) was added at 0 °C. The mixture was stirred at 50 °C for 1 h (called system A). 3-((dimethylamino)methyl)-4-isopropoxybenzenesulfonamide (95 mg) was weighed out and placed into a single-neck flask, and then DCM (3 mL) and DIEA (3 eq.) were added. The mixture was stirred at room temperature for 1 h (called system B). System B was slowly added to system A at room temperature, and the mixture was stirred at 50 °C for 3 h. Work-up and purification: LCMS confirmed the formation of the target compound, and confirmed that the raw materials were reacted completely. The reaction system was concentrated and purified by flash column chromatography (TFA.H₂O/MeCN) to yield a crude product. The crude product was purified by flash column chromatography (NH₄HCO₃.H₂O/MeCN) and lyophilized to yield 16 mg of white solid product. MS: 472 [M+H]⁺. ¹H NMR (400 MHz, DMSO-*d*₆) δ 9.65 (s, 1H), 8.57 (s, 1H), 8.11 (d, *J* = 2.3 Hz, 1H), 8.01 (dd, *J* = 8.9, 2.3 Hz, 1H), 7.39 (d, *J* = 9.0 Hz, 1H), 6.94 (s, 1H), 4.87 (dq, *J* = 11.8, 6.0 Hz, 1H), 4.32 (s, 2H), 2.78 (d, *J* = 7.1 Hz, 11H), 2.70 (t, *J* = 7.2 Hz, 1H), 2.56 (s, 2H), 1.92 (p, *J* = 7.3 Hz, 4H), 1.37 (d, *J* = 6.0 Hz, 6H).

### Example 16: 3-((dimethylamino)methyl)-5-fluoro-N-((1,2,3,5,6,7-hexahydro-s-indacen-4-yl)carbamoyl)-4-methoxybenzenesulfonamide

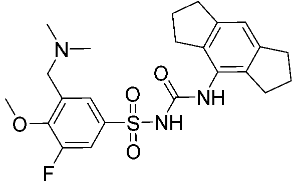

### Synthetic route:

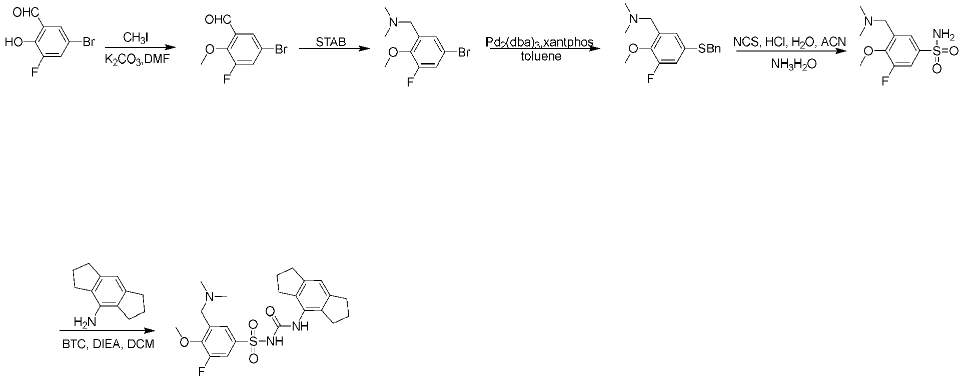

### Step 1: 5-bromo-3-fluoro-2-methoxybenzaldehyde

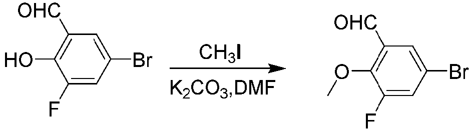

5-bromo-3-fluoro-2-hydroxybenzaldehyde (0.8 g) was weighed out and placed into a single-neck flask, and then DMF (20 mL), potassium carbonate (2 eq.), and methyl iodide (1.2 eq.) were added. The mixture was stirred at 60 °C for 2 h. Work-up and purification: LCMS confirmed the formation of the target compound. The system was mixed with silica gel and purified by normal-phase flash column chromatography (dichloromethane/methanol) to yield 0.67 g of the target compound.

### Step 2: l-(5-bromo-3-fluoro-2-methoxyphenyl)-N,N-dimethylmethanamine

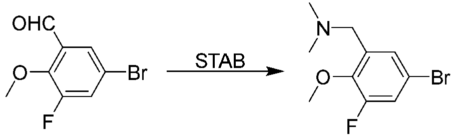

5-bromo-3-fluoro-2-methoxybenzaldehyde (0.67 g) was weighed out and placed into a single-neck flask, and then dichloromethane (20 mL), a solution of dimethylamine in tetrahydrofuran (2 eq.), and acetic acid (1 drop) were added. The mixture was stirred at room temperature for 15 min. The mixture was added with sodium triacetoxyborohydride (4 eq.) and stirred at room temperature for 2 h. Work-up and purification: LCMS showed the formation of the target compound, and showed that the raw materials were reacted completely. The reaction system was diluted by adding water, adjusted to a pH>7 with saturated sodium bicarbonate solution. The system was extracted with dichloromethane, dried, and concentrated to yield 0.47 g of crude target compound.

### Step 3: l-(5-(benzylmercapto)-3-fluoro-2-methoxyphenyl)-N,N-dimethylmethanamine

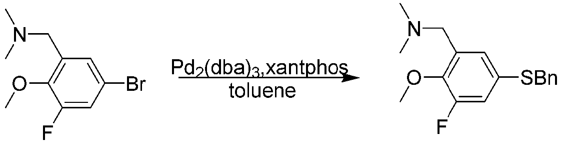

1-(5-bromo-3-fluoro-2-methoxyphenyl)-N,N-dimethylmethylamine (400 mg) was weighed out and placed into a single-neck flask, and then Pd₂(dba)₃ (0.1 eq.), Xantphos (0.2 eq.), diisopropylethylamine (2 eq.), toluene (10 mL), and benzyl mercaptan (1.5 eq.) were added. The mixture was stirred at 110 °C overnight under nitrogen protection. Work-up and purification: LCMS confirmed the formation of the target compound, and confirmed that the raw materials were reacted completely. The reaction system was filtered, the filter cake was rinsed with dichloromethane, and the filtrate was concentrated to yield a crude product. The crude product was purified by flash column chromatography (dichloromethane/methanol) to yield 480 mg of pure target compound.

### Step 4: 3-((dimethylamino)methyl)-5-fluoro-4-methoxybenzenesulfonamide

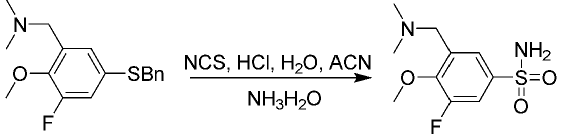

NCS (4 eq.) was weighed out and placed into a single-neck flask, and then acetonitrile (10 mL) and 6 M HCl (5 mL) were added. The mixture was stirred at 0 °C for 15 min. 1-(5-(benzylmercapto)-3-fluoro-2-methoxyphenyl)-N,N-dimethylmethylamine (400 mg) was added and the mixture was stirred at 0 °C for 1 h. The reaction system was added dropwise to aqueous ammonia (40 mL) slowly, and stirred at room temperature for 1 h. Work-up and purification: LCMS confirmed the formation of the target compound. An appropriate amount of DCM was added to extract the reaction system three times, and the organic phases were combined, washed once with saturated brine, and concentrated to yield 270 mg of crude product.

### Step 5: 3-((dimethylamino)methyl)-5-fluoro-N-((1,2,3,5,6,7-hexahydro-s-indacen-4-yl)carbamoyl)-4-methoxybenzenesulfonamide

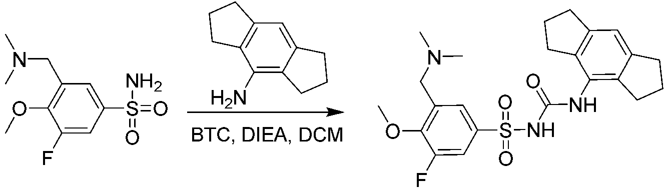

1,2,3,5,6,7-hexahydro-s-indacen-4-amine (1 eq.) was weighed out and placed into a single-neck flask. Then DCM (8 mL) and DIEA (3 eq.) were added, and triphosgene (0.5 eq.) was added at 0 °C. The mixture was stirred at 50 °C for 1 h (called system A). 3-((dimethylamino)methyl)-5-fluoro-4-methoxybenzenesulfonamide (200 mg) was weighed out and placed into a single-neck flask, and then DCM (8 mL) and DIEA (3 eq.) were added. The mixture was stirred at room temperature for 1 h (called system B). System B was slowly added to system A at room temperature, and the mixture was stirred at 50 °C for 3 h. Work-up and purification: LCMS confirmed the formation of the target compound, and confirmed that the raw materials were reacted completely. The reaction system was concentrated and purified by flash column chromatography (TFA.H₂O/MeCN) to yield a crude product. The crude product was purified by flash column chromatography (NH₄HCO₃.H₂O/MeCN) and lyophilized to yield 58 mg of white solid product. MS: 462 [M+H]⁺. ¹H NMR (400 MHz, DMSO-*d*₆) δ 10.40 (s, 1H), 7.97 (s, 1H), 7.77 (s, 1H), 7.70 (dd, *J* = 11.4, 1.8 Hz, 1H), 6.87 (s, 1H), 3.95 (d, *J* = 2.3 Hz, 3H), 3.77 (s, 2H), 2.76 (t, *J* = 7.3 Hz, 4H), 2.56 (t, *J* = 7.1 Hz, 4H), 2.50 (s, 6H), 1.90 (p, *J* = 7.3 Hz, 4H).

### Example 17: 3-((dimethylamino)methyl)-5-fluoro-N-((1,2,3,5,6,7-hexahydro-s-indacen-4-yl)carbamoyl)-4-hydroxybenzenesulfonamide

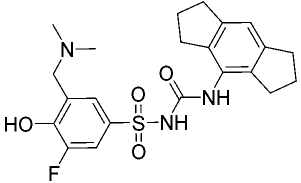

### Synthetic route:

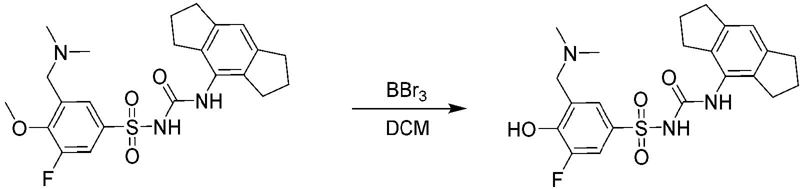

3-((dimethylamino)methyl)-5-fluoro-N-((1,2,3,5,6,7-hexahydro-s-indacen-4-yl)carbamoyl)-4-methoxybenzenesulfonamide (40 mg) was weighed out and placed into a single-neck flask. Then ultra-dry dichloromethane (3 mL) was added, and boron tribromide (2 eq.) was added at 0 °C under nitrogen protection. The mixture was heated to room temperature and stirred overnight. Work-up and purification: LCMS showed the formation of the target compound, and showed that the raw materials were reacted completely. Methanol was added at 0 °C to quench the reaction system. The crude product was purified by flash column chromatography (NH₄HCO₃.H₂O/MeCN) and lyophilized to yield 5 mg of white solid product. MS: 448 [M+H]⁺. ¹H NMR (400 MHz, DMSO-*d*₆) δ 7.32 (d, *J* = 26.1 Hz, 2H), 6.70 (d, *J* = 41.6 Hz, 1H), 3.67 (s, 2H), 2.74 (t, *J* = 7.0 Hz, 3H), 2.62 (s, 3H), 2.50 (s, 4H), 2.27 (s, 4H), 1.92 - 1.84 (m, 4H).

### Example 18: 3-((dimethylamino)methyl)-N-((1,2,3,5,6,7-hexahydro-s-indacen-4-yl)carbamoyl)-2-nitrobenzenesulfonamide

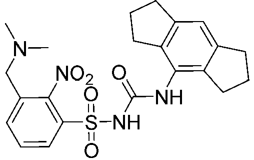

### Synthetic route:

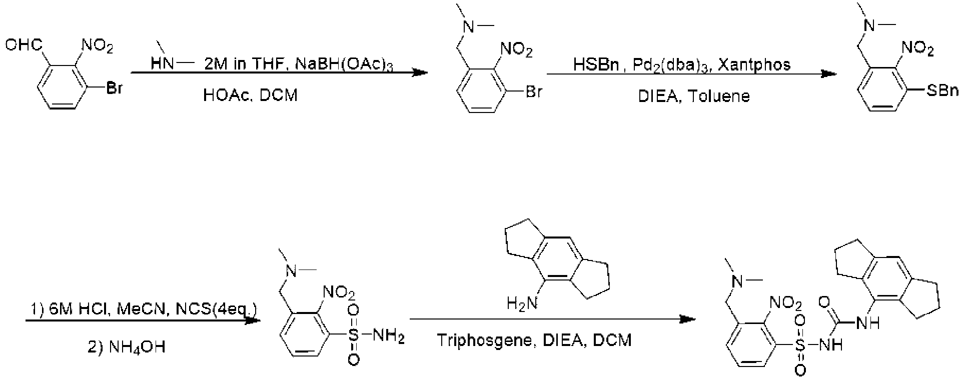

### Step 1: 1-(3-bromo-2-nitrophenyl)-N,N-dimethylmethylamine

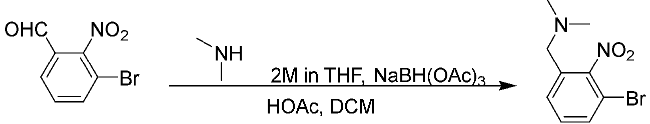

3-bromo-2-nitrobenzaldehyde (600 mg) was weighed out and placed into a 100 mL single-neck flask. Then DCM (25 mL) was added, and dimethylamine (2 M in THF) (2 eq.) and HOAc (0.2 eq.) were added while stirring. The mixture was stirred at room temperature for 2 h. 2 h later, NaBH(OAc)₃ (3 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 v/v (30 mL), the organic phases were combined, washed with saturated brine, dried and rotary evaporated, and passed through flash column chromatography (DCM/MeOH) to yield a crude target compound, 520 mg of yellow oily liquid.

### Step 2: 1-(3-(benzylmercapto)-2-nitrophenyl)-N,N-dimethylmethanamine

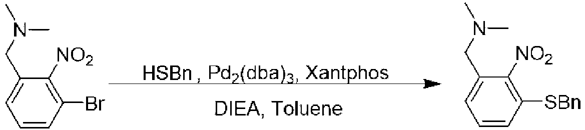

1-(3-bromo-2-nitrophenyl)-N,N-dimethylmethylamine (520 mg) was weighed out and placed into a 40 mL microwave tube, and then toluene (12 mL), benzyl mercaptan (2 eq.), DIEA (3 eq.), Pd₂(dba)₃ (0.1 eq.), and Xantphos (0.2 eq.) were added under a nitrogen atmosphere. The mixture was heated to 115 °C to react for 12 h. LCMS showed the formation of the target compound. Work-up and purification: The reaction solution was mixed with silica gel, rotary evaporated, and passed through flash column chromatography (DCM/MeOH) to yield a crude target compound, 420 mg of yellow oily liquid.

### Step 3: 3-((dimethylamino)methyl)-2-nitrobenzenesulfonamide

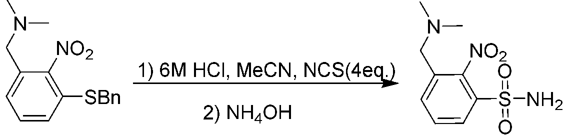

6 M HCl (1.0 mL) was measured out and placed into a 50 mL single-neck flask, and MeCN (15 mL) was added. The mixture was stirred and cooled to 0 °C, and added with NCS (3 eq.). 1-(3-(benzylmercapto)-2-nitrophenyl)-N,N-dimethylmethylamine (420 mg) was dissolved in 10 mL MeCN as the reaction solution. The reaction solution was added dropwise into the single-neck flask at 0 °C, and stirred for 0.5 h. NH₄OH (35 mL) was placed into another 100 mL single-neck flask. The above reaction solution was added dropwise into NH₄OH while stirring at room temperature. The mixture was stirred for 0.5 h. LCMS confirmed the formation of the target compound. Work-up and purification: The reaction solution was rotary evaporated to remove MeCN, and extracted twice with DCM:MeOH=10:1 v/v (50 mL), followed by drying and concentration under reduced pressure to yield the target compound, 0.5 g of yellow oily crude product.

### Step 4: 3-((dimethylamino)methyl)-N-((1,2,3,5,6,7-hexahydro-s-indacen-4-yl)carbamoyl)-2-nitrobenzenesulfonamide

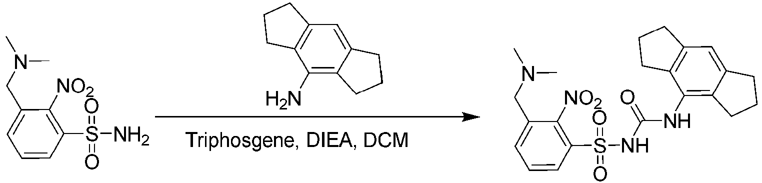

300 mg of 1,2,3,5,6,7-hexahydro-s-indacen-4-amine was weighed out and placed into a 40 mL single-neck flask. DCM (10 mL) and DIEA (5 eq.) were added, and triphosgene (0.4 eq.) was added batchwise while stirring at room temperature. The reaction solution was heated to 50 °C to react for 1 h. 500 mg of crude 3-((dimethylamino)methyl)-2-nitrobenzenesulfonamide was dissolved in DCM (5 mL) and DIEA (3 eq.). Once fully dissolved, the solution was added dropwise into the above reaction solution at 50 °C. The reaction was carried out at 50 °C for 2 h. LCMS confirmed the formation of the target compound. Work-up and purification: The reaction solution was directly mixed with silica gel and passed through flash column chromatography (DCM/MeOH) to yield a crude product. The crude product was subjected to preparative purification by flash column chromatography (0.05% NH₄HCO₃) and lyophilization to yield the target compound, 280 mg of white solid. MS: 459 [M+H]⁺. ¹H NMR (400 MHz, DMSO-*d*₆) δ 10.53 (s, 1H), 8.09 (d, *J* = 7.6 Hz, 1H), 7.94 (s, 1H), 7.85 (d, *J* = 7.2 Hz, 1H), 7.76 (t, *J* = 7.6 Hz, 1H), 6.90 (s, 1H), 3.64 (s, 2H), 2.78 (t, *J* = 7.2 Hz, 4H), 2.60 (t, *J* = 7.0 Hz, 4H), 2.27 (s, 6H), 1.98 - 1.86 (m, 4H).

### Example 19: 2-amino-3-((dimethylamino)methyl)-N-((1,2,3,5,6,7-hexahydro-s-indacen-4-yl)carbamoyl)benzenesulfonamide

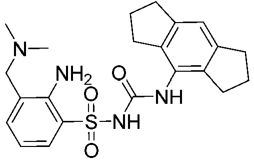

### Synthetic route:

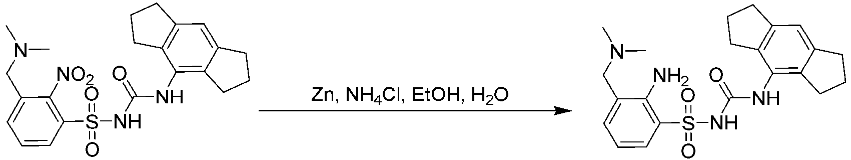

3-((dimethylamino)methyl)-N-((1,2,3,5,6,7-hexahydro-s-indacen-4-yl)carbamoyl)-2-nitrobenzenesulfonamide (280 mg) was weighed out and placed into a 40 mL single-neck flask. EtOH (6 mL) and H₂O (3 mL) were added, and zinc powder (15 eq.) and ammonium chloride (5 eq.) were added while stirring at room temperature. After the addition, the reaction solution was heated to 60 °C to react for 4 h. LCMS confirmed the formation of the target compound, and confirmed that the raw materials were reacted completely. Work-up and purification: The reaction solution was filtered with celite. The filter cake was rinsed with 100 mL of DCM/MeOH=10/1 v/v, the filtrate was concentrated, and when a small amount remained, the crude product was passed through flash column chromatography. The crude product was subjected to preparative purification by flash column chromatography (0.05% NH₄HCO₃) and lyophilization to yield the target compound, 210 mg of white solid. MS: 429 [M+H]⁺. ¹H NMR (400 MHz, DMSO-*d*₆) δ 8.00 (s, 1H), 7.63 (d, *J* = 8.4, Hz, 1H), 7.24 (d, *J* = 6.8 Hz, 1H), 6.90 (s, 1H), 6.62 (t, *J* = 7.6 Hz, 1H), 3.56 (s, 2H), 2.76 (t, *J* = 7.4 Hz, 4H), 2.52 (t, *J* = 7.4 Hz, 4H), 2.24 (s, 6H), 1.95 - 1.86 (m, 4H).

### Example 20: 5-((dimethylamino)methyl)-2-fluoro-N-((1,2,3,5,6,7-hexahydro-s-indacen-4-yl)carbamoyl)-4-methoxybenzenesulfonamide

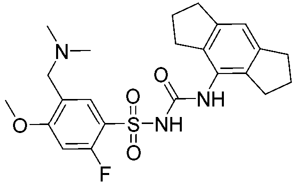

### Synthetic route:

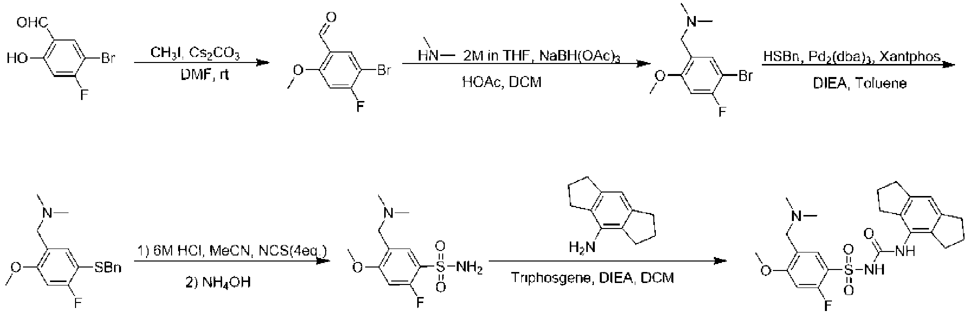

### Step 1: 5-bromo-4-fluoro-2-methoxybenzaldehyde

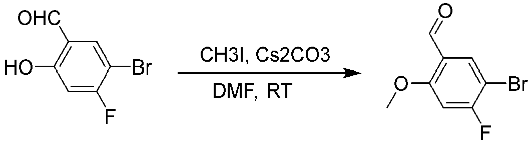

5-bromo-4-fluoro-2-hydroxybenzaldehyde (1.0 g) was weighed out and placed into a 100 mL single-neck flask. Then DMF (20 mL) was added, and Cs₂CO₃ (3 eq.) was added, and CH₃I (2 eq.) was added while stirring at room temperature. The mixture was stirred at room temperature for 4 h. LCMS showed the formation of the target compound. Work-up and purification: The reaction solution was poured into an appropriate amount of ice water, and extracted twice with ethyl acetate, and the organic phase was washed three times with water, and washed with saturated brine, dried, and rotary evaporated to yield the target compound, 1 g of yellow solid.

### Step 2: 1-(5-bromo-4-fluoro-2-methoxyphenyl)-N,N-dimethylmethanamine

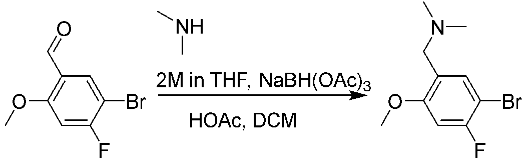

5-bromo-4-fluoro-2-methoxybenzaldehyde (1.0 g) was weighed out and placed into a 100 mL single-neck flask. Then DCM (30 mL) was added, and dimethylamine (2 M in THF) (2 eq.) and HOAc (0.2 eq.) were added while stirring. The mixture was stirred at room temperature for 2 h. 2 h later, NaBH(OAc)₃ (3 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 v/v (30 mL), the organic phases were combined, washed with saturated brine, dried and rotary evaporated, and passed through flash column chromatography (DCM/MeOH) to yield a crude target compound, 800 mg of yellow oily liquid.

### Step 3: l-(5-(benzylmercapto)-4-fluoro-2-methoxyphenyl)-N,N-dimethylmethanamine

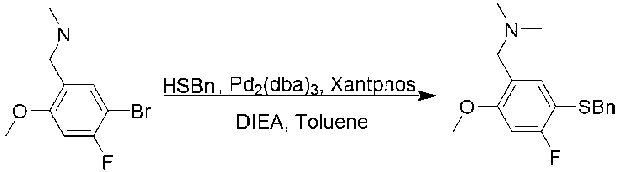

1-(5-bromo-4-fluoro-2-methoxyphenyl)-N,N-dimethylmethylamine (600 mg) was weighed out and placed into a 40 mL microwave tube, and then toluene (12 mL), 8 drops of THF, benzyl mercaptan (2 eq.), DIEA (3 eq.), Pd₂(dba)₃ (0.1 eq.), and Xantphos (0.2 eq.) were added under a nitrogen atmosphere. The mixture was heated to 115 °C to react for 12 h. LCMS showed the formation of the target compound. Work-up and purification: The reaction solution was mixed with silica gel, rotary evaporated, and passed through flash column chromatography (DCM/MeOH) to yield a crude target compound, 300 mg of yellow oily liquid.

### Step 4: 5-((dimethylamino)methyl)-2-fluoro-4-methoxybenzenesulfonamide

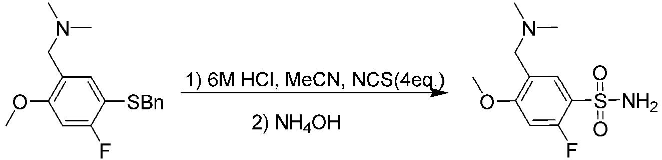

6 M HCl (0.8 mL) was measured out and placed into a 50 mL single-neck flask, and MeCN (10 mL) was added. The mixture was stirred and cooled to 0 °C, and added with NCS (3 eq.). 1-(5-(benzylmercapto)-4-fluoro-2-methoxyphenyl)-N,N-dimethylmethylamine (300 mg) was dissolved in 5 mL MeCN, and then added dropwise into the above reaction solution at 0 °C. The mixture was stirred for 0.5 h. NH₄OH (25 mL) was placed into another 100 mL single-neck flask, and the above reaction solution was added dropwise into NH₄OH via a constant-pressure dropping funnel while stirring at room temperature. The mixture was stirred for 0.5 h. LCMS confirmed the formation of the target compound. Work-up and purification: The reaction solution was rotary evaporated to remove MeCN, extracted twice with DCM:MeOH=10:1 v/v (50 mL), dried, concentrated under reduced pressure, and purified by flash column chromatography (0.05% NH₄HCO₃) to yield the target compound, 60 mg of light yellow solid product.

### Step 5: 5-((dimethylamino)methyl)-2-fluoro-N-((1,2,3,5,6,7-hexahydro-s-indacen-4-yl)carbamoyl)-4-methoxybenzenesulfonamide

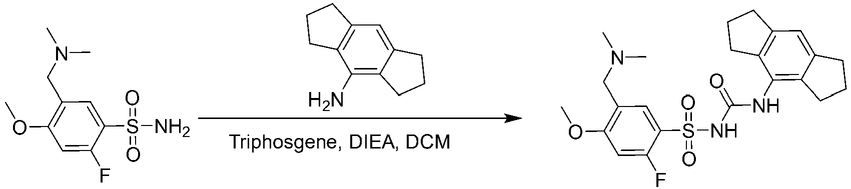

1,2,3,5,6,7-hexahydro-s-indacen-4-amine (100 mg) was weighed out and placed into a 40 mL single-neck flask. DCM (6 mL) and DIEA (5 eq.) were added, and triphosgene (0.4 eq.) was added batchwise while stirring at room temperature. The reaction solution was heated to 50 °C to react for 1 h. 60 mg of 5-((dimethylamino)methyl)-2-fluoro-4-methoxybenzenesulfonamide was dissolved in DCM (5 mL) and DIEA (3 eq.). Once fully dissolved, the solution was added dropwise into the above reaction solution at 50 °C. The reaction was carried out at 50 °C for 2 h. LCMS confirmed the formation of the target compound. Work-up and purification: The reaction solution was directly mixed with silica gel and passed through flash column chromatography (DCM/MeOH) to yield a crude product. The crude product was subjected to preparative purification by flash column chromatography (acetonitrile/water, 0.05% NH₄HCO₃) and lyophilization to yield the target compound, 40 mg of white solid. MS: 462 [M+H]⁺. ¹H NMR (400 MHz, DMSO-*d*₆) δ 10.24 (s, 1H), 8.01 - 7.88 (m, 2H), 7.17 (d, *J* = 12.0 Hz, 1H), 6.87 (s, 1H), 3.94 (s, 2H), 3.89 (s, 3H), 2.76 (t, *J* = 7.2 Hz, 4H), 2.57 (t, *J* = 7.2 Hz, 4H), 2.50 (s, 6H), 1.95 - 1.86 (m, 4H).

### Example 21: 5-((dimethylamino)methyl)-2-fluoro-N-((1,2,3,5,6,7-hexahydro-s-indacen-4-yl)carbamoyl)-4-hydroxybenzamide

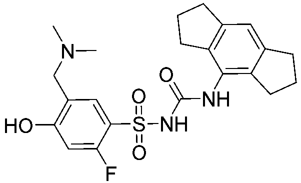

### Synthetic route:

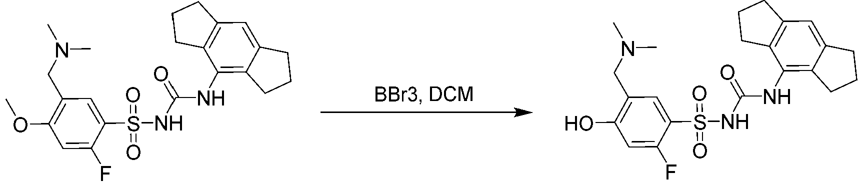

5-((dimethylamino)methyl)-2-fluoro-N-((1,2,3,5,6,7-hexahydro-s-indacen-4-yl)carbamoyl)-4-methoxybenzenesulfonamide (30 mg) was weighed out and placed into a 20 mL single-neck flask, and DCM (5 mL) was added. The mixture was cooled to 0 °C under nitrogen protection, added with 3 drops of BBr₃ with a dropper, heated to room temperature, and stirred for 2 h. LCMS confirmed the formation of the target compound, and confirmed that the raw materials were reacted completely (if the raw materials were not reacted completely, BBr₃ was replenished). Work-up and purification: The reaction solution was cooled to 0 °C, and 0.5 mL methanol was added dropwise to quench the reaction, followed by addition of 3 mL of acetonitrile and removal of dichloromethane by rotary evaporation at low temperature. When a small amount of liquid remained, the reaction solution was subjected to flash column chromatography. The crude product was subjected to preparative flash column chromatography (0.05% HCOOH) and lyophilization to yield the target compound, 210 mg of white solid. MS: 448 [M+H]⁺. ¹H NMR (400 MHz, DMSO-*d*₆) δ 7.50 (s, 1H), 6.81 (s, 1H), 6.51 (s, 1H), 3.57 (s, 2H), 2.82 - 2.61 (m, 8H), 2.25 (s, 6H), 1.99 - 1.83 (m, 4H).

### Example 22: 4-amino-5-((dimethylamino)methyl)-2-fluoro-N-((1,2,3,5,6,7-hexahydros-indacen-4-yl)carbamoyl)benzenesulfonamide

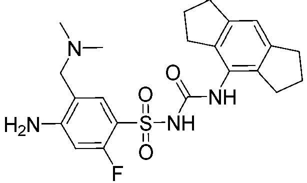

### Synthetic route:

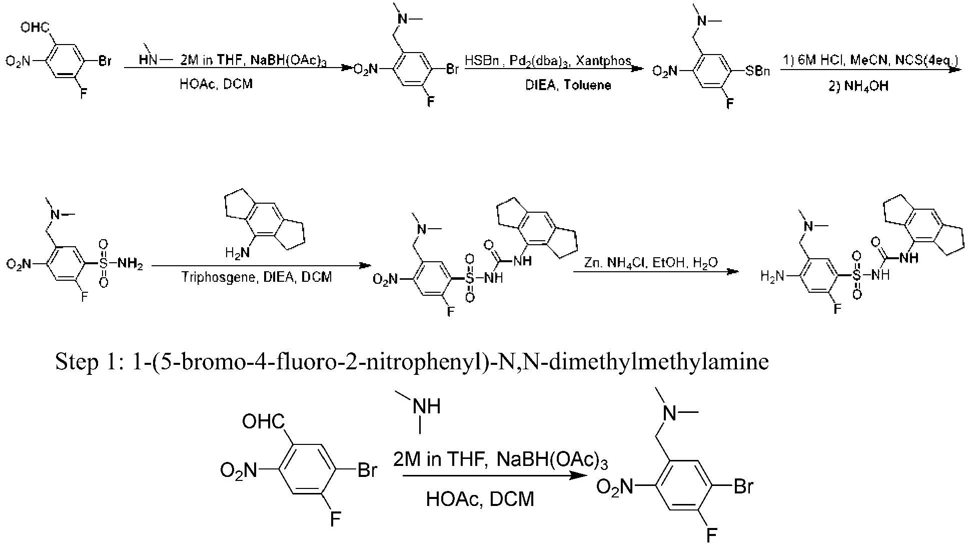

5-bromo-4-fluoro-2-nitrobenzaldehyde (500 mg) was weighed out and placed into a 100 mL single-neck flask. Then DCM (25 mL) was added, and dimethylmethylamine (2 M in THF) (2 eq.) and HOAc (0.2 eq.) were added while stirring. The mixture was stirred at room temperature for 2 h. 2 h later, NaBH(OAc)₃ (3 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 v/v (50 mL), the organic phases were combined, washed with saturated brine, dried and rotary evaporated, and passed through flash column chromatography (DCM/MeOH) to yield the target compound, 300 mg of yellow oily liquid.

### Step 2: 1-(5-(benzylmercapto)-4-fluoro-2-nitrophenyl)-N,N-dimethylmethanamine

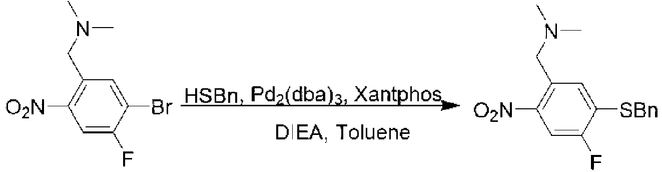

1-(5-bromo-4-fluoro-2-nitrophenyl)-N,N-dimethylmethylamine (300 mg) was weighed out and placed into a 40 mL microwave tube, and then toluene (8 mL), benzyl mercaptan (2 eq.), DIEA (3 eq.), Pd₂(dba)₃ (0.1 eq.), and Xantphos (0.2 eq.) were added under a nitrogen atmosphere. The mixture was heated to 115 °C to react for 12 h. LCMS showed the formation of the target compound. Work-up and purification: The reaction solution was mixed with silica gel, rotary evaporated, and passed through flash column chromatography (DCM/MeOH) to yield a crude target compound, 100 mg of yellow oily liquid.

### Step 3: 5-((dimethylamino)methyl)-2-fluoro-4-nitrobenzenesulfonamide

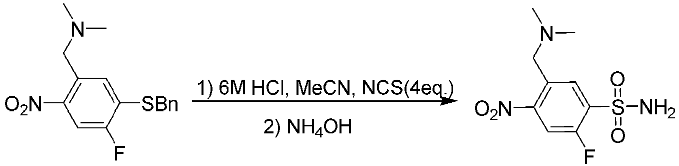

6 M HCl (0.25 mL) was measured out and placed into a 50 mL single-neck flask, and MeCN (15 mL) was added. The mixture was cooled to 0 °C, and added with NCS (3 eq.). 1-(5-(benzylmercapto)-4-fluoro-2-nitrobenzene)-N,N-dimethylmethylamine (100 mg) was dissolved in 3 mL MeCN, and then added dropwise into the above reaction solution at 0 °C. The mixture was stirred for 0.5 h. NH₄OH (10 mL) was placed into another 100 mL single-neck flask, and the above reaction solution was added dropwise into NH₄OH via a constant-pressure dropping funnel while stirring at room temperature. The mixture was stirred for 0.5 h. LCMS confirmed the formation of the target compound. Work-up and purification: The reaction solution was rotary evaporated to remove MeCN, extracted twice with DCM:MeOH=10:1 (50 mL), dried, and concentrated under reduced pressure to yield the target compound, 125 mg of yellow oily crude product.

### Step 4: 5-((dimethylamino)methyl)-2-fluoro-N-((1,2,3,5,6,7-hexahydro-s-indacen-4-yl)carbamoyl)-4-nitrobenzenesulfonamide

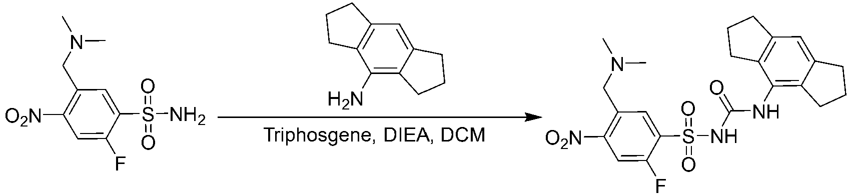

1,2,3,5,6,7-hexahydro-s-indacen-4-amine (100 mg) was weighed out and placed into a 40 mL single-neck flask. DCM (8 mL) and DIEA (5 eq.) were added, and triphosgene (0.4 eq.) was added batchwise while stirring at room temperature. The reaction solution was heated to 50 °C to react for 1 h. 125 mg of crude 5-((dimethylamino)methyl)-2-fluoro-4-nitrobenzenesulfonamide was dissolved in DCM (4 mL) and DIEA (3 eq.). Once fully dissolved, the solution was added dropwise into the above reaction solution at 50 °C. The reaction was carried out at 50 °C for 2 h. LCMS confirmed the formation of the target compound. Work-up and purification: The reaction solution was directly mixed with silica gel and passed through flash column chromatography (DCM/MeOH) to yield a crude product. The crude product was subjected to preparative purification by flash column chromatography (0.05% NH₄HCO₃) and lyophilization to yield the target compound, 50 mg of white solid.

### Step 5: 4-amino-5-((dimethylamino)methyl)-2-fluoro-N-((1,2,3,5,6,7-hexahydro-s-indacen-4-yl)carbamoyl)benzenesulfonamide

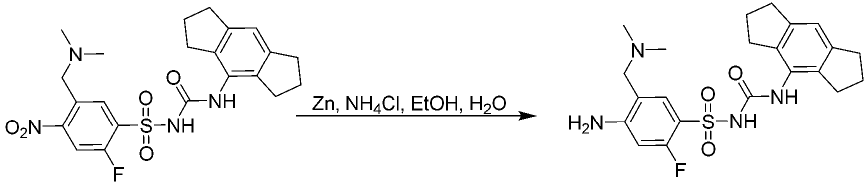

5-((dimethylamino)methyl)-2-fluoro-N-((1,2,3,5,6,7-hexahydro-s-indacen-4-yl)carbamoyl)-4-nitrobenzenesulfonamide (35 mg) was weighed out and placed into a 40 mL single-neck flask. EtOH (6 mL) and H₂O (3 mL) were added, and zinc powder (15 eq.) and ammonium chloride (5 eq.) were added while stirring at room temperature. After the addition, the reaction solution was heated to 60 °C to react for 4 h. LCMS confirmed the formation of the target compound, and confirmed that the raw materials were reacted completely. Work-up and purification: The reaction solution was filtered with celite. The filter cake was rinsed with 100 mL of DCM/MeOH=10/1 v/v, the filtrate was concentrated, and when a small amount remained, the crude product was passed through flash column chromatography. The crude product was subjected to preparative purification by flash column chromatography (0.05% NH₄HCO₃) and lyophilization to yield the target compound, 10 mg of white solid. MS: 447 [M+H]⁺. ¹H NMR (400 MHz, DMSO-*d*₆) δ 10.47 (s, 1H), 7.88 (s, 1H), 7.41 (d, *J* = 8.2 Hz, 1H), 6.90 (s, 1H), 6.54 - 6.30 (m, 3H), 3.36 (s, 2H), 2.77 (t, *J* = 7.2 Hz, 4H), 2.55 (t, *J* = 7.2 Hz, 4H), 2.16 (s, 6H), 1.97 - 1.88 (m, 4H).

### Example 23: 3-((dimethylamino)methyl)-2,4-difluoro-N-((1,2,3,5,6,7-hexahydro-s-indacen-4-yl)carbamoyl)benzenesulfonamide

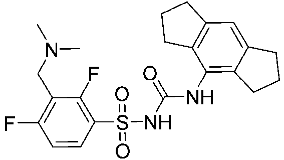

### Synthetic route:

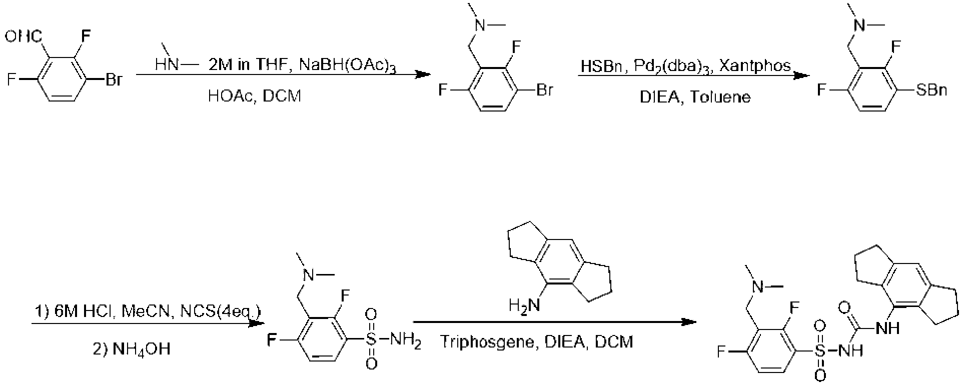

### Step 1: 1-(3-bromo-2,6-difluorophenyl)-N,N-dimethylmethanamine

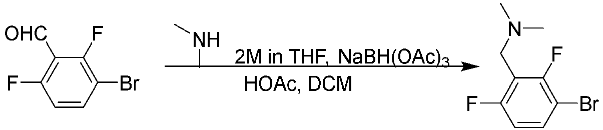

3-bromo-2,6-difluorobenzaldehyde (400 mg) was weighed out and placed into a 100 mL single-neck flask. Then DCM (20 mL) was added, and dimethylamine (2 M in THF) (2 eq.) and HOAc (0.2 eq.) were added while stirring. The mixture was stirred at room temperature for 2 h. 2 h later, NaBH(OAc)₃ (3 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 (30 mL), the organic phases were combined, washed with saturated brine, dried and rotary evaporated, and passed through flash column chromatography (DCM/MeOH) to yield a crude target compound, 200 mg of yellow oily liquid.

### Step 2: 1-(3-(benzylmercapto)-2,6-difluorobenzene)-N,N-dimethylmethanamine

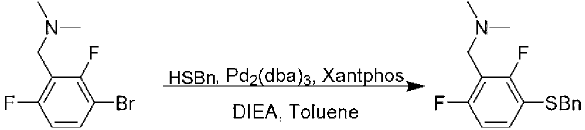

1-(3-bromo-2,6-difluorophenyl)-N,N-dimethylmethylamine (200 mg) was weighed out and placed into a 40 mL microwave tube, and then toluene (6 mL), SHBn (2 eq.), DIEA (3 eq.), Pd₂(dba)₃ (0.1 eq.), and Xantphos (0.2 eq.) were added under a nitrogen atmosphere. The mixture was heated to 115 °C to react for 12 h. LCMS showed the formation of the target compound. Work-up and purification: The reaction solution was mixed with silica gel, rotary evaporated, and passed through flash column chromatography (DCM/MeOH) to yield a crude target compound, 120 mg of yellow oily liquid.

### Step 3: 3-((dimethylamino)methyl)-2,4-difluorobenzenesulfonamide

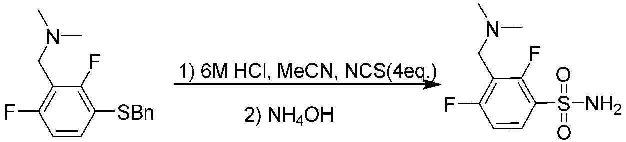

6 M HCl (0.3 mL) was measured out and placed into a 50 mL single-neck flask, and MeCN (6 mL) was added. The mixture was stirred and cooled to 0 °C, and added with NCS (3 eq.). 1-(3-(benzylmercapto)-2,6-difluorobenzene)-N,N-dimethylmethylamine (120 mg) was dissolved in 3 mL MeCN, and then added dropwise into the above reaction solution at 0 °C. The mixture was stirred for 0.5 h. NH₄OH (15 mL) was placed into another 100 mL single-neck flask, and the above reaction solution was added dropwise into NH₄OH via a constant-pressure dropping funnel while stirring at room temperature. The mixture was stirred for 0.5 h. LCMS confirmed the formation of the target compound. Work-up and purification: The reaction solution was rotary evaporated to remove MeCN, extracted twice with DCM:MeOH=10:1 (50 mL), dried, and concentrated under reduced pressure. The crude product was subjected to preparative purification by flash column chromatography (acetonitrile/water, 10-100% acetonitrile, added with 0.05% HCOOH) to yield the target compound, 50 mg of light yellow solid.

### Step 4: 3-((dimethylamino)methyl)-2,4-difluoro-N-((1,2,3,5,6,7-hexahydro-s-indacen-4-yl)carbamoyl)benzenesulfonamide

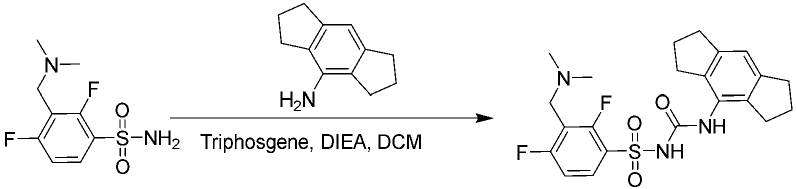

1,2,3,5,6,7-hexahydro-s-indacen-4-amine (60 mg) was weighed out and placed into a 40 mL single-neck flask. DCM (6 mL) and DIEA (5 eq.) were added, and triphosgene (0.4 eq.) was added batchwise while stirring at room temperature. The reaction solution was heated to 50 °C to react for 1 h. 60 mg of crude 3-((dimethylamino)methyl)-2,4-difluorobenzenesulfonamide was dissolved in DCM (3 mL) and DIEA (3 eq.). Once fully dissolved, the solution was added dropwise into the above reaction solution at 50 °C. The reaction was carried out at 50 °C for 2 h. LCMS confirmed the formation of the target compound. Work-up and purification: The reaction solution was directly mixed with silica gel and passed through flash column chromatography (DCM/MeOH) to yield a crude product. The crude product was subjected to preparative purification by flash column chromatography (acetonitrile/water, added with 0.05% NH₄HCO₃) and lyophilization to yield the target compound, 15 mg of white solid. MS: 450 [M+H]⁺. ¹H NMR (400 MHz, DMSO-*d*₆) δ 10.82 (s, 1H), 7.98 - 7.88 (m, 2H), 7.31 (t, *J* = 8.8 Hz, 1H), 6.90 (s, 1H), 3.77 (s, 2H), 2.78 (t, *J* = 7.2 Hz, 4H), 2.57 (t, *J* = 7.2 Hz, 4H), 2.32 (s, 6H), 1.97 - 1.88 ( m, 4H).

### Example 24: 3-((dimethylamino)methyl)-2-fluoro-N-((1,2,3,5,6,7-hexahydro-s-indacen-4-yl)carbamoyl)benzenesulfonamide

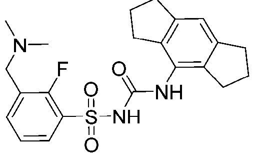

### Synthetic route:

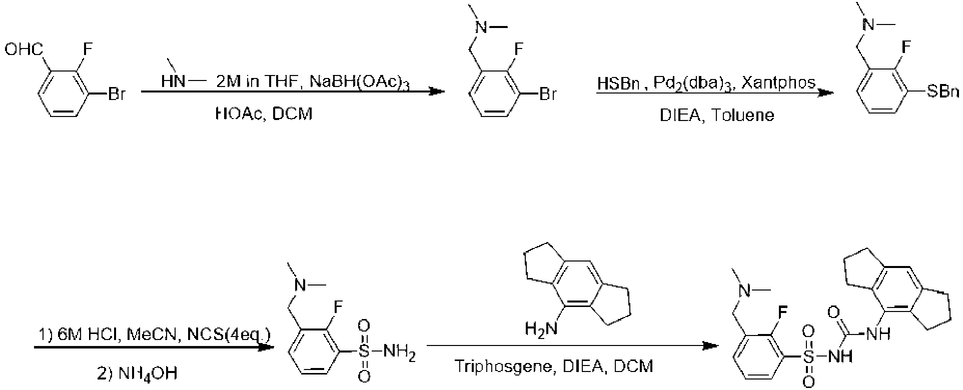

### Step 1: 1-(3-bromo-2-fluorophenyl)-N,N-dimethylmethanamine

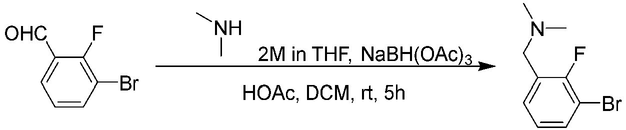

3-bromo-2-fluorobenzaldehyde (1.0 g) was weighed out and placed into a 100 mL single-neck flask. Then DCM (30 mL) was added, and dimethylamine (2 M in THF) (2 eq.) and HOAc (0.2 eq.) were added while stirring. The mixture was stirred at room temperature for 2 h. 2 h later, NaBH(OAc)₃ (3 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 v/v (50 mL), the organic phases were combined, washed with saturated brine, dried and rotary evaporated, and passed through flash column chromatography (DCM/MeOH) to yield the target compound, 900 mg of yellow oily liquid.

### Step 2: 1-(3-(benzylmercapto)-2-fluorophenyl)-N,N-dimethylmethanamine

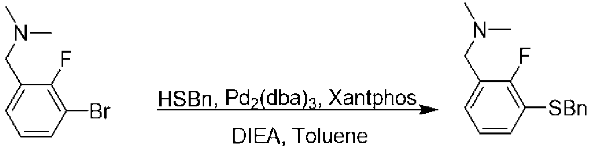

1-(3-bromo-2-fluorophenyl)-N,N-dimethylmethanamine (400 mg) was weighed out and placed into a 40 mL microwave tube. Toluene (15 mL) and benzyl mercaptan (2 eq.) were added, and DIEA (3 eq.), Pd₂(dba)₃ (0.1 eq.), and Xantphos (0.2 eq.) were added under a nitrogen atmosphere. The mixture was heated to 115 °C to react for 12 h. LCMS showed the formation of the target compound. Work-up and purification: The reaction solution was mixed with silica gel, rotary evaporated, and passed through flash column chromatography (DCM/MeOH) to yield a crude target compound, 100 mg of yellow oily liquid.

### Step 3: 3-((dimethylamino)methyl)-2-fluorobenzenesulfonamide

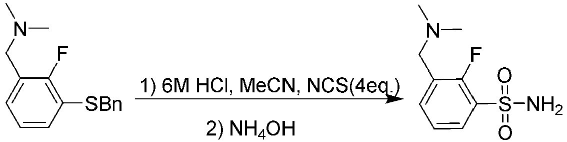

6 M HCl (0.3 mL) was measured out and placed into a 50 mL single-neck flask, and MeCN (5 mL) was added. The mixture was stirred and cooled to 0 °C, and added with NCS (3 eq.). 1-(3-(benzylmercapto)-2-fluorophenyl)-N,N-dimethylmethylamine (200 mg) was dissolved in 3 mL MeCN, and then added dropwise into the above reaction solution at 0 °C. The mixture was stirred for 0.5 h. NH₄OH (10 mL) was placed into another 100 mL single-neck flask, and the above reaction solution was added dropwise into NH₄OH via a constant-pressure dropping funnel while stirring at room temperature. The mixture was stirred for 0.5 h. LCMS confirmed the formation of the target compound. Work-up and purification: The reaction solution was rotary evaporated to remove MeCN, extracted twice with DCM:MeOH=10:1 (50 mL), dried, and concentrated under reduced pressure to yield the target compound, 95 mg of yellow oily crude product.

### Step 4: 3-((dimethylamino)methyl)-2-fluoro-N-((1,2,3,5,6,7-hexahydro-s-indacen-4-yl)carbamoyl)benzenesulfonamide

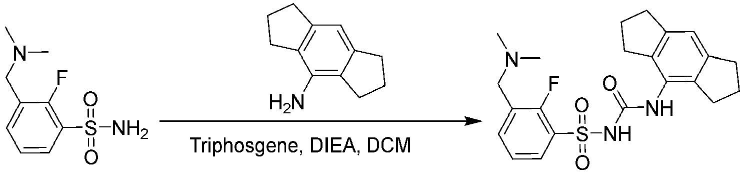

1,2,3,5,6,7-hexahydro-s-indacen-4-amine (100 mg) was weighed out and placed into a 40 mL single-neck flask. DCM (8 mL) and DIEA (5 eq.) were added, and triphosgene (0.4 eq.) was added batchwise while stirring at room temperature. The reaction solution was heated to 50 °C to react for 1 h. 95 mg of crude 3-((dimethylamino)methyl)-2-fluorobenzenesulfonamide was dissolved in DCM (4 mL) and DIEA (3 eq.). Once fully dissolved, the solution was added dropwise into the above reaction solution at 50 °C. The reaction was carried out at 50 °C for 2 h. LCMS confirmed the formation of the target compound. Work-up and purification: The reaction solution was directly mixed with silica gel and passed through flash column chromatography (DCM/MeOH) to yield a crude product. The crude product was subjected to preparative purification by flash column chromatography (acetonitrile/water, 0.05% NH₄HCO₃) and lyophilization to yield the target compound, 80 mg of white solid. MS: 432 [M+H]⁺. ¹H NMR (400 MHz, DMSO-*d*₆) δ 10.71 (s, 1H), 7.97 (s, 1H), 7.82 (t, *J* = 7.2 Hz, 1H), 7.67 (t, *J* = 6.6 Hz, 1H), 7.33 (t, *J* = 7.6 Hz, 1H), 6.86 (s, 1H), 3.79 (s, 2H), 2.75 (t, *J* = 7.2 Hz, 4H), 2.55 (t, *J* = 6.8 Hz, 4H), 2.35 (s, 6H), 1.95 - 1.85 (m, 4H).

### Example 25: 3-((dimethylamino)methyl)-2,6-difluoro-N-((1,2,3,5,6,7-hexahydro-s-indacen-4-yl)carbamoyl)benzenesulfonamide

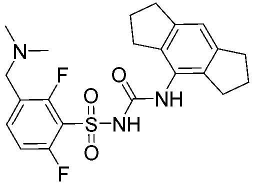

### Synthetic route:

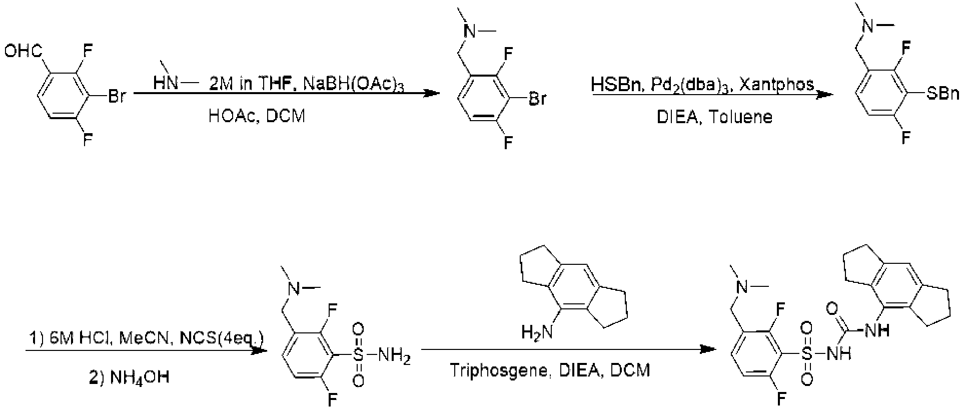

### Step 1: 1-(3-bromo-2,4-difluorophenyl)-N,N-dimethylmethanamine

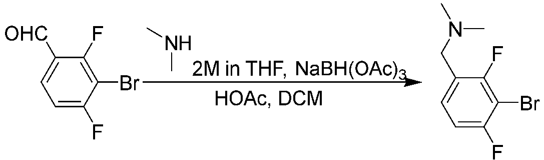

3-bromo-2,4-difluorobenzaldehyde (600 mg) was weighed out and placed into a 100 mL single-neck flask. Then DCM (25 mL) was added, and dimethylamine (2 M in THF) (2 eq.) and HOAc (0.2 eq.) were added while stirring. The mixture was stirred at room temperature for 2 h. 2 h later, NaBH(OAc)₃ (3 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 v/v (50 mL), the organic phases were combined, washed with saturated brine, dried and rotary evaporated, and passed through flash column chromatography (DCM/MeOH) to yield the target compound, 450 mg of yellow oily liquid.

### Step 2: l-(3-(benzylmercapto)-2,4-difluorophenyl)-N,N-dimethylmethanamine

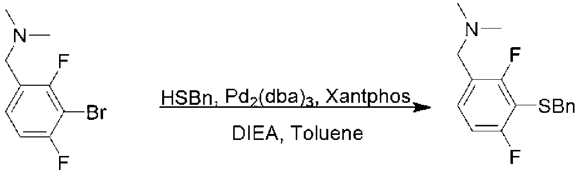

1-(3-bromo-2,4-difluorophenyl)-N,N-dimethylmethylamine (450 mg) was weighed out and placed into a 40 mL microwave tube. Toluene (15 mL) and benzyl mercaptan (2 eq.) were added, and DIEA (3 eq.), Pd₂(dba)₃ (0.1 eq.), and Xantphos (0.2 eq.) were added under a nitrogen atmosphere. The mixture was heated to 115 °C to react for 12 h. LCMS showed the formation of the target compound. Work-up and purification: The reaction solution was mixed with silica gel, rotary evaporated, and passed through flash column chromatography (DCM/MeOH) to yield a crude target compound, 400 mg of yellow oily liquid.

### Step 3: 3-((dimethylamino)methyl)-2,6-difluorobenzenesulfonamide

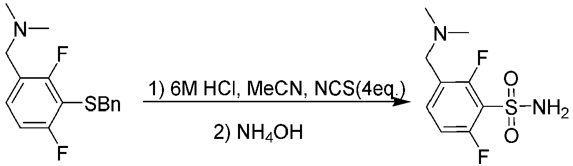

6 M HCl (0.4 mL) was measured out and placed into a 50 mL single-neck flask, and MeCN (6 mL) was added. The mixture was stirred and cooled to 0 °C, and added with NCS (4 eq.). 1-(3-(benzylmercapto)-2,4-difluorophenyl)-N,N-dimethylmethylamine (200 mg) was dissolved in 4 mL MeCN, and then added dropwise into the above reaction solution at 0 °C. The mixture was stirred for 0.5 h. NH₄OH (10 mL) was placed into another 100 mL single-neck flask, and the above reaction solution was added dropwise into NH₄OH via a constant-pressure dropping funnel while stirring at room temperature. The mixture was stirred for 0.5 h. LCMS confirmed the formation of the target compound. Work-up and purification: The reaction solution was rotary evaporated to remove MeCN, extracted twice with DCM:MeOH=10:1 (50 mL), dried, and concentrated under reduced pressure to yield the target compound, 200 mg of yellow oily crude product.

### Step 4: 3-((dimethylamino)methyl)-2,6-difluoro-N-((1,2,3,5,6,7-hexahydro-s-indacen-4-yl)carbamoyl)benzenesulfonamide

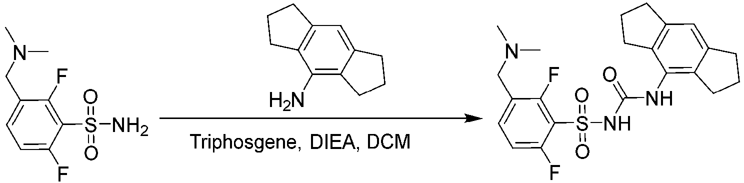

1,2,3,5,6,7-hexahydro-s-indacen-4-amine (120 mg) was weighed out and placed into a 40 mL single-neck flask. DCM (10 mL) and DIEA (5 eq.) were added, and triphosgene (0.4 eq.) was added batchwise while stirring at room temperature. The reaction solution was heated to 50 °C to react for 1 h. 800 mg of crude 3-((dimethylamino)methyl)-2,6-difluorobenzenesulfonamide was dissolved in DCM (5 mL) and DIEA (3 eq.). Once fully dissolved, the solution was added dropwise into the above reaction solution at 50 °C. The reaction was carried out at 50 °C for 2 h. LCMS confirmed the formation of the target compound. Work-up and purification: The reaction solution was directly mixed with silica gel and passed through flash column chromatography (DCM/MeOH) to yield a crude product. The crude product was subjected to preparative purification by flash column chromatography (0.05% NH₄HCO₃) and lyophilization to yield the target compound, 25 mg of white solid. MS: 450 [M+H]⁺. ¹H NMR (400 MHz, DMSO-*d*₆) δ 10.49 (s, 1H), 7.71 (s, 1H), 7.64 - 7.55 (m, 1H), 7.16 (t, *J* = 9.2 Hz, 1H), 6.81 (s, 1H), 3.94 (s, 2H), 2.75 (t, *J* = 7.2 Hz, 4H), 2.60 (t, *J* = 7.2 Hz, 4H), 2.48 (s, 6H), 1.95 - 1.84 (m, 4H).

### Example 26: 3-((dimethylamino)methyl)-N-((1,2,3,5,6,7-hexahydro-s-indacen-4-yl)carbamoyl)-2-methylbenzenesulfonamide

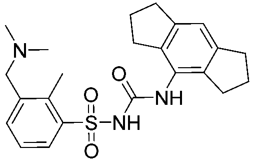

### Synthetic route:

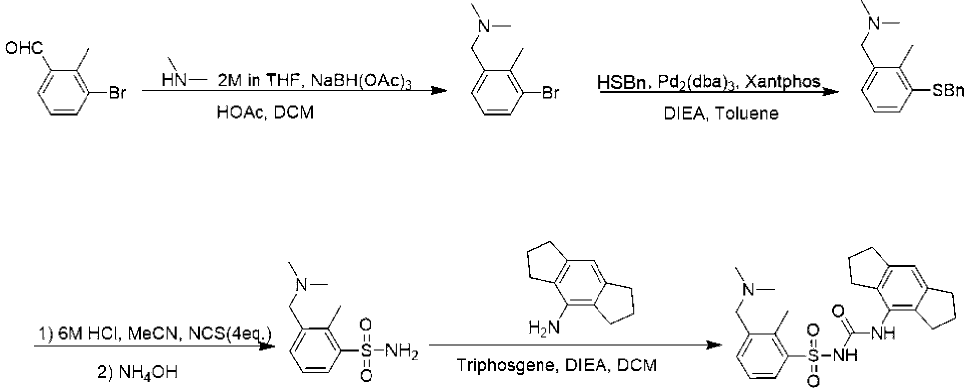

### Step 1: 1-(3-bromo-2-methylphenyl)-N,N-dimethylmethylamine

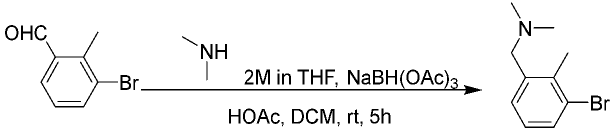

3-bromo-2-methylbenzaldehyde (500 mg) was weighed out and placed into a 100 mL single-neck flask. Then DCM (25 mL) was added, and dimethylamine (2 M in THF) (2 eq.) and HOAc (0.2 eq.) were added while stirring. The mixture was stirred at room temperature for 2 h. 2 h later, NaBH(OAc)₃ (3 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 v/v (50 mL), the organic phases were combined, washed with saturated brine, dried and rotary evaporated, and passed through flash column chromatography (DCM/MeOH) to yield the target compound, 400 mg of yellow oily liquid.

### Step 2: l-(3-(benzylmercapto)-2-methylphenyl)-N,N-dimethylmethanamine

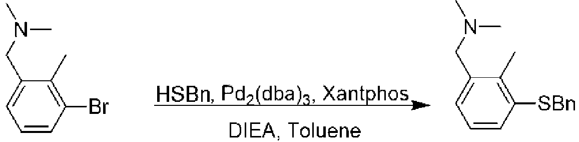

1-(3-bromo-2-methylphenyl)-N,N-dimethylmethylamine (400 mg) was weighed out and placed into a 40 mL microwave tube. Toluene (10 mL) and benzyl mercaptan (2 eq.) were added, and DIEA (3 eq.), Pd₂(dba)₃ (0.1 eq.), and Xantphos (0.2 eq.) were added under a nitrogen atmosphere. The mixture was heated to 115 °C to react for 12 h. LCMS showed the formation of the target compound. Work-up and purification: The reaction solution was mixed with silica gel, rotary evaporated, and passed through flash column chromatography (DCM/MeOH) to yield a crude target compound. The crude product was subjected to preparative purification by flash column chromatography (0.05% HCOOH) to yield 50 mg of yellow oily liquid.

### Step 3: 3-((dimethylamino)methyl)-2-methylbenzenesulfonamide

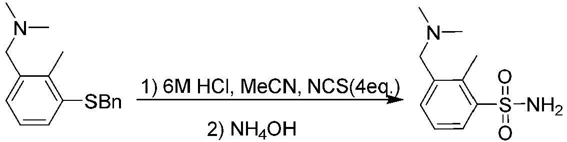

6 M HCl (0.2 mL) was measured out and placed into a 50 mL single-neck flask, and MeCN (5 mL) was added. The mixture was stirred and cooled to 0 °C, and added with NCS (3 eq.). 1-(3-(benzylmercapto)-2-methylphenyl)-N,N-dimethylmethylamine (50 mg) was dissolved in 3 mL MeCN, and then added dropwise into the above reaction solution at 0 °C. The mixture was stirred for 0.5 h. NH₄OH (8 mL) was placed into another 50 mL single-neck flask, and the above reaction solution was added dropwise into NH₄OH via a constant-pressure dropping funnel while stirring at room temperature. The mixture was stirred for 0.5 h. LCMS confirmed the formation of the target compound. Work-up and purification: The reaction solution was rotary evaporated to remove MeCN, extracted twice with DCM:MeOH=10:1 v/v (50 mL), dried, and concentrated under reduced pressure to yield the target compound, 45 mg of yellow oily crude product.

### Step 4: 3-((dimethylamino)methyl)-N-((1,2,3,5,6,7-hexahydro-s-indacen-4-yl)carbamoyl)-2-methylbenzenesulfonamide

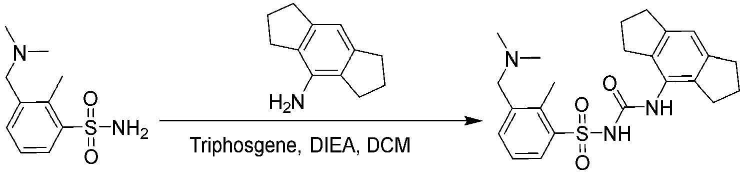

1,2,3,5,6,7-hexahydro-s-indacen-4-amine (50 mg) was weighed out and placed into a 40 mL single-neck flask. DCM (8 mL) and DIEA (5 eq.) were added, and triphosgene (0.4 eq.) was added batchwise while stirring at room temperature. The reaction solution was heated to 50 °C to react for 1 h. 45 mg of crude 3-((dimethylamino)methyl)-2-methylbenzenesulfonamide was dissolved in DCM (4 mL) and DIEA (3 eq.). Once fully dissolved, the solution was added dropwise into the above reaction solution at 50 °C. The reaction was carried out at 50 °C for 2 h. LCMS confirmed the formation of the target compound. Work-up and purification: The reaction solution was directly mixed with silica gel and passed through flash column chromatography (DCM/MeOH) to yield a crude product. The crude product was subjected to preparative purification by flash column chromatography (0.05% NH₄HCO₃) and lyophilization to yield the target compound, 80 mg of white solid.

MS: 428 [M+H]⁺.

¹H NMR (400 MHz, DMSO-*d*₆) δ 10.79 (s, 1H), 8.10 (s, 1H), 7.90 (d, *J* = 8.0 Hz, 1H), 7.51(d, *J* = 7.2 Hz, 1H), 7.32 (t, *J* = 8.0 Hz, 1H), 6.88 (s, 1H), 3.60 (s, 2H), 2.75 (t, *J* = 7.2 Hz, 4H), 2.66 (s, 3H), 2.52 (t, *J* = 7.2 Hz, 4H), 2.25 (s, 6H), 1.94 - 1.85 (m, 4H).

### Example 27: 3-((dimethylamino)methyl)-N-((1,2,3,5,6,7-hexahydro-s-indacen-4-yl)carbamoyl)-2-hydroxybenzenesulfonamide

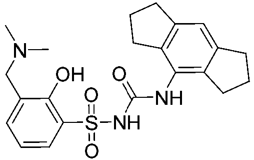

### Synthetic route:

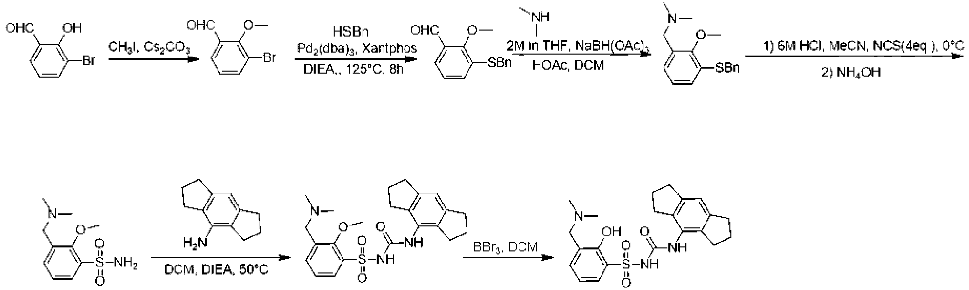

### Step 1: 3-bromo-2-methoxybenzaldehyde

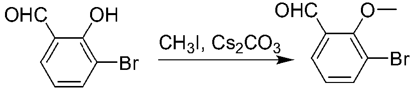

3-bromo-2-hydroxybenzaldehyde (1.8 g, 1 eq.) was weighed out and placed into a 100 mL single-neck flask. Then DMF (40 mL) was added and Cs₂CO₃ (5.85 g, 2 eq.) was added, and CH₃I (1.9 g, 1.5 eq.) was added while stirring at room temperature. The reaction was carried out at room temperature for 2 h. TLC showed the formation of the target compound. Work-up and purification: The reaction solution was poured into an appropriate amount of water, extracted with EA 60mL*2, washed twice with water, washed with saturated brine, dried, mixed with silica gel, rotary evaporated, and purified by flash column chromatography (PE/EA) to yield the target compound, 1.8 g of light yellow solid product.

### Step 2: 3-(benzylmercapto)-2-methoxybenzaldehyde

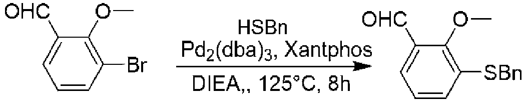

3-bromo-2-methoxybenzaldehyde (900 mg, 1 eq.) was weighed out and placed into a 40 mL single-neck flask, and xylene (10 mL), benzyl mercaptan (780 mg, 1.5 eq.), DIEA (1.65 g, 3 eq.), Pd₂(dba)₃ (385 mg, 0.1 eq.), and Xantphos (486 mg, 0.2 eq.) were added under a nitrogen atmosphere. The mixture was heated to 125 °C to react for 8 h. TLC showed the formation of the target compound. Work-up and purification: The reaction solution was mixed with silica gel, and rotary evaporated. The crude product was purified by flash column chromatography (PE/EA) to yield a crude target compound, 1.2 g of yellow oily liquid.

### Step 3: 1-(3-(benzylmercapto)-2-methoxyphenyl)-N,N-dimethylmethanamine

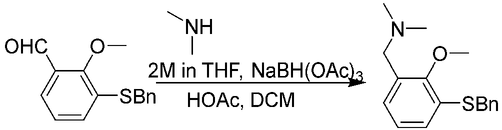

3-(benzylmercapto)-2-methoxybenzaldehyde (1.2 g, 1 eq.) was weighed out and placed into a 100 mL single-neck flask. Then DCM (30 mL) was added, and dimethylamine (2 M in THF) (4.6 mL, 2 eq.) and HOAc (55 mg, 0.2 eq.) were added while stirring. The mixture was stirred at room temperature for 3 h. 2 h later, NaBH(OAc)₃ (3.45 g, 3.5 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 (50 mL), the organic phases were combined, washed with saturated brine, dried and rotary evaporated, and passed through flash column chromatography (DCM/MeOH) to yield a crude target compound, 450 mg of yellow oily liquid.

### Step 4: 3-((dimethylamino)methyl)-2-methoxybenzenesulfonamide

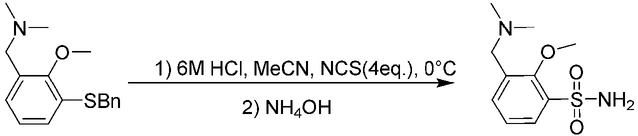

6 M HCl (1.5 mL) was measured out and placed into a 40 mL single-neck flask, and MeCN (6 mL) was added. The mixture was stirred and cooled to 0 °C, and added with NCS (625 mg, 3 eq.). 1-(3-(benzylmercapto)-2-methoxyphenyl)-N,N-dimethylmethanamine (450 mg, 1 eq.) was dissolved in 4 mL MeCN, and then added dropwise into the above reaction solution at 0 °C. The mixture was stirred for 0.5 h and used as a standby solution. NH₄OH (15 mL) was placed into another 100 mL single-neck flask. The above standby solution was added dropwise to NH₄OH at 0 °C while stirring at room temperature. After the addition, the mixture was heated to room temperature and stirred for 0.5 h. LCMS confirmed the formation of the target compound. Work-up and purification: The reaction solution was rotary evaporated to remove MeCN, extracted twice with DCM:MeOH=10:1 (30 mL), dried, and concentrated under reduced pressure to yield the target compound, 500 mg of yellow oily crude product.

### Step 5: 3-((dimethylamino)methyl)-N-((1,2,3,5,6,7-hexahydro-s-indacen-4-yl)carbamoyl)-2-methoxybenzenesulfonamide

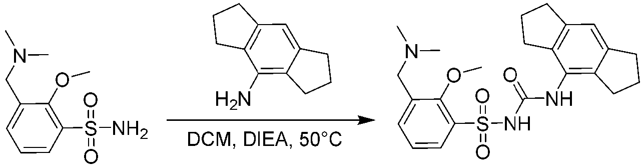

1,2,3,5,6,7-hexahydro-s-indacen-4-amine (354 mg, 1.0 eq.) was weighed out and placed into a 40 mL single-neck flask. DCM (10 mL) and DIEA (1.3 g, 5 eq.) were added, and triphosgene (242 mg, 0.4 eq.) was added batchwise while stirring at room temperature. After the addition, the reaction solution was heated to 50 °C to react for 1 h. Crude 3-((dimethylamino)methyl)-2-methoxybenzenesulfonamide (500 mg, 1.0 eq.) was dissolved in DCM (5 mL) and DIEA (790 mg, 3 eq.). Once fully dissolved, the solution was added dropwise into the above reaction solution at 50 °C. The reaction was carried out at 50 °C for 2 h. LCMS confirmed the formation of the target compound. Work-up and purification: The reaction solution was directly mixed with silica gel, and purified by flash column chromatography (DCM/MeOH) to yield 350 mg of off-white solid.

### Step 6: 3-((dimethylamino)methyl)-N-((1,2,3,5,6,7-hexahydro-s-indacen-4-yl)carbamoyl)-2-hydroxybenzenesulfonamide

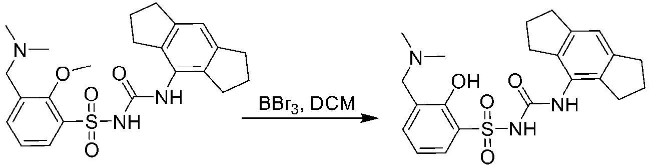

3-((dimethylamino)methyl)-N-((1,2,3,5,6,7-hexahydro-s-indacen-4-yl)carbamoyl)-2-methoxybenzenesulfonamide (200 mg, 1 eq.) was weighed out and placed into a 25 mL single-neck flask, and DCM (5 mL) was added. The mixture was cooled to 0 °C, and added dropwise with BBr₃ (15 drops). The mixture was heated to room temperature to react for 4 h. LCMS confirmed the formation of the target compound. Work-up and purification: 2 mL of methanol was added dropwise to the reaction solution in an ice-water bath to quench the reaction. 5 mL of MeCN was added. The mixture was subjected to rotary evaporation at low temperature to remove DCM. The remaining liquid was purified by flash column chromatography (0.05% HCOOH) and lyophilized to yield the target compound, 14 mg of white solid. MS: 430 [M+H]⁺. ¹H NMR (400 MHz, DMSO-d₆) δ 8.18 (s, 1H), 7.68 (d, J = 7.6Hz, 1H), 7.35 (d, J = 7.4, 1H), 6.86 - 6.78 (m, 2H), 4.15 (s, 2H), 2.76 (t, J = 7.4 Hz, 4H), 2.71 - 2.61 (m, 10 H), 1.98 - 1.86 (m, 4H).

### Example 28: 2-chloro-3-((dimethylamino)methyl)-N-((1,2,3,5,6,7-hexahydro-s-indacen-4-yl)carbamoyl)-4-methoxybenzenesulfonamide

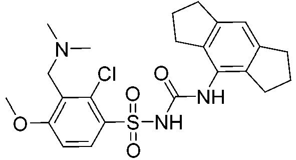

### Synthetic route:

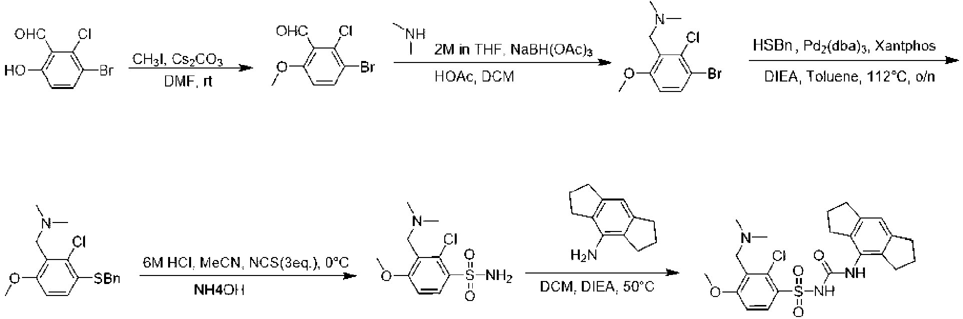

### Step 1: 3-bromo-2-chloro-6-methoxybenzaldehyde

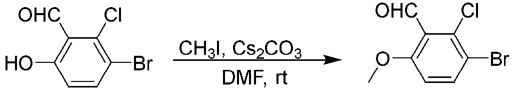

3-bromo-2-chloro-6-hydroxybenzaldehyde (610 mg, 1 eq.) was weighed out and placed into a 100 mL single-neck flask. Then DMF (40 mL) was added and Cs₂CO₃ (1.7 g, 2 eq.) was added, and CH₃I (556 mg, 1.5 eq.) was added while stirring at room temperature. The reaction was carried out at room temperature for 2 h. TLC showed the formation of the target compound. Work-up and purification: The reaction solution was poured into an appropriate amount of water, extracted with EA 40mL*2, washed twice with water, washed with saturated brine, dried, mixed with silica gel, rotary evaporated, and purified by flash column chromatography (PE/EA) to yield the target compound, 620 mg of light yellow solid product.

### Step 2: 1-(3-bromo-2-chloro-6-methoxyphenyl)-N,N-dimethylmethanamine

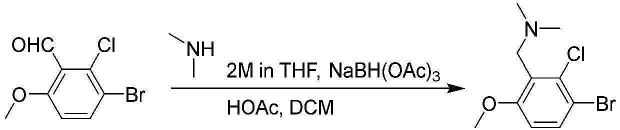

3-bromo-2-chloro-6-methoxybenzaldehyde (320 mg, 1 eq.) was weighed out and placed into a 100 mL single-neck flask. Then DCM (15 mL) was added, and dimethylamine (2 M in THF) (1.3 mL, 2 eq.) and HOAc (17 mg, 0.2 eq.) were added while stirring. The mixture was stirred at room temperature for 2 h. 2 h later, NaBH(OAc)₃ (1.1 g, 4 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 (50 mL), the organic phases were combined, washed with saturated brine, dried and rotary evaporated, and passed through flash column chromatography (DCM/MeOH) to yield a crude target compound, 200 mg of yellow oily liquid.

### Step 3: l-(3-(benzylmercapto)-2-chloro-6-methoxyphenyl)-N,N-dimethylmethanamine

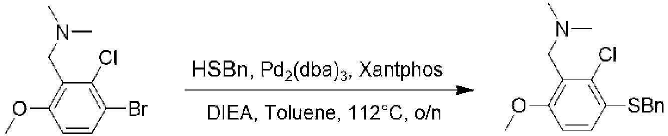

1-(3-bromo-2-chloro-6-methoxyphenyl)-N,N-dimethylmethylamine (200 mg, 1 eq.) was weighed out and placed into a 20 mL microwave tube. Toluene (5 mL), benzyl mercaptan (135 mg, 1.5 eq.), DIEA (279 mg, 3 eq.), Pd₂(dba)₃ (66 mg, 0.1 eq.), and Xantphos (83 mg, 0.2 eq.) were added under a nitrogen atmosphere. The mixture was heated to 112 °C to react for 24 h. LCMS showed the formation of the target compound. Work-up and purification: The reaction solution was mixed with silica gel, and rotary evaporated. The crude product was subjected to flash column chromatography (DCM/MeOH) to yield a crude target compound, 220 mg of yellow oily liquid.

### Step 4: 2-chloro-3-((dimethylamino)methyl)-4-methoxybenzenesulfonamide

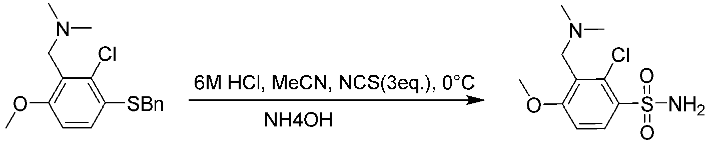

6 M HCl (0.5 mL) was measured out and placed into a 40 mL single-neck flask, and MeCN (4 mL) was added. The mixture was stirred and cooled to 0 °C, and added with NCS (274 mg, 3 eq.). 1-(3-(benzylmercapto)-2-chloro-6-methoxyphenyl)-N,N-dimethylmethanamine (220 mg, 1 eq.) was dissolved in 3mL MeCN, and then added dropwise into the above reaction solution at 0 °C. The mixture was stirred for 0.5 h and used as a standby solution. NH₄OH (10 mL) was placed into another 100 mL single-neck flask. The above standby solution was added dropwise to NH₄OH at 0 °C while stirring at room temperature. After the addition, the mixture was heated to room temperature and stirred for 0.5 h. LCMS confirmed the formation of the target compound. Work-up and purification: The reaction solution was rotary evaporated to remove MeCN, extracted twice with DCM:MeOH=10:1 (30 mL), dried, and concentrated under reduced pressure to yield the target compound, 240 mg of yellow oily crude product.

### Step 5: 2-chloro-3-((dimethylamino)methyl)-N-((1,2,3,5,6,7-hexahydro-s-indacen-4-yl)carbamoyl)-4-methoxybenzenesulfonamide

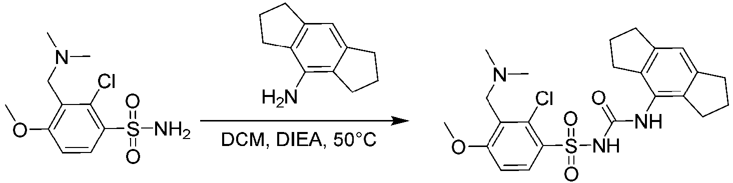

1,2,3,5,6,7-hexahydro-s-indacen-4-amine (150 mg, 1.0 eq.) was weighed out and placed into a 40 mL single-neck flask. DCM (6 mL) and DIEA (560 mg, 5 eq.) were added, and triphosgene (102 mg, 0.4 eq.) was added batchwise while stirring at room temperature. After the addition, the reaction solution was heated to 50 °C to react for 1 h. Crude 2-chloro-3-((dimethylamino)methyl)-4-methoxybenzenesulfonamide (240 mg, 1.0 eq.) was dissolved in DCM (3 mL) and DIEA (340 mg, 3 eq.). Once fully dissolved, the solution was added dropwise into the above reaction solution at 50 °C. The reaction was carried out at 50 °C for 2 h. LCMS confirmed the formation of the target compound. Work-up and purification: The reaction solution was directly mixed with silica gel, and purified by flash column chromatography (DCM/MeOH) to yield a crude product. The crude product was purified by flash column chromatography (0.05% NH₄HCO₃) and lyophilized to yield the target compound, 25 mg of white solid. MS: 478 [M+H]⁺. ¹H NMR (400 MHz, DMSO-d6) δ 10.34 (s, 1H), 8.02 (d, J = 9.2 Hz, 1H), 7.96 (s, 1H), 7.17 (d, J = 9.2 Hz, 1H), 6.87 (s, 1H), 3.89 (s, 3H), 3.79 (s, 2H), 2.75 (t, J = 7.6 Hz, 4H), 2.55 (t, J = 7.6 Hz, 4H), 2.33 (s, 6H), 1.96 - 1.84 (m, 4H).

### Example 29: N-((1,2,3,5,6,7-hexahydro-s-indacen-4-yl)carbamoyl)-2-methyl-1,2,3,4-tetrahydroisoquinoline-7-sulfonamide

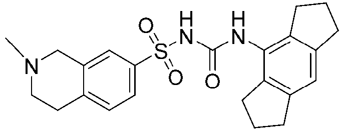

### Synthetic route:

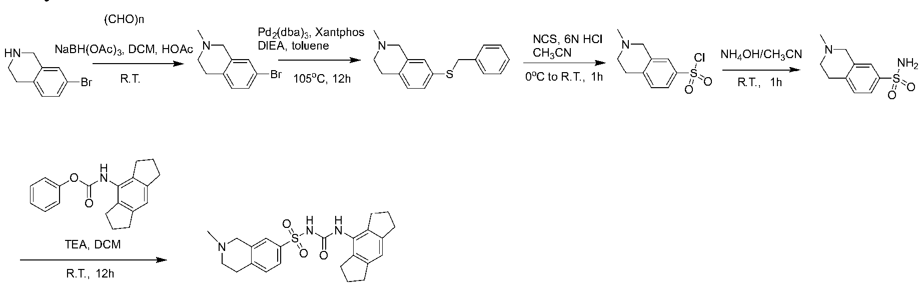

### Step 1: 7-bromo-2-methyl-1,2,3,4-tetrahydroisoquinoline

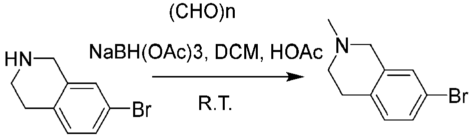

7-bromo-1,2,3,4-tetrahydroisoquinoline (2.5 g) was weighed out and placed into a 100 mL single-neck flask. Then paraformaldehyde (3.5 g) and DCM (50 mL) were added, and NaBH(OAc)₃ (7.5 g) was added batchwise and two drops of acetic acid was added. The mixture was stirred at room temperature for 12 h. Work-up and purification: LCMS showed the formation of the target compound, and showed that the raw materials were reacted completely. The system was diluted by adding an appropriate amount of water, and extracted with DCM (30 mL*3). The organic phases were combined, washed with saturated brine, dried with anhydrous sodium sulfate, and concentrated to yield a crude target compound, 2.2 g of white solid.

### Step 2: 7-(benzylmercapto)-2-methyl-1,2,3,4-tetrahydroisoquinoline

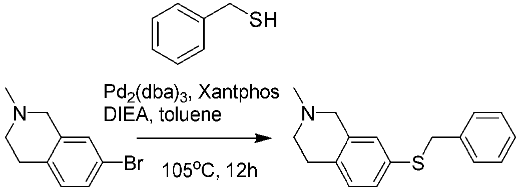

7-bromo-2-methyl-1,2,3,4-tetrahydroisoquinoline (1.0 g) was weighed out and placed into a 100 mL single-neck flask, and then benzyl mercaptan (658 mg), Pd₂(dba)₃ (200 mg, 0.05 eq.), XantPhos (128 mg), DIEA (1.14 g), and toluene (50 mL) were added respectively. The mixture was heated to 105 °C and stirred overnight. Work-up and purification: TLC confirmed that the raw materials were reacted completely. After cooling to room temperature, the reaction system was diluted by adding an appropriate amount of water, added with 6 N HCl solution and stirred for 30 min, and extracted with ethyl acetate. The phases were separated, and the organic phase was discarded. The aqueous phase was adjusted to basic pH with aqueous ammonia. The aqueous layer was extracted three times with ethyl acetate. The organic phases were combined, washed twice with saturated brine, dried with anhydrous Na₂SO₄, and concentrated to yield a crude target compound, 920 mg of yellow solid.

### Step 3: 2-methyl-1,2,3,4-tetrahydroisoquinoline-7-sulfonamide

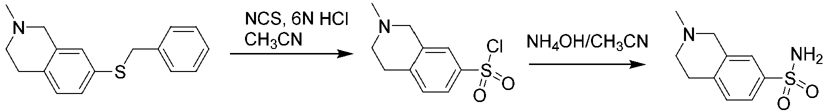

Acetonitrile (10 mL) and 6 N HCl (1.7 mL) aqueous solution were placed into a 50 mL single-neck flask. The mixture was cooled to below 0 °C. NCS (1.0 g) was added batchwise, and then a solution of 7-(benzylmercapto)-2-methyl-1,2,3,4-tetrahydroisoquinoline (500 mg) in acetonitrile was added dropwise. After stirring at room temperature for 1 h, the reaction solution was added dropwise to aqueous ammonia (30 mL) slowly, and stirring was continued at room temperature for 1 h. Work-up and purification: TLC confirmed that the raw materials were reacted completely. The reaction solution was concentrated and purified by column chromatography {dichloromethane/methanol} to yield the target product, 320 mg of white solid.

### Step 4: N-((1,2,3,5,6,7-hexahydro-s-indacen-4-yl)carbamoyl)-2-methyl-1,2,3,4-tetrahydroisoquinoline-7-sulfonamide

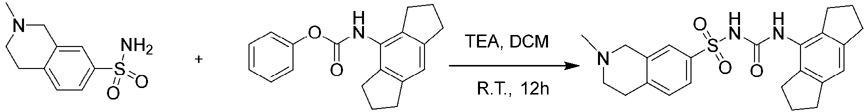

2-methyl-1,2,3,4-tetrahydroisoquinoline-7-sulfonamide (100 mg), phenyl (1,2,3,5,6,7-hexahydro-s-indacen-4-yl)carbamate (129 mg), triethylamine (90 mg), and DCM (20 mL) were weighed out and placed into a 100 mL single-neck flask. The mixture was stirred at room temperature for 12 h. Work-up and purification: LCMS confirmed the formation of the target compound, and confirmed that the raw materials were reacted completely. The mixture was concentrated, purified by prep-HPLC, and lyophilized to yield the trifluoroacetate salt of the product, 150 mg of white solid. MS: 426 [M+H]⁺. ¹H NMR (400 MHz, DMSO) δ 8.02 (s, 1H), 7.68 (dd, J = 8.1, 2.0 Hz, 1H), 7.63 (d, J = 2.0 Hz, 1H), 7.34 (d, J = 8.1 Hz, 1H), 6.90 (s, 1H), 3.72 (s, 2H), 2.93 (t, J = 6.0 Hz, 2H), 2.83 - 2.71 (m, 6H), 2.54 (t, J = 7.3 Hz, 4H), 2.46 (s, 3H), 1.92 (p, J = 7.4 Hz, 4H).

### Example 30: 3-((dimethylamino)methyl)-N-((1,2,3,5,6,7-hexahydro-s-indacen-4-yl)carbamoyl)-4-hydroxy-2-methylbenzenesulfonamide

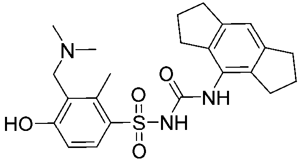

### Synthetic route:

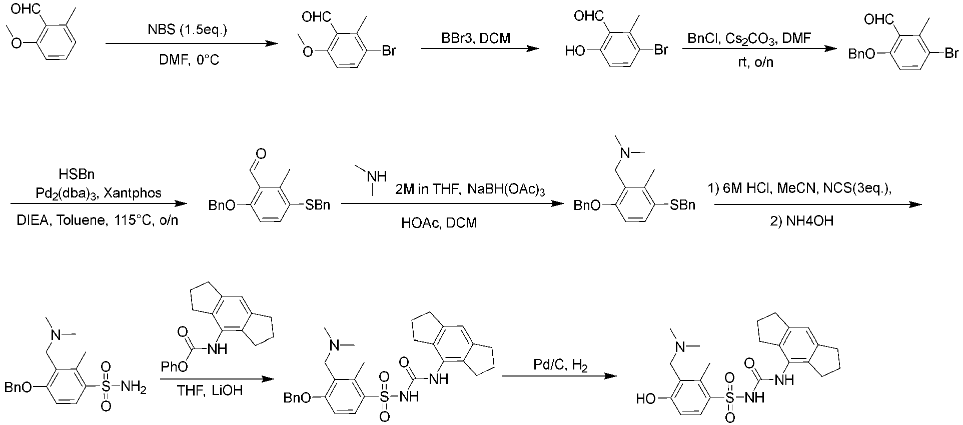

### Step 1: 3-bromo-6-methoxy-2-methylbenzaldehyde

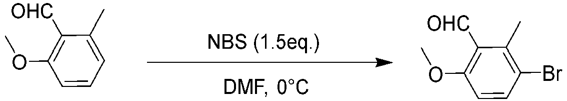

2-methoxy-6-methylbenzaldehyde (0.9 g, 1 eq.) was weighed out and placed into a 100 mL single-neck flask, then DMF (20 mL) was added. The mixture was cooled to 0 °C. NBS (1.6 g, 1.5 eq.) was added batchwise. After the addition, the mixture was kept at the temperature and reacted for 2 h. TLC showed the formation of the target compound. Work-up and purification: The reaction solution was poured into an appropriate amount of ice water, and extracted with EA 50 mL*2. The organic phase was washed with saturated brine, dried, rotary evaporated, and purified by flash column chromatography (PE/EA) to yield the target compound, 0.95 g of light yellow solid.

### Step 2: 3-bromo-6-hydroxy-2-methylbenzaldehyde

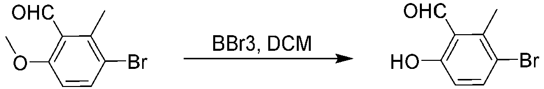

3-bromo-6-methoxy-2-methylbenzaldehyde (350 mg, 1 eq.) was weighed out and placed into a 25 mL single-neck flask, and then DCM (10 mL) was added. The mixture was cooled to 0 °C. BBr₃ (1.2 g, 3 eq.) was added dropwise. After the addition, the mixture was kept at the temperature and reacted for 4 h. LCMS showed the formation of the target compound. Work-up and purification: 0.5 mL methanol was added dropwise under an ice bath to quench the reaction. The reaction solution was poured into an appropriate amount of ice water, and extracted with DCM 30 mL*2. The organic phase was washed with saturated brine, dried, rotary evaporated, and purified by flash column chromatography (PE/EA) to yield the target compound, 250 mg of off-white solid.

### Step 3: 6-(benzyloxy)-3-bromo-2-methylbenzaldehyde

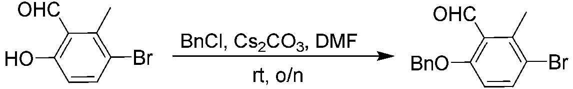

3-bromo-6-hydroxy-2-methylbenzaldehyde (250 mg, 1 eq.) was weighed out and placed into a 20 mL single-neck flask. Then DMF (6 mL) was added and Cs₂CO₃ (760 mg, 2 eq.) was added, and benzyl chloride (275 mg, 1.5 eq.) was added while stirring at room temperature. The mixture was reacted at room temperature overnight. TLC showed the formation of the target compound. Work-up and purification: The reaction solution was poured into an appropriate amount of water, extracted with EA 30 mL*2, washed twice with water, washed with saturated brine, dried, mixed with silica gel, rotary evaporated, and purified by flash column chromatography (PE/EA) to yield a crude target compound, 280 mg of light yellow oily liquid.

### Step 4: 6-benzyloxy-3-benzylmercapto-2-methylbenzaldehyde

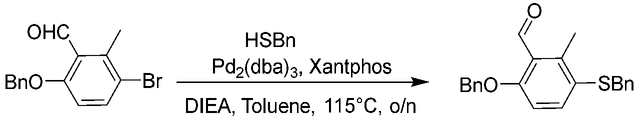

6-(benzyloxy)-3-bromo-2-methylbenzaldehyde (280 mg, 1 eq.) was weighed out and placed into a 20 mL microwave tube. Then toluene (5 mL), benzyl mercaptan (225 mg, 2 eq.), DIEA (475 mg, 4 eq.), Pd₂(dba)₃ (84 mg, 0.1 eq.), and Xantphos (105 mg, 0.2 eq.) were added under a nitrogen atmosphere. The mixture was heated to 115 °C to react for 24 h. TLC showed the formation of the target compound. Work-up and purification: The reaction solution was mixed with silica gel, and rotary evaporated. The crude product was purified by flash column chromatography (PE/EA) to yield the target compound, 200 mg of yellow oily liquid.

### Step 5: 1-(6-(benzyloxy)-3-(benzylmercapto)-2-methylphenyl)-N,N-dimethylmethanamine

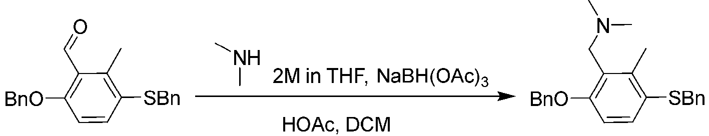

6-benzyloxy-3-benzylmercapto-2-methylbenzaldehyde (200 mg, 1 eq.) was weighed out and placed into a 40 mL single-neck flask. Then DCM (10 mL) was added, and dimethylamine (2 M in THF) (0.57 mL, 2 eq.) and HOAc (7 mg, 0.2 eq.) were added while stirring. After stirring at room temperature for 2 h, NaBH(OAc)₃ (425 mg, 3.5 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 (30 mL). The organic phases were combined, washed with saturated brine, dried, rotary evaporated, and passed through flash column chromatography (DCM/MeOH) to yield a crude target compound, 150 mg of yellow oily liquid.

### Step 6: 4-benzyloxy-3-((dimethylamino)methyl)-2-methylbenzenesulfonamide

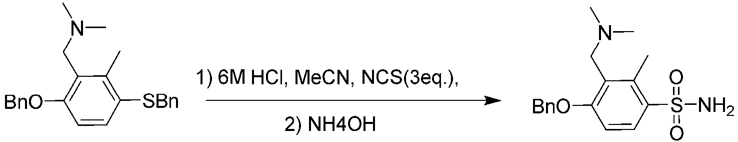

6 M HCl (0.4 mL) was measured out and placed into a 20 mL single-neck flask, and added with MeCN (6 mL). The mixture was stirred and cooled to 0 °C. NCS (158 mg, 3 eq.) was added. 1-(6-(benzyloxy)-3-(benzylmercapto)-2-methylphenyl)-N,N-dimethylmethanamine (150 mg, 1 eq.) was dissolved in 3 mL MeCN, and then added dropwise into the above reaction solution at 0 °C. The mixture was stirred for 0.5 h and used as a standby solution. NH₄OH (6 mL) was placed into another 40 mL single-neck flask. The standby solution was added dropwise into NH₄OH while stirring at room temperature. The mixture was stirred for 0.5 h. LCMS confirmed the formation of the target compound. Work-up and purification: The reaction solution was rotary evaporated to remove MeCN, extracted twice with DCM:MeOH=10:1 (30 mL), dried, and concentrated under reduced pressure to yield the target compound, 180 mg of yellow oily crude product.

### Step 7: 4-(benzyloxy)-3-((dimethylamino)methyl)-N-((1,2,3,5,6,7-hexahydro-s-indacen-4-yl)carbamoyl)-2-methylbenzenesulfonamide

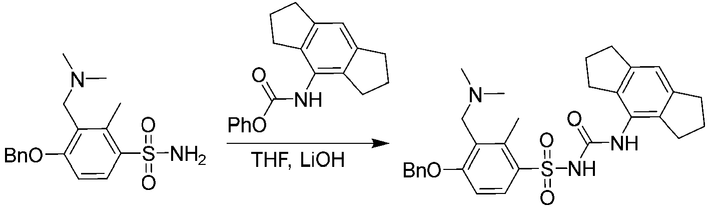

4-benzyloxy-3-((dimethylamino)methyl)-2-methylbenzenesulfonamide (180 mg, 1 eq.) was weighed out and placed into a 25 mL single-neck flask. THF (6 mL) was added and then phenyl (1,2,3,5,6,7-hexahydro-s-indacen-4-yl)carbamate (126 mg, 0.8 eq.) was added, and LiOH (20 mg, 1.5 eq.) was added while stirring at room temperature. The mixture was stirred at room temperature for 2 h. LCMS confirmed the formation of the target compound. Work-up and purification: The reaction solution was filtered, and rotary evaporated. The crude product was purified by TLC (DCM/MeOH=10:1) to yield 70 mg of yellow solid product.

### Step 8: 3-((dimethylamino)methyl)-N-((1,2,3,5,6,7-hexahydro-s-indacen-4-yl)carbamoyl)-4-hydroxy-2-methylbenzenesulfonamide

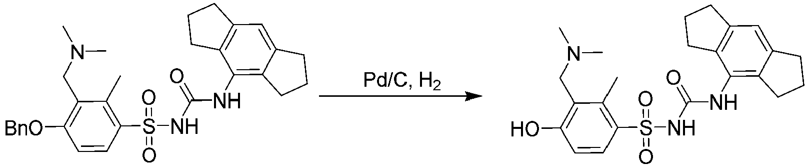

4-(benzyloxy)-3-((dimethylamino)methyl)-N-((1,2,3,5,6,7-hexahydro-s-indacen-4-yl))carbamoyl)-2-methylbenzenesulfonamide (70 mg, 1 eq.) was weighed out and placed into a 25 mL single-neck flask, and added with EtOH (3 mL) and DCM (3 mL), and was undissolved. Pd/C (60% of water content, 50 mg) was added while stirring at room temperature. The flask was purged with H₂. The mixture was stirred at room temperature for 3 h. LCMS confirmed the formation of the target compound. Work-up and purification: The reaction solution was filtered with celite. The filter cake was rinsed with DCM:MeOH=2:1, 50 mL. The filtrate was rotary evaporated, purified by flash column chromatography (0.5‰ TFA), and lyophilized to yield the target compound, 20 mg of white solid. MS: 444 [M+H]⁺. ¹H NMR (400 MHz, DMSO-d6) δ 11.88 - 10.71 (m, 2H), 9.00 (s, 1H), 8.24 (s, 1H), 7.97 (d, J = 8.8 Hz, 1H), 6.97 (d, J = 8.8 Hz, 1H), 6.94 (s, 1H), 4.39 (s, 2H), 2.83 - 2.76 (m, 10H), 2.69 (s, 3H), 2.54(t, J = 6.4 Hz, 4H), 1.98 - 1.87 (m, 4H).

### Example 31: 3-((dimethylamino)methyl)-2-fluoro-N-((1,2,3,5,6,7-hexahydro-s-indacen-4-yl)carbamoyl)-4-hydroxybenzenesulfonamide

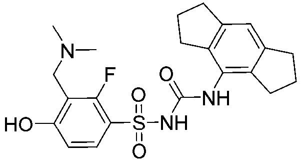

### Synthetic route:

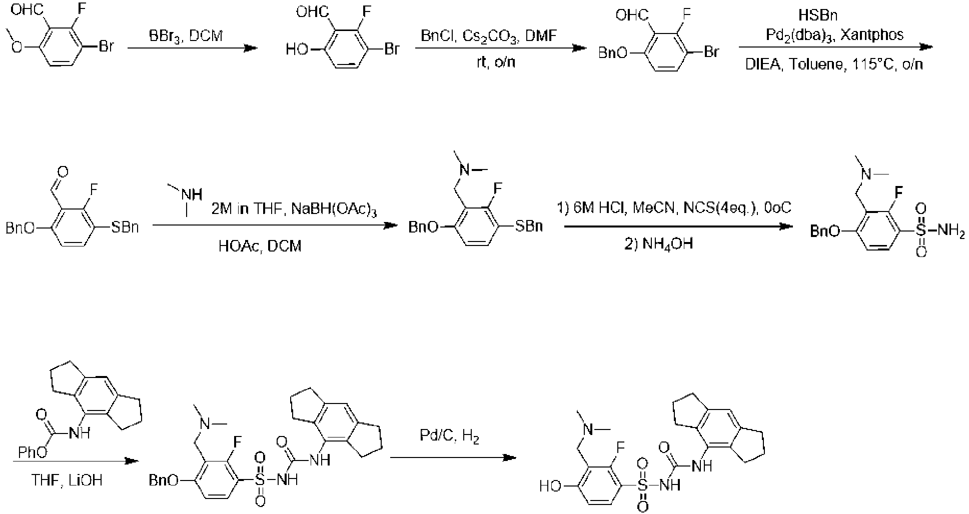

### Step 1: 3-bromo-2-fluoro-6-hydroxybenzaldehyde

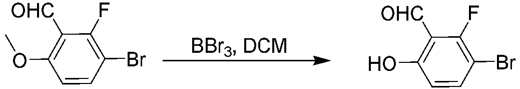

3-bromo-2-fluoro-6-methoxybenzaldehyde (350 mg, 1 eq.) was weighed out and placed into a 25 mL single-neck flask, and then added with DCM (10 mL). The mixture was cooled to 0 °C. BBr₃ (1.15 g, 3 eq.) was added dropwise. After the addition, the mixture was kept at the temperature and reacted for 4 h. LCMS showed the formation of the target compound. Work-up and purification: 0.5 mL methanol was added dropwise under an ice bath to quench the reaction. The reaction solution was poured into an appropriate amount of ice water, and extracted with DCM 30 mL*2. The organic phase was washed with saturated brine, dried, rotary evaporated, and purified by flash column chromatography (PE/EA) to yield the target compound, 280 mg of off-white solid.

### Step 2: 6-(benzyloxy)-3-bromo-2-fluorobenzaldehyde

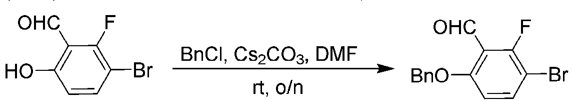

3-bromo-2-fluoro-6-hydroxybenzaldehyde (280 mg, 1 eq.) was weighed out and placed into a 20 mL single-neck flask. Then DMF (6 mL) was added and Cs₂CO₃ (835 mg, 2 eq.) was added, and BnCl (305 mg, 1.5 eq.) was added while stirring at room temperature. The mixture was reacted at room temperature overnight. TLC showed the formation of the target compound. Work-up and purification: The reaction solution was poured into an appropriate amount of water, extracted with EA 30 mL*2, washed twice with water, washed with saturated brine, dried, mixed with silica gel, rotary evaporated, and purified by flash column chromatography (PE/EA) to yield a crude target compound, 250 mg of light yellow oily liquid.

### Step 3: 6-(benzyloxy)-3-(benzylmercapto)-2-fluorobenzaldehyde

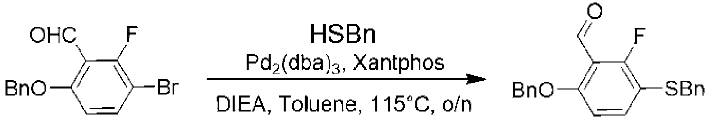

6-(benzyloxy)-3-bromo-2-fluorobenzaldehyde (240 mg, 1 eq.) was weighed out and placed into a 20 mL microwave tube. Then toluene (5 mL), benzyl mercaptan (190 mg, 2 eq.), DIEA (402 mg, 4 eq.), Pd₂(dba)₃ (70 mg, 0.1 eq.), and Xantphos (90 mg, 0.2 eq.) were added under a nitrogen atmosphere. The mixture was heated to 115 °C to react for 24 h. TLC showed the formation of the target compound. Work-up and purification: The reaction solution was mixed with silica gel, and rotary evaporated. The crude product was purified by flash column chromatography (PE/EA) to yield the target compound, 150 mg of yellow oily liquid.

### Step 4: 1-(6-(benzyloxy)-3-(benzylmercapto)-2-fluorophenyl)-N,N-dimethylformamide

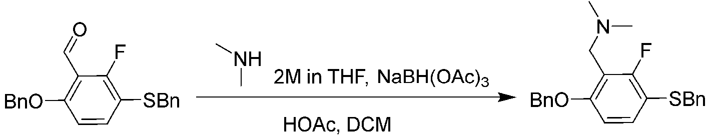

6-(benzyloxy)-3-(benzylmercapto)-2-fluorobenzaldehyde (150 mg, 1 eq.) was weighed out and placed into a 40 mL single-neck flask. Then DCM (10 mL) was added, and dimethylamine (2 M in THF) (0.4 mL, 2 eq.) and HOAc (6 mg, 0.2 eq.) were added while stirring. The mixture was stirred at room temperature for 2 h. 2 h later, NaBH(OAc)₃ (271 mg, 3 eq.) was added batchwise to the reaction solution at room temperature. The mixture was stirred at room temperature for 2 h. LCMS showed the formation of the target compound. Work-up and purification: The reaction solution was poured into an appropriate amount of ice water, and extracted twice with DCM:MeOH=10:1 (30 mL). The organic phases were combined, washed with saturated brine, dried, rotary evaporated, and passed through flash column chromatography (DCM/MeOH) to yield a crude target compound, 80 mg of yellow oily liquid.

### Step 5: 4-(benzyloxy)-3-((dimethylamino)methyl)-2-fluorobenzenesulfonamide

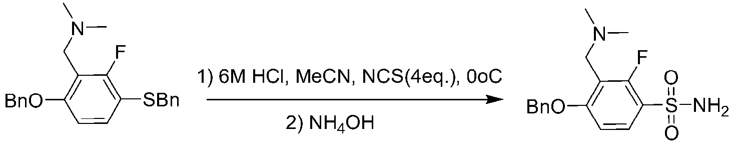

6 M HCl (0.2 mL) was measured out and placed into a 20 mL single-neck flask, and add with MeCN (6 mL). The mixture was stirred and cooled to 0 °C. NCS (84 mg, 3 eq.) was added. 1-(6-(benzyloxy)-3-(benzylmercapto)-2-fluorophenyl)-N,N-dimethylformamide (80 mg, 1 eq.) was dissolved in 3 mL MeCN, and then added dropwise into the above reaction solution at 0 °C. The mixture was stirred for 0.5 h and used as a standby solution. Aqueous ammonia (5 mL) was placed into another 40 mL single-neck flask. The standby solution was added dropwise into aqueous ammonia while stirring at room temperature. The mixture was stirred for 0.5 h. LCMS confirmed the formation of the target compound. Work-up and purification: The reaction solution was rotary evaporated to remove MeCN, extracted twice with DCM:MeOH=10:1 (30 mL), dried, and concentrated under reduced pressure to yield the target compound, 100 mg of yellow oily crude product.

### Step 6: 4-(benzyloxy)-3-((dimethylamino)methyl)-2-fluoro-N-((1,2,3,5,6,7-hexahydro-s-indacen-4-yl)carbamoyl)benzenesulfonamide

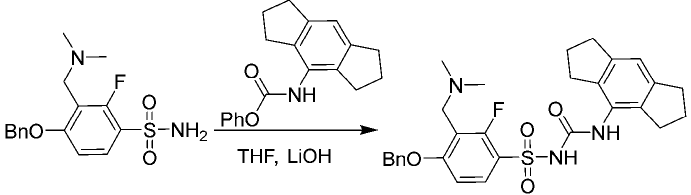

4-(benzyloxy)-3-((dimethylamino)methyl)-2-fluorobenzenesulfonamide (100 mg, 1 eq.) was weighed out and placed into a 25 mL single-neck flask. THF (5 mL) was added and then phenyl (1,2,3,5,6,7-hexahydro-s-indacen-4-yl)carbamate (70 mg, 0.8 eq.) was added, and LiOH (11 mg, 1.5 eq.) was added while stirring at room temperature. The mixture was stirred at room temperature for 2 h. LCMS confirmed the formation of the target compound. Work-up and purification: The reaction solution was filtered, and rotary evaporated. The crude product was purified by TLC (DCM/MeOH=10:1) to yield 80 mg of yellow solid product.

### Step 7: 3-((dimethylamino)methyl)-2-fluoro-N-((1,2,3,5,6,7-hexahydro-s-indacen-4-yl)carbamoyl)-4-hydroxybenzenesulfonamide

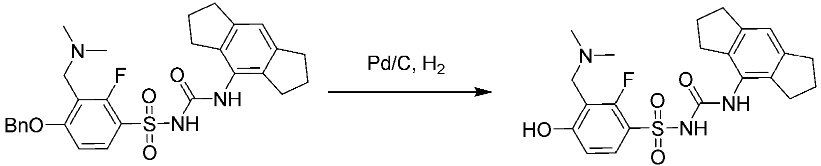

4-(benzyloxy)-3-((dimethylamino)methyl)-2-fluoro-N-((1,2,3,5,6,7-hexahydro-s-indacene-4-yl)carbamoyl)benzenesulfonamide (80 mg, 1 eq.) was weighed out and placed into a 25 mL single-neck flask, added with ethanol (3 mL) and DCM (3 mL), and was undissolved. Pd/C (60% of water content, 50 mg) was added while stirring at room temperature. The flask was purged with H₂. The mixture was stirred at room temperature for 3 h. LCMS confirmed the formation of the target compound. Work-up and purification: The reaction solution was filtered with celite. The filter cake was rinsed with DCM:MeOH=2:1, 50 mL. The filtrate was rotary evaporated, purified by flash column chromatography (0.5 ‰ TFA), and lyophilized to yield the target compound, 20 mg of white solid. MS: 448 [M+H]⁺. ¹H NMR (400 MHz, DMSO-d6) δ 12.80 - 10.52 (m, 1H), 9.63 (s, 1H), 8.38 - 8.23 (m, 1H), 7.84 (t, J = 8.8 Hz, 1H), 6.98 - 6.90 (m, 2H), 4.31 (s, 2H), 2.83 - 2.74 (m, 10H), 2.54 (t, J = 7.6 Hz, 4H), 1.98 - 1.87 (m, 4H).

### Example 32: 4-(dimethylamino)-N-((1,2,3,5,6,7-hexahydro-s-indacen-4-yl)carbamoyl)chromane-8-sulfonamide

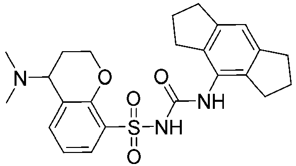

### Synthetic route:

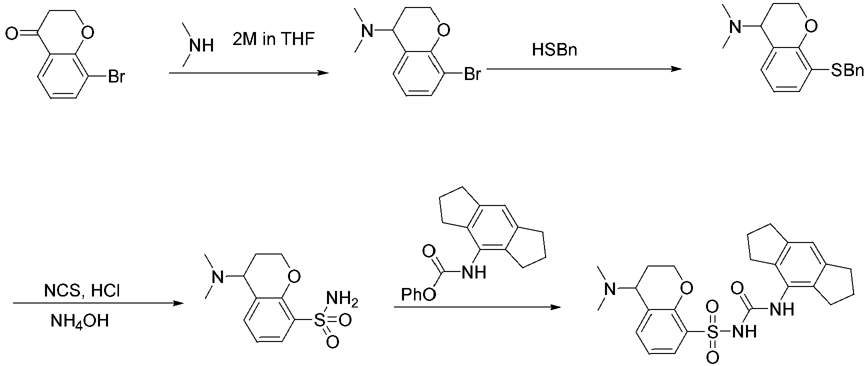

### Step 1: 8-bromo-N,N-dimethylchroman-4-amine

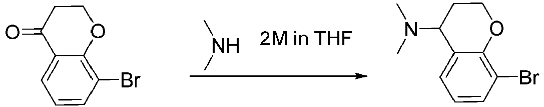

8-bromochroman-4-one (240 mg, 1 eq.) was weighed out and placed into a 20 mL microwave tube, added with MeOH (3 mL), dimethylamine (2 M in THF, 1 mL, 2 eq.), zinc chloride (1 M in THF, 4.5 mL 4 eq.), and NaBH₃CN (290 mg, 4 eq.), and then sealed tightly. The mixture was heated to 65 °C to react for 72 h. LCMS showed the formation of the target compound. Work-up and purification: The reaction solution was added to 20 mL of water, extracted with DCM:MeOH=10:1 (30 mL*2), dried, rotary evaporated, and purified by reversed-phase flash column chromatography (acetonitrile/water+0.05% HCOOH) to yield 60 mg of light yellow oily product.

### Step 2: 8-(benzylmercapto)-N,N-dimethylchroman-4-amine

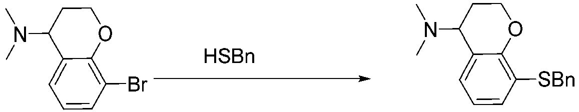

8-bromo-N,N-dimethylchroman-4-amine (60 mg, 1 eq.) was weighed out and placed into a 20 mL microwave tube. Then toluene (3 mL), benzyl mercaptan (58 mg, 2 eq.), DIEA (150 mg, 5 eq.), Pd₂(dba)₃ (21 mg, 0.1 eq.), and Xantphos (27 mg, 0.2 eq.) were added under a nitrogen atmosphere. The mixture was heated to 115 °C to react for 24 h. LCMS showed the formation of the target compound. Work-up and purification: The reaction solution was filtered, and rotary evaporated. The crude product was purified by flash column chromatography (acetonitrile/water + 0.05% HCOOH) to yield the target compound, 48 mg of light yellow oily product.

### Step 3: 4-(dimethylamino)chromane-8-sulfonamide

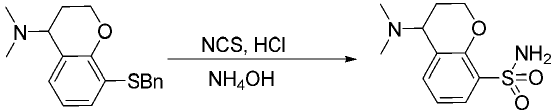

6 M HCl (0.12 mL) was measured out and placed into a 20 mL single-neck flask, and added with MeCN (3 mL). The mixture was stirred and cooled to 0 °C. NCS (64 mg, 3 eq.) was added. 8-(benzylmercapto)-N,N-dimethylchroman-4-amine (48 mg, 1 eq.) was dissolved in 2 mL MeCN, and then added dropwise into the above reaction solution at 0 °C. The mixture was stirred for 15 min and used as a standby solution. Aqueous ammonia (4 mL) was placed into another 40 mL single-neck flask. The standby solution was added dropwise into aqueous ammonia at 0 °C while stirring. After the addition, the mixture was heated to room temperature and stirred for 20 min. LCMS confirmed the formation of the target compound. Work-up and purification: The reaction solution was rotary evaporated to remove MeCN, extracted with DCM:MeOH=9:1 (30 mL*2), dried, and concentrated under reduced pressure to yield the target compound, 80 mg of yellow oily crude product.

### Step 4: 4-(dimethylamino)-N-((1,2,3,5,6,7-hexahydro-s-indacen-4-yl)carbamoyl)chromane-8-sulfonamide

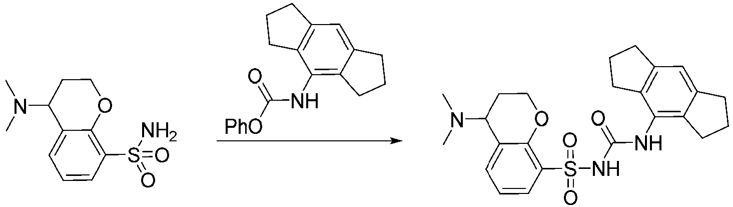

4-(dimethylamino)chromane-8-sulfonamide (80 mg, 1 eq.) was weighed out and placed into a 25 mL single-neck flask. THF (6 mL) was added, and phenyl (1,2,3,5,6,7-hexahydro-s-indacen-4-yl)carbamate (73 mg, 0.8 eq.) and LiOH (12 mg, 1.5 eq.) were added while stirring at room temperature. The mixture was stirred at room temperature for 2 h. LCMS confirmed the formation of the target compound. Work-up and purification: 1 mL MeOH was added to the reaction solution to aid dissolution. The mixture was subjected to preparative purification by flash column chromatography (NH₄HCO₃ 0.05%) and lyophilization to yield the target compound, 13 mg of white solid. MS: 456 [M+H]⁺. ¹H NMR (400 MHz, DMSO-d6) δ 10.28 (s, 1H), 8.01 (s, 1H), 7.66 (d, J = 7.8 Hz, 2H), 7.01 (t, J = 7.8 Hz, 1H), 6.91 (s, 1H), 4.53 - 4.46 (m, 1H), 4.33 - 4.24 (m, 1H), 3.86 - 3.79 (m, 1H), 2.76 (t, J = 7.4 Hz, 4H), 2.52 (t, J = 6.8 Hz, 4H) 2.20 (s, 6H), 2.01 - 1.85 (m, 6H).

### Biological evaluation: Study on NLRP3 inhibitory activity of the compounds of the present invention

### Contents of experiments:

1) The mouse macrophage cell line J774A.1 was used, and LPS was used as the first signal and ATP was used as the second signal to activate NLRP3. NLRP3 will release mature IL-1β after being activated, and NLRP3 activation can be determined by detecting IL-1β via ELISA. Based on this, the compounds were added to observe their effects on IL-1β release.
2) The mouse macrophage cell line J774A.1 was used, and APOE peptide was used as the first and second signals to activate NLRP3. NLRP3 will release mature IL-1β after being activated, and NLRP3 activation can be determined by detecting IL-1β via ELISA. Based on this, the compounds were added to observe their effects on IL-1β release.
3) The mouse macrophage cell line J774A.1 was used, and LPS was used as the first signal and ATP was used as the second signal to activate NLRP3. NLRP3 will release mature IL-1β after being activated, and NLRP3 activation can be determined by detecting IL-1β via ELISA. Based on this, different concentrations of the same compounds were added to plot their inhibition curves.
4) Human monocyte cell line THP-1 was used and induced to differentiate into monocyte-macrophage-like cells using PMA, and LPS was used as the first signal and Nig (nigericin) was used as the second signal to activate NLRP3. NLRP3 will release mature IL-1β after being activated, and NLRP3 activation can be determined by detecting IL-1β via ELISA. Based on this, the compounds were added to observe their effects on IL-1β release.
5) Human monocyte cell line THP-1 was used and induced to differentiate into monocyte-macrophage-like cells using PMA, and LPS was used as the first signal and Nig (nigericin) was used as the second signal to activate NLRP3. NLRP3 will release mature IL-1β after being activated, and NLRP3 activation can be determined by detecting IL-1β via ELISA. Based on this, different concentrations of the same compounds were added to plot their inhibition curves.
6) Human whole blood was used, and LPS was used as the first signal and nigericin was used as the second signal to activate NLRP3. NLRP3 will release mature IL-1β after being activated, and NLRP3 activation can be determined by detecting IL-1β via ELISA. Based on this, different concentrations of the same compounds were added to plot their inhibition curves.
7) In the LPS-induced sepsis model in C57 mice, LPS injection will cause NLRP3 activation and IL-1β release, affecting mouse survival. Based on this, the compounds were added preemptively to observe their effects on IL-1β release and on mouse survival rate.
8) The compounds were administered to mice by tail vein injection, and pharmacokinetic properties of the compounds could be analyzed by determining their concentrations in plasma, liver, and brain tissues.
9) The determination of hERG inhibition.

### Experimental Example 1: The inhibition of the compounds on canonical activation of NLRP3 in the murine cell line J774A.1

J774A.1 cells were plated in a 48-well plate at a density of 1.0×10⁶ cells/mL, with 300 µL per well. Subsequently, NLRP3 was activated using LPS+5 mM ATP (adenosine triphosphate) or 10 µM Nig (nigericin). The cell culture medium comprises: DMEM (brand: Gibco, Cat. No.: C12430500) + 10% fetal bovine serum (brand: Gibco, Cat. No.: 10099-141) + 1% sodium pyruvate (brand: Gibco, Cat. No.: 11360-070) + 1% penicillin-streptomycin solution (brand: Solarbio, Cat. No.: P1400). The J774A.1 mouse monocyte-macrophage cell line was purchased from the Cell Resource Center of the Institute of Basic Medical Sciences of Chinese Academy of Medical Sciences (Resource No. 1101MOU-PUMC000222). ATP was purchased from Sigma, Cat. No.: L2654. Nig was purchased from Shanghai Yuanye Bio-Technology Co., Ltd., Cat. No.: S45490.

Experimental groups: a negative control group, an LPS-alone incubation control group, an LPS+ATP or +Nig positive control group, and an example compound experimental group.

After the plated J774A.1 cells adhering to the plate, the culture medium was replaced. For all the groups except the negative control group, the culture medium was completely replaced with cell culture medium containing 1 µg/mL LPS. The negative control group received cell culture medium. Then, all the groups were incubated overnight. 18 h later, the culture medium was replaced again. The culture medium of the example compound experimental group was completely replaced with cell culture medium containing the compounds; while the negative control group, the LPS-alone incubation control group, and the LPS+ATP/Nig positive control group received cell culture medium. 1 h later, the culture mediums of the LPS+ATP/Nig positive control group and the example compound experimental group were completely replaced with cell culture medium containing 5 mM ATP or 10 µM Nig. 1 h later, the cell supernatants were collected for IL-1β detection.

### Experimental results

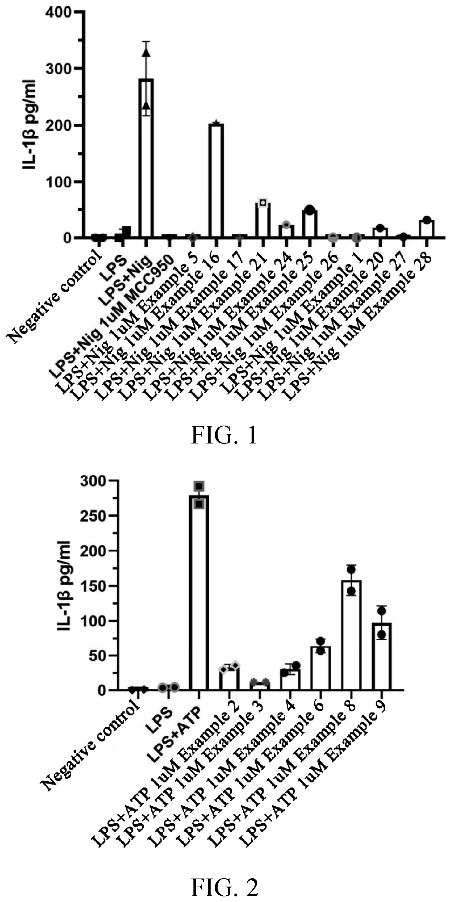

Activation of NLRP3 by LPS+ATP/Nig will result in release of mature IL-1β, and the release can be inhibited by NLRP3 inhibitors. Therefore, the relative change in the amount of IL-1β release may directly reflect NLRP3 inhibitory effect. The results are shown in FIGS. 1-4.

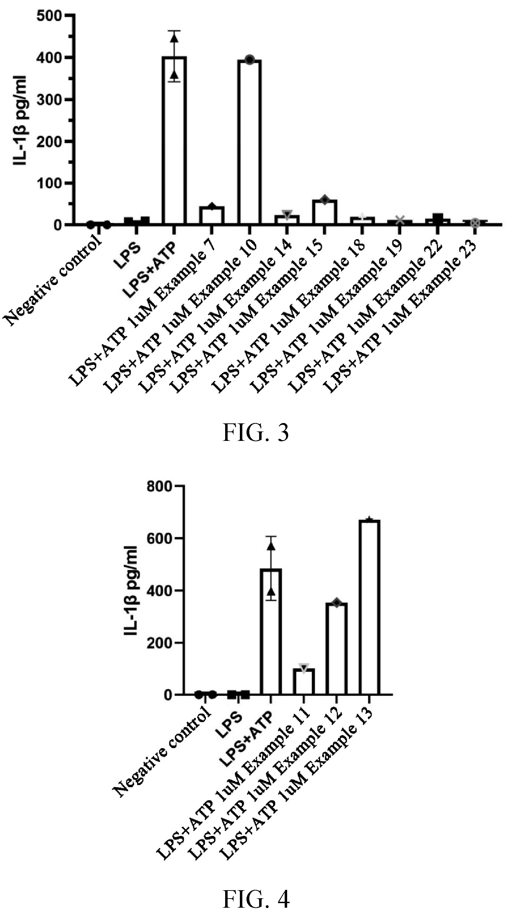

In this experimental example, effectively inhibiting the release of IL-1β means that the release amount is reduced by 80% or more, compared with the positive control, and with a weaker inhibitory effect means that the release amount is reduced by 20%-80%, compared with the positive control. The results show that Example 16 has a weaker inhibitory effect on IL-1β release at the concentration of 1 µM; MCC950, Example 1, Example 5, Example 17, Example 20, Example 21, Example 24, Example 25, Example 26, Example 27, and Example 28 can effectively inhibit IL-1β release at the concentration of 1 µM (FIG. 1). Example 2, Example 3, Example 4, and Example 6 can effectively inhibit IL-1β release at the concentration of 1 µM; while Example 8 and Example 9 only have weaker inhibitory effects at the concentration of 1 µM (FIG. 2). Example 7, Example 14, Example 15, Example 18, Example 19, Example 22, and Example 23 can effectively inhibit IL-1β release at the concentration of 1 µM; while Example 10 has no inhibitory effect at the concentration of 1 µM (FIG. 3). Example 11 can effectively inhibit IL-1β release at the concentration of 1 µM, Example 12 has a weaker inhibitory effect on IL-1β release at the concentration of 1 µM, and Example 13 has no inhibitory effect at the concentration of 1 µM (FIG. 4).

### Experimental Example 2: The inhibition of the compounds on APOE peptide-induced NLRP3 activation in the murine cell line J774A.1

J774A.1 cells were plated in a 48-well plate at a density of 1.0×10⁶ cells/mL, with 300 µL per well. Subsequently, NLRP3 was activated by APOE peptide. The cell culture medium comprises: DMEM (brand: Gibco, Cat. No.: C12430500) + 10% fetal bovine serum (brand: Gibco, Cat. No.: 10099-141) + 1% sodium pyruvate (brand: Gibco, Cat. No.: 11360-070) + 1% penicillin-streptomycin solution (brand: Solarbio, Cat. No.: P1400). The J774A.1 mouse monocyte-macrophage cell line was purchased from the Cell Resource Center of the Institute of Basic Medical Sciences of Chinese Academy of Medical Sciences (Resource No. 1101MOU-PUMC000222). APOE2/3 peptide 1-151 was obtained through recombinant protein expression and purification by Biocells (Beijing) Biotech Co., Ltd. The procedures were as follows: the sequence (SEQ ID NO: 1) shown below was inserted into the pET-28A plasmid and transformed into BL-21(DE3) expression strain; the expression of target protein was induced with IPTG at a low temperature; after collecting the bacteria via centrifugation, ultrasonic disruption was performed; and after centrifugation, the supernatant was purified by nickel ion affinity chromatography (AKTA pure), and then the product was concentrated by ultrafiltration and identified by BCA+protein electrophoresis. Sequence:

CCATGGGCCATCATCATCATCATCACGGTGGCTCCGGCGGTAGCAAGGTTGAACAG
GCTGTTGAAACCGAACCGGAACCGGAACTGCGTCAGCAAACCGAGTGGCAGTCTG
GCCAGCGTTGGGAGCTGGCGCTGGGCCGCTTTTGGGACTACCTGCGTTGGGTACAG
ACTCTGTCCGAACAGGTTCAGGAAGAACTGCTGTCTTCCCAGGTAACCCAGGAGCT
GCGCGCACTGATGGACGAGACCATGAAAGAACTGAAAGCCTACAAGTCCGAACTG
GAAGAACAGCTGACGCCGGTTGCGGAAGAAACTCGTGCTCGCCTGTCTAAAGAAC
TGCAGGCTGCGCAGGCGCGCCTGGGCGCGGATATGGAAGATGTTTGCGGTCGTCTG
GTACAGTACCGTGGCGAAGTTCAAGCTATGCTGGGCCAGAGCACCGAAGAACTGC
GTGTTCGCCTGGCATCCCACCTGCGCAAACTGCGCAAACGTCTGCTGCGTGACTAA
CTCGAG (SEQ ID NO: 1).

Experimental groups: a negative control group, an APOE treatment control group, and an example compound experimental group.

After the plated J774A.1 cells adhering to the plate, the culture medium was replaced. The culture medium of the APOE treatment control group was replaced with cell culture medium containing 30 µg/mL APOE peptide, the culture medium of the example compound experimental group was replaced with culture medium containing 30 µg/mL APOE peptide combined with the compounds, and the negative control group received cell culture medium. Then, all the groups were incubated overnight. 18 h later, the cell supernatants were collected for IL-1β detection.

### Experimental results

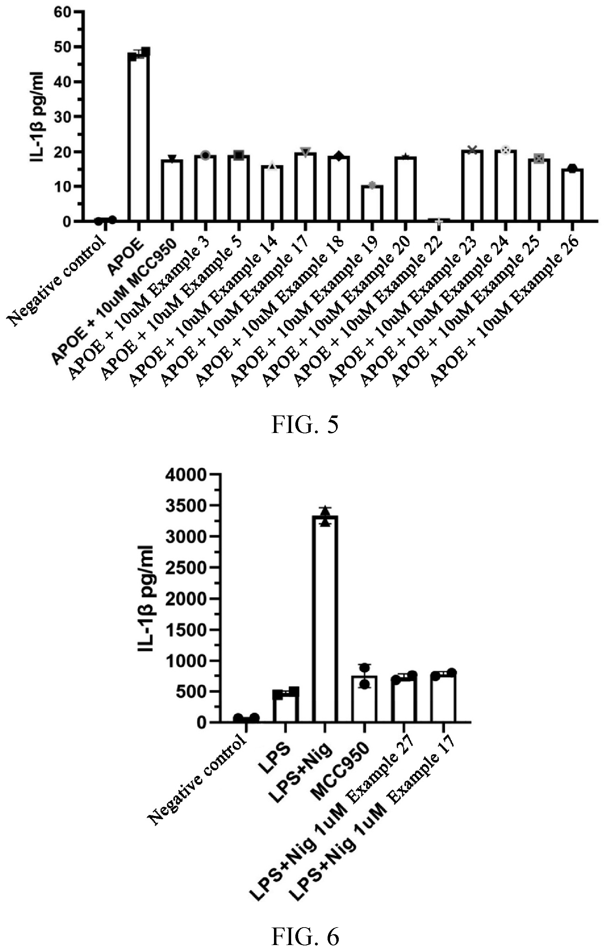

Activation of NLRP3 by APOE peptide will result in release of mature IL-1β, and the release can be inhibited by NLRP3 inhibitors. Therefore, the relative change in the amount of IL-1β release may directly reflect NLRP3 inhibitory effect. The results are shown in FIG. 5.

In this experimental example, effective inhibition refers to a reduction of 70% or more in the release amount, compared with the release amount of the positive control minus the negative control. The results show that Example 3, Example 5, Example 14, Example 17, Example 18, Example 19, Example 20, Example 22, Example 23, Example 24, Example 25, and Example 26 can effectively inhibit IL-1β release at the concentration of 10 µM (FIG. 5).

### Experimental Example 3: Inhibition curves of the inhibition of the compounds on canonical activation of NLRP3 in the murine cell line J774A.1

J774A.1 cells were plated in a 48-well plate at a density of 1.0×10⁶ cells/mL, with 300 µL per well. Subsequently, NLRP3 was activated by LPS+ATP or LPS+Nig. The cell culture medium comprises: DMEM (brand: Gibco, Cat. No.: C12430500) + 10% fetal bovine serum (brand: Gibco, Cat. No.: 10099-141) + 1% sodium pyruvate (brand: Gibco, Cat. No.: 11360-070) + 1% penicillin-streptomycin solution (brand: Solarbio, Cat. No.: P1400). The J774A.1 mouse monocyte-macrophage cell line was purchased from the Cell Resource Center of the Institute of Basic Medical Sciences of Chinese Academy of Medical Sciences (Resource No. 1101MOU-PUMC000222). ATP was purchased from Sigma, Cat. No.: L2654. Nig was purchased from Shanghai Yuanye Bio-Technology Co., Ltd., Cat. No.: S45490.

Experimental groups: a negative control group, an LPS-alone incubation control group, an LPS+ATP/Nig positive control group, and an example compound experimental group with different concentrations.

After the plated J774A.1 cells adhering to the plate, the culture medium was replaced. For all the groups except the negative control group, the culture medium was completely replaced with cell culture medium containing 1 µg/mL LPS. The negative control group received cell culture medium. Then, all the groups were incubated overnight. 18 h later, the culture medium was replaced again. The culture medium of the example compound experimental group was completely replaced with culture medium containing different concentrations of compounds; while the negative control group, the LPS-alone incubation control group, and the LPS+ATP/Nig positive control group received cell culture medium. 1 h later, the culture mediums of the LPS+ATP/Nig positive control group and the example compound experimental group with different concentrations were completely replaced with cell culture medium containing 5 mM ATP or 10 µM Nig. 1 h later, the cell supernatants were collected for IL-1β detection.

### Experimental results

Activation of NLRP3 by LPS+ATP will result in release of mature IL-1β, and the release can be inhibited by NLRP3 inhibitors. Therefore, the relative change in the amount of IL-1β release may directly reflect NLRP3 inhibitory effect. The inhibitory effect of the compounds varies with different concentrations, and based on this, an inhibition curve can be constructed, and the inhibitory effects of the compounds were assessed by IC₅₀. The results are shown in Tables 1-2.

The results show that IC₅₀ of Example 3 is 203.4 nM; IC₅₀ of Example 5 is 224.3 nM; IC₅₀ of Example 19 is 113.9 nM (Table 1); IC₅₀ of Example 1 is 64.6 nM; IC₅₀ of Example 17 is 194.3 nM; IC₅₀ of Example 22 is 127.4 nM; IC₅₀ of Example 26 is 81.61 nM; IC₅₀ of Example 27 is 79.6 nM; IC₅₀ of Example 30 is 144.2 nM; IC₅₀ of Example 31 is 203.2 nM; IC₅₀ of Example 32 is 389.6 nM; and IC₅₀ of MCC950 is 121.5 nM (Table 2).

**Table 1 (Experimental Example 3. Results of IC₅₀ determination in J774A.1 cell LPS+ATP activation models)**

| Example No. | IC₅₀ (nM) |
|---|---|
| Example 19 | 113.9 |
| Example 3 | 203.4 |
| Example 5 | 224.3 |

**Table 2 (Experimental Example 3. Results of IC₅₀ determination in J774A.1 cell LPS+Nig activation models)**

| Example No. | IC₅₀ (nM) |
|---|---|
| Example 1 | 64.6 |
| Example 17 | 194.3 |
| Example 22 | 127.4 |
| Example 26 | 81.61 |
| Example 27 | 79.6 |
| Example 30 | 144.2 |
| Example 31 | 203.2 |
| Example 32 | 389.6 |
| MCC950 | 121.5 |

### Experimental Example 4: The inhibition of the compounds on canonical activation of NLRP3 in the human cell line THP-1

THP-1 cells were plated in a 96-well plate at a density of 1.0×10⁶ cells/mL, with 100 µL per well. Subsequently, PMA-induced differentiation and cell resting were performed, followed by activation of NLRP3 by LPS+Nig. The cell culture medium comprises: 1640 culture medium (brand: Gibco, Cat. No.: C11875500BT) + 10% fetal bovine serum (brand: Gibco, Cat. No.: 11360-070) + 1% penicillin-streptomycin solution (brand: Solarbio, Cat. No.: P1400). THP-1 human acute monocytic leukemia cell line was purchased from the Cell Resource Center of the Institute of Basic Medical Sciences of Chinese Academy of Medical Sciences (Resource No. 1101HUM-PUMC000057). PMA was purchased from Sgima, Cat. No.: P1585. Nig was purchased from Shanghai Yuanye Bio-Technology Co., Ltd., Cat. No.: S45490.

Experimental groups: a negative control group, an LPS-alone incubation control group, an LPS+Nig positive control group, and a 1 µM example compound experimental group.

100 ng/mL PMA was added at the same time as THP-1 cells were plated. After incubation for 24 h, the cells were washed three times with PBS, and then the culture medium was changed and the cells were allowed to rest for 24 h. Then the culture medium was completely replaced with medium containing 1 µg/mL LPS. The cells were incubated overnight. On the next day, the wells to which LPS was added completely received culture medium, and then the wells were incubated with specified example compounds for 1 h. 5 µM Nig was then added directly for incubation for 45 min, and finally, the cell supernatants were collected for IL-1β detection.

### Experimental results

Activation of NLRP3 by LPS+Nig will result in release of mature IL-1β, and the release can be inhibited by NLRP3 inhibitors. Therefore, the relative change in the amount of IL-1β release may directly reflect NLRP3 inhibitory effect. The results are shown in FIGS. 6-7.

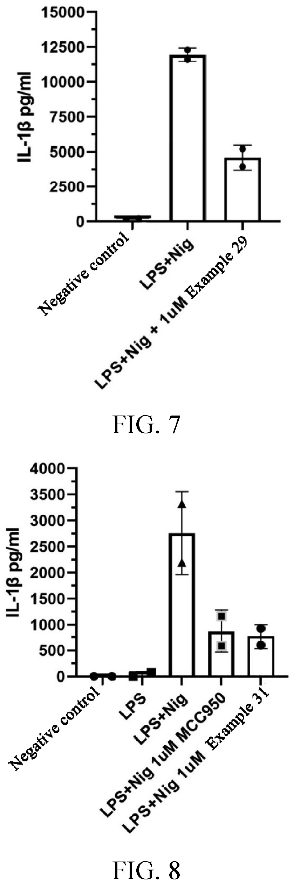

In this experimental example, effectively inhibiting IL-1β release means that the release amount in each group is reduced by 50% or more compared with that of the positive control. The results show that MCC950, Example 17 and Example 27 can effectively inhibit IL-1β release at the concentration of 1 µM (FIG. 6). Example 29, Example 31, and Example 32 can effectively inhibit IL-1β release at the concentration of 1 µM (FIGS. 7-9).

### Experimental Example 5: Inhibition curves of the inhibition of the compounds on canonical activation of NLRP3 in the human cell line THP-1

THP-1 cells were plated in a 96-well plate at a density of 1.0×10⁶ cells/mL, with 100 µL per well. Subsequently, PMA-induced differentiation and cell resting were performed, followed by activation of NLRP3 by LPS+Nig. The cell culture medium comprises: 1640 culture medium (brand: Gibco, Cat. No.: C11875500BT) + 10% fetal bovine serum (brand: Gibco, Cat. No.: 11360-070) + 1% penicillin-streptomycin solution (brand: Solarbio, Cat. No.: P1400). THP-1 human acute monocytic leukemia cell line was purchased from the Cell Resource Center of the Institute of Basic Medical Sciences of Chinese Academy of Medical Sciences (Resource No. 1101HUM-PUMC000057). PMA was purchased from Sgima, Cat. No.: P1585. Nig was purchased from Shanghai Yuanye Bio-Technology Co., Ltd., Cat. No.: S45490.

Experimental groups: a negative control group, an LPS-alone incubation control group, an LPS+Nig positive control group, and an example compound experimental group with different concentrations.

100 ng/mL PMA was added at the same time as THP-1 cells were plated. After incubation for 24 h, the cells were washed three times with PBS, and then the culture medium was changed and the cells were allowed to rest for 24 h. Then the culture medium was completely replaced with medium containing 1 µg/mL LPS. The cells were incubated overnight. On the next day, the wells to which LPS was added completely received culture medium, and then the wells were incubated with different concentrations of specified example compounds for 1 h. 5 µM Nig was then added directly for incubation for 45 min, and finally, the cell supernatants were collected for IL-1β detection.

### Experimental results

Activation of NLRP3 by LPS+Nig will result in release of mature IL-1β, and the release can be inhibited by NLRP3 inhibitors. Therefore, the relative change in the amount of IL-1β release may directly reflect NLRP3 inhibitory effect. The inhibitory effect of the compounds varies with different concentrations, and based on this, an inhibition curve can be constructed, and the inhibitory effects of the compounds were assessed by IC₅₀. The results are shown in Tables 1-2.

The results show that IC₅₀ of Example 19 is 278.0 nM; IC₅₀ of Example 30 is 36.7 nM; and IC₅₀ of MCC950 is 315.0 nM (Table 3).

**Table 3 (Experimental Example 3. Results of IC₅₀ determination in THP-1 cell LPS+Nig activation models)**

| Example No. | IC₅₀ (nM) |
|---|---|
| Example 19 | 278.0 |
| Example 30 | 36.7 |
| MCC950 | 315.0 |

### Experimental Example 6: Plotting of inhibition curves of the inhibition of the example compounds on canonical activation of NLRP3 in healthy human whole blood

10 mL of human whole blood was collected intravenously aseptically, and plated into a 96-well plate, with 198 µL per well. Then, 2 µL of 100× gradient dilutions of the example compounds at specified concentrations were added, immediately followed by the addition of 10 µL of 21 µg/mL LPS gradient dilution. 3 h later, nigericin with a working concentration of 5 µM or 1 mM ATP was added. After stimulation for 1 h, the whole blood was collected and centrifuged to collect the supernatant. IL-1β in the sample supernatant was detected by ELISA method. Heparin sodium injection solution was purchased from Changzhou Qianhong Biopharma Co.,Ltd.; LPS was purchased from Sigma, Cat. No.: L2654; ATP was purchased from Sigma, Cat. No.: L2654; nigericin (Nig) was purchased from Shanghai Yuanye Bio-Technology Co., Ltd., Cat. No.:
S45490; PBS (1×) PH 7.4 was purchased from EallBio, Cat. No.: 03.15018C; and human IL-1β ELISA detection kit was purchased from Thermo Invitrogen, Cat. No.: 88-7261-88.

Experimental groups: a negative control group, an LPS-alone incubation control group, an LPS+Nig/ATP positive control group, and a 1 µM example compound experimental group.

10 mL of human whole blood was collected intravenously aseptically. The anticoagulant heparin sodium was added preemptively into a 50 mL centrifuge tube. The collected whole blood was directly plated in a 96-well plate, with 198 µL per well. Then, 2 µL of 100× gradient dilutions of the example compounds at specified concentrations were added, immediately followed by the addition of 10 µL of 21 µg/mL LPS gradient dilution. 3 h later, Nig with a working concentration of 5 µM/1 mM ATP was added. After stimulation for 1 h, the whole blood was collected and centrifuged to collect the supernatant. IL-1β in the sample supernatant was detected by ELISA method.

### Experimental results

Canonical activation of NLRP3 will result in release of mature IL-1β, and the release can be inhibited by NLRP3 inhibitors. Therefore, the relative change in the amount of IL-1β release may directly reflect NLRP3 inhibitory effect. The inhibitory effect of the compounds varies with different concentrations, and based on this, an inhibition curve can be constructed, and the inhibitory effect of the compounds were assessed by IC₅₀. The results are shown in Table 4.

The results show that in the LPS+1 mM ATP model system, IC₅₀ of MCC950 is 151.3 µM; and IC₅₀ of Example 27 is 55.8 µM (Table 4). In the LPS+5 µM Nig model system, IC₅₀ of MCC950 is 3.22 µM; and IC₅₀ of Example 19 is 1.27 µM (Table 5).

**Table 4 (Experimental Example 6. Results of IC₅₀ determination in human whole blood LPS+ATP canonical activation models)**

| Example No. | IC₅₀ (µM) |
|---|---|
| Example 27 | 55.8 |
| MCC950 | 151.3 |

**Table 5 (Experimental Example 6. Results of IC₅₀ determination in human whole blood LPS+Nig canonical activation models)**

| Example No. | IC₅₀ (µM) |
|---|---|
| Example 19 | 1.27 |
| MCC950 | 3.22 |

### Experimental Example 7: The inhibition of the example compounds on NLRP3 activation in LPS-induced sepsis models

Intraperitoneal injection of 20 mg/kg LPS can induce a septic state in C57BL/6 mice and cause a significant increase in serum IL-1β after injection. Example compounds were intraperitoneally injected preemptively to study the effects on IL-1β increase and mouse survival rate. LPS was purchased from Sigma, Cat. No. L2630; and C57BL/6J male mice aged 6-8 weeks were purchased from National Institutes for Food and Drug Control.

Experimental groups: a sham group, a model group, and an example compound dosing group.

30 mg/kg of example compounds were injected intraperitoneally. 1 h later, 20 mg/kg LPS was injected intraperitoneally into the model group and the example compound dosing group for modeling, and the control solvent was injected into the sham group. Serum samples were collected from a batch of mice at 2.5 h and 4 h after modeling, and the levels of IL-1β were analyzed. For another batch of mice, the survival rate of the mice was calculated after modeling.

### Experimental results

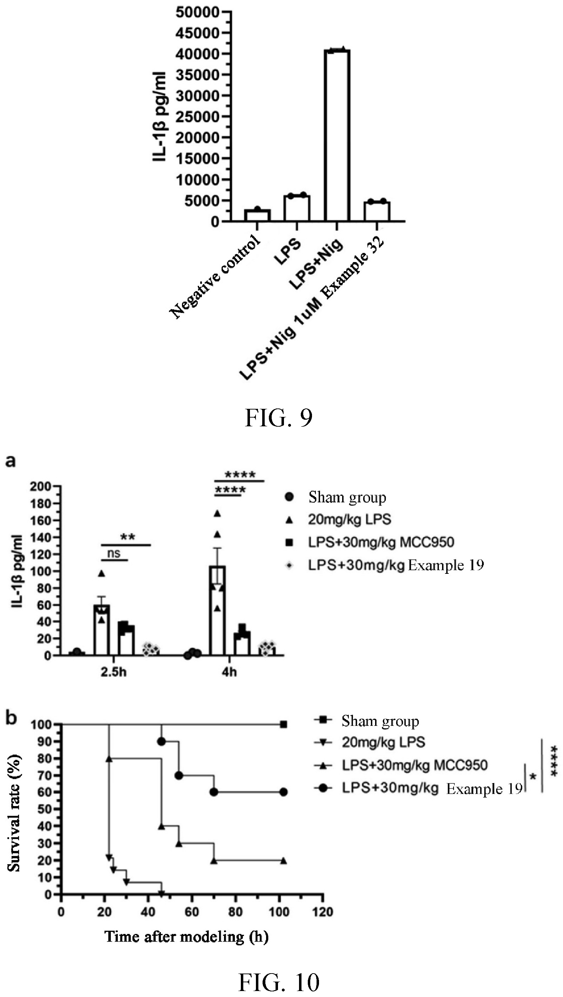

The detection results of IL-1β in serum at 2.5 h and 4 h after modeling are shown in FIG. 10(a) (5 mice in each group), and the statistical results of mouse survival rate are shown in FIG. 10(b) (10 mice in each group). The results of statistical analysis show that in terms of IL-1β level, compared with the model group, MCC950 showed no significant difference at 2.5 h (ns, P>0.05), and showed significant differences at 4 h (****, P<0.0001); and Example 19 showed significant differences at both 2.5 h and 4 h (**, P<0.01, 2.5h; ****, P<0.0001, 4h). In terms of survival rate, Example 19 significantly improved the mouse survival rate compared with the model group (P<0.0001, ****), and Example 19 significantly further improved the survival rate compared with MCC950 (P<0.05, *).

### Experimental Example 8: The inhibition of the example compounds on NLRP3 activation in MSU-induced mouse peritonitis models

Intraperitoneal injection of LPS and monosodium urate crystals (MSU) in mice can induce the activation of NLRP3 and release of large amounts of IL-1β. IL-1β release can be inhibited by preemptive gavage of NLRP3 inhibitors. LPS was purchased from Sigma, Cat. No. L2654. MSU was purchased from Bide Pharmatech Co., Ltd., Cat. No. BD367417. C57BL/6J female mice aged 8 weeks were purchased from National Institutes for Food and Drug Control.

The mice were administered the example compounds via oral gavage. 2 h later, they received an intraperitoneal injection of 1.25 µg LPS, and an intraperitoneal injection of MSU after another 2 h. Ascites was collected 30 min later, and then IL-1β protein detection was performed on the ascites.

Experimental groups: a model group, and an example compound dosing group.

### Experimental results

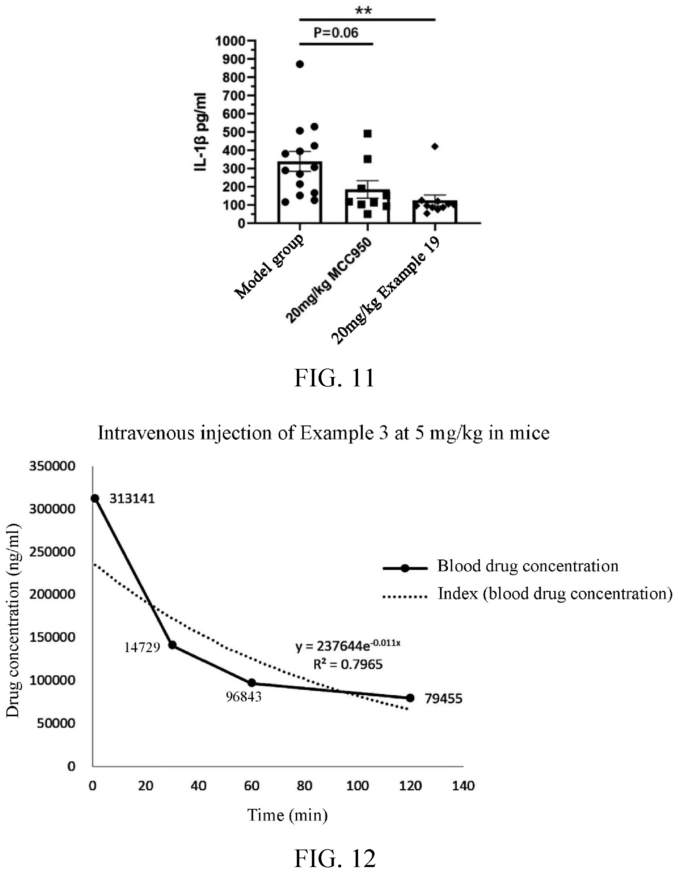

IL-1β levels in ascites are shown in FIG. 11 (9-14 mice per group). The results of statistical analysis show that compared with the model group, Example 19 significantly inhibited the level of IL-1β (**, P<0.01), and MCC950 showed only a trend towards inhibition (ns, P=0.06).

### Experimental Example 9: Analysis of Drug Metabolic Properties of Compounds

C57 mice were administered intravenously, at a dosage concentration of 5 mg/kg in each group. Retro-orbital blood collection was performed on the mice at 1 min, 30 min, 1 h, and 2 h post-dosing. The collected blood was centrifuged at 3000 rpm for 10 min at 4 °C to obtain plasma. Acetonitrile was added at a 1:3 volume ratio of plasma: acetonitrile to precipitate proteins. After centrifugation, the supernatant was collected and filtered. The concentration of the compound was detected using LC-MS (Agilent 1260-G6125, SIM mode). In addition, 2 h after drug injection, the mice were subjected to cardiac perfusion and then their brains were harvested. Following tissue homogenization and centrifugation, the supernatant was collected. Acetonitrile was added at a 1:3 volume ratio of supernatant: acetonitrile to precipitate proteins. After centrifugation, the supernatant was collected and filtered. The concentration of the compound was detected using LC-MS (Agilent 1260-G6125, SIM mode).

Experimental groups: a vehicle control group, and a dosing group.

### Experimental results

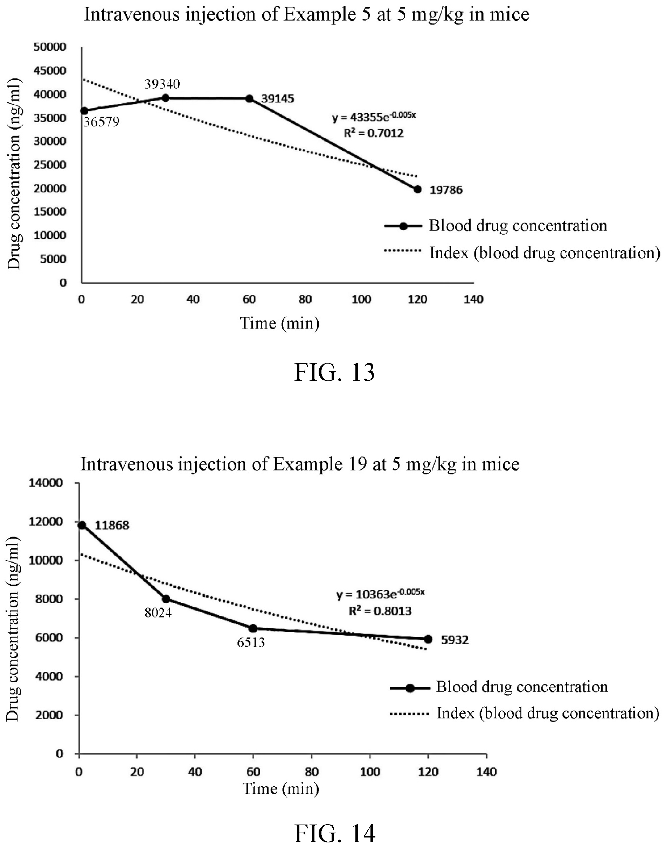

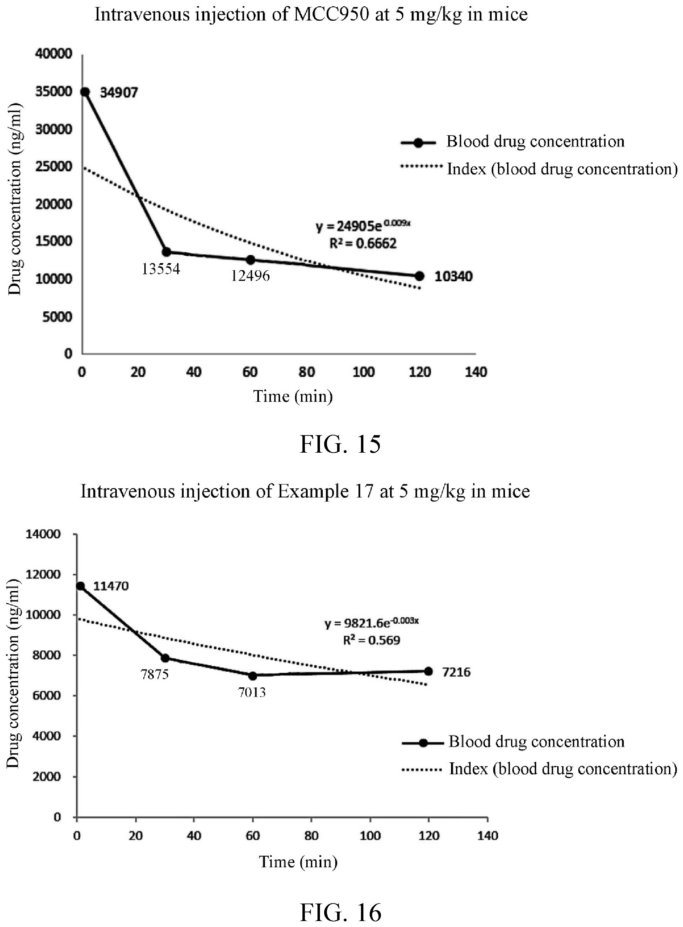

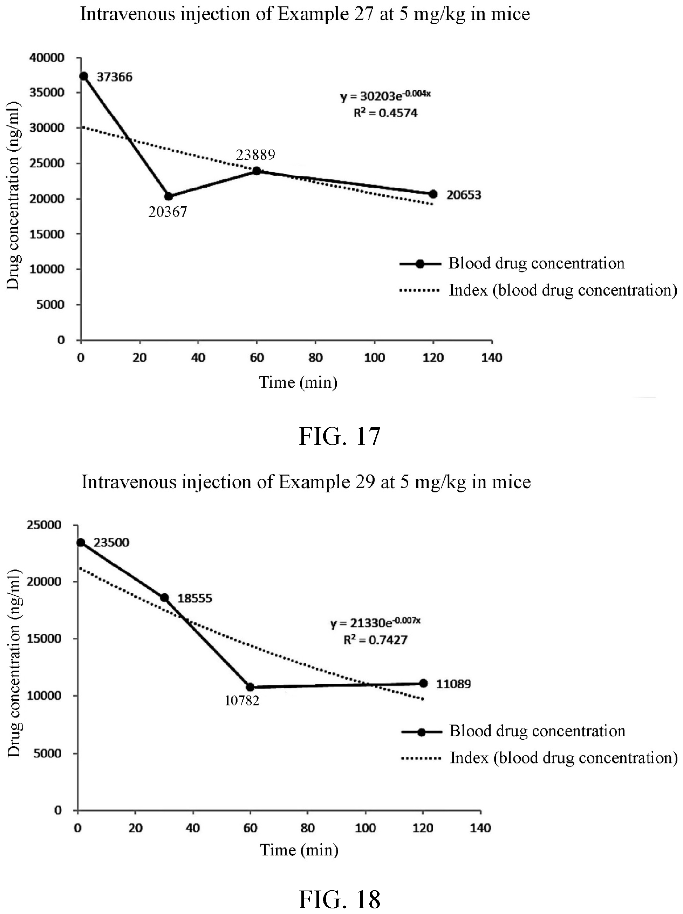

The results of plasma pharmacokinetic analysis of Example 3, Example 5, Example 19, and the control drug MCC950 are shown in FIGS. 12-18.

The results show that Example 3 has a half-life of 63 min, an area under the curve (AUC) from 1 to 120 min of 257227 ng·h/mL, and a brain drug concentration of 35492 ng/mL at 120 min.

Example 5 has a half-life of 138 min, an AUC from 1 to 120 min of 67433 ng·h/mL, and a brain drug concentration of 3869 ng/mL at 120 min.

Example 19 has a half-life of 139 minutes, an AUC from 1 to 120 min of 14664 ng·h/mL, and a brain drug concentration of 242 ng/mL at 120 min.

The control drug MCC950 tested in parallel has a half-life of 77 min, an AUC from 1 to 120 min of 29642 ng·h/mL, and a brain drug concentration of 0 ng/mL at 120 min (below the detection limit).

Example 17 has a half-life of 231 min, an AUC from 1 to 120 min of 15511 ng·h/mL, and a brain drug concentration of 184 ng/mL at 120 min.

Example 27 has a half-life of 173 min, an AUC from 1 to 120 min of 47287 ng·h/mL, and a brain drug concentration of 96 ng/mL at 120 min.

Example 29 has a half-life of 99 minutes, an AUC from 1 to 120 min of 14861 ng·h/mL, and a brain drug concentration of 197 ng/mL at 120 min.

### Experimental Example 10: Analysis of Drug Metabolic Properties of Compounds in rats

SD rats were administered intravenously, at a dosage concentration of 2.5 mg/kg in each group. Blood was collected at the time points of 1 min, 30 min, 1 h, 2 h, 4 h, 6 h, and 24 h. The collected blood was centrifuged at 3000 rpm for 10 min at 4 °C to obtain plasma. Acetonitrile was added at a 1:3 volume ratio of plasma: acetonitrile to precipitate proteins. After centrifugation, the supernatant was collected and filtered. The concentration of the compound was detected using LC-MS (Agilent 1260-G6125, SIM mode).

Experimental groups: a vehicle control group, and a dosing group.

### Experimental results

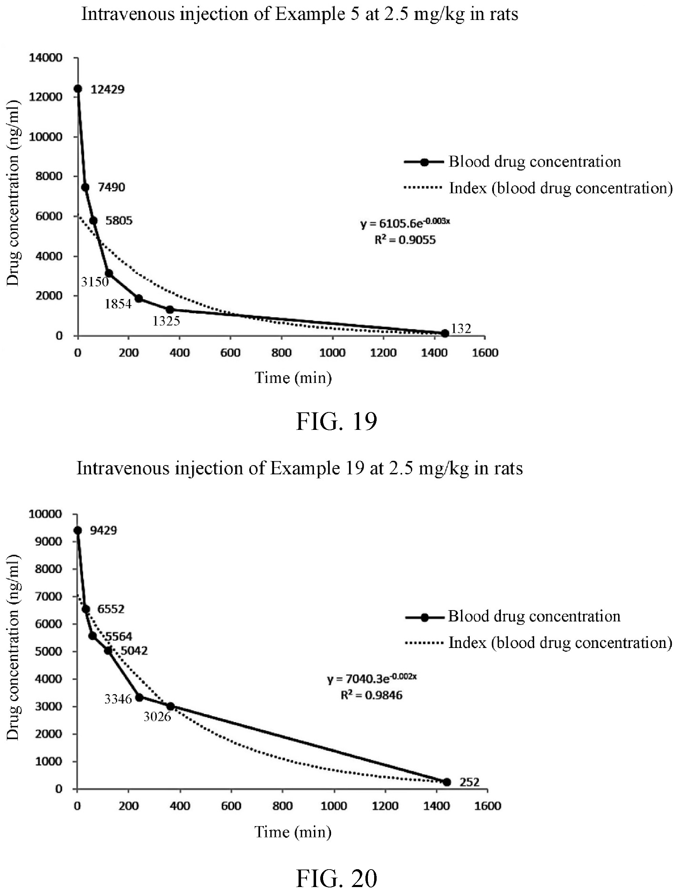

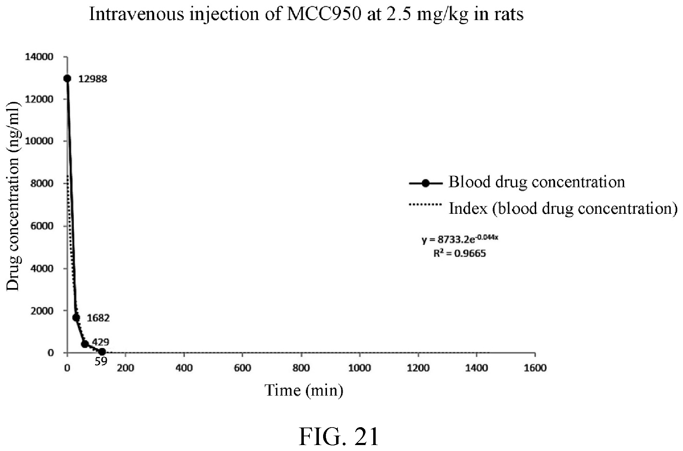

The results of plasma pharmacokinetic analysis of Example 5, Example 19, and the control drug MCC950 are shown in FIGS. 19-21.

The results show that Example 5 has a half-life of 116 min, and an AUC from 1 to 1440 min of 33916 ng·h/mL.

Example 19 has a half-life of 231 min, and an AUC from 1 to 1440 min of 56453 ng·h/mL.

The control drug MCC950 tested in parallel has a half-life of 16 min, and an AUC from 1 to 1440 min of 4376 ng·h/mL.

### Experimental Example 11: Evaluation of cardiotoxicity of example compounds - determination of hERG inhibition

### 5.1 Cell culture

In this study, the HEK-293 cell line stably expressing hERG potassium channel was used. hERG potassium channel cells were purchased from Creacell (Cat. No.: A-0320). The cells were cultured by the following methods:
The HEK-293 cell line stably expressing hERG potassium channel was cultured in DMEM medium (hereinafter briefly referred to as complete medium) containing 10% fetal bovine serum (brand: Gibco, Cat. No.: 10099-141) and 0.8 mg/mL G418 (geneticin, ST081, Biyuntian). The culture temperature was 37 °C, and the carbon dioxide concentration was 5%.

Cell passage: The old medium was removed, and the cells were washed once with PBS. Then 1 mL TrypLE^{™} Express solution (Gibco 12604-021) was added to incubate the cells at 37 °C for about 1 min. Once the cells were detached from the bottom of the dish, approximately 5 mL of complete medium pre-warmed at 37 °C was added. The cell suspension was gently triturated with a pipette to dissociate aggregated cells. The cell suspension was transferred to a sterile centrifuge tube and centrifuged at 1000 rpm for 5 min to collect the cells. For expansion or maintenance, the cells were seeded into 10 cm cell culture dishes at a density of 6×10⁵ cells per cell culture dish (final volume: 5 mL).

To maintain the electrophysiological activity of the cells, the cell density must not exceed 80%.

Prior to patch clamp detection, the cells were dissociated using TrypLE^{™} Express (Gibco 12604-021). Medium was added to terminate digestion, followed by centrifugation. The cells were then resuspended and counted. The cell density was adjusted to 2-3×10⁶ cells/mL. Subsequently, the cells were gently mixed for 15-20 min on a balance shaker at room temperature, and then loaded for detection.

### 5.2 Electrophysiological recordings

### 5.2.1 Fluids used for recordings

### Extracellular fluid: K-007-1

140 mM NaCl, 3.5 mM Kcl, 1 mM MgCl₂•6H₂O, 2 mM CaCl₂•2H₂O, 10 mM D-Glucose, 10 mM HEPES, 1.25 mM NaH₂PO₄•2H₂O, NaOH adjusted pH=7.4.

### Intracellular fluid: K-002-2

20 mM KCl, 115 mM K-Aspartic, 1 mM MgCl₂•6H₂O, 5 mM EGTA, 10 mM HEPES, 2 mM Na₂-ATP, KOH adjusted pH=7.2.

The extracellular fluid was stored for one week. The intracellular fluid was formulated and then aliquoted into 10 mL per tube and stored in a refrigerator at -20 °C. Freshly thawed intracellular fluid was used for experiments every day. All intracellular fluid was used up within one week. The old intracellular fluid was discarded after one week, and intracellular fluid was reformulated.

### 5.2.2 Patch clamp detection

In this study, the automated patch clamp QPatch 48X (Sophion) system was used for electrophysiological detection.

First, the prepared cells were placed on the centrifuge of the Qpatch workstation. The cells were washed with multiple centrifugation/suspension cycles, and the cell culture medium was replaced with extracellular fluid. One MTP-96 plate was retrieved and placed at the MTP source position. The QPlate chip was retrieved and placed at the QPlate source position. The robotic arm scanned the barcodes of MTP-96 plate and QPlate chip, and transferred them to the measurement station. The intracellular fluid and extracellular fluid were aspirated from the reservoir and added to the intracellular fluid pool and the pool of cell and compound of the QPlate chip, respectively. At the measurement station, all measurement sites on the QPlate experienced initial quality control. The quality control process involved aspirating the cell suspension from the cell container of the centrifuge, positioning the cells onto the chip wells via a pressure controller, and establishing high-resistance seal to form a whole-cell recording mode. Once a stable control current baseline was obtained, the test substances can be sequentially aspirated from the test substance MTP-96 plate in order of concentration and applied to the cells.

The voltage stimulation protocol for whole-cell patch-clamp recording of hERG current was as follows: after the whole-cell seal was formed, the cell membrane voltage was clamped at -80 mV. The clamped voltage was depolarized from -80 mV to -50 mV and maintained for 0.5 s (as leak current detection), then stepped to 30 mV and maintained for 2.5 s, and then rapidly returned to -50 mV and maintained for 4 s, whereby tail current of the hERG channel can be elicited. Data was collected repeatedly every 10 s to observe the effect of the drug on hERG tail current. Experimental data was collected by the QPatch screening workstation and stored in the database server.

For each drug concentration, a two-dose regimen was set for a duration of at least 5 min. For each cell, the current detected in the compound-free extracellular fluid served as self-controls, and two cells were detected in independent replicates. All electrophysiological experiments were conducted at room temperature.

### 5.3 Standards for data quality

The following standards are used to determine whether the data is acceptable:
(1) $seal resistance > 1 GΩ$
(2) $initial access resistance < 15 MΩ$
(3) $final access resistance < 15 MΩ$
(4) $initial tail current peak > 200 pA$
(5) initial tail current peak being greater than activation current peak
(6) no significant spontaneous decay of tail current
(7) no significant leak current at a membrane potential of -80 mV (absolute value no significant maximum leak current peak)

### 5.4 Data Analysis

The second of the two dosages was used for data analysis. For each drug concentration, the average of the last three data points prior to the next drug concentration was taken to represent the current value for the drug concentration. The current value representing each drug concentration was normalized against the reference current value used as a blank control, and then the inhibition rate corresponding to each drug concentration was calculated.

### Experimental results

Multiple examples were tested for hERG inhibition rate at 1 µM and 10 µM respectively using the methods described above, and compared with the antiarrhythmic agent Dofetilide whose IC₅₀ was determined as 0.016 µM. The results of the inhibition rate of each example are shown in the table below.

| Compounds | Structural formula | hERG inhibition rate | |
|---|---|---|---|
| | | 1 µM | 10 µM |
| Example 3 | 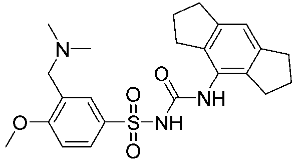 | 0.45 | 1.56 |
| Example 5 | 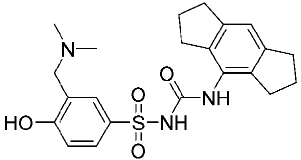 | 0.28 | -0.61 |
| Example 19 | 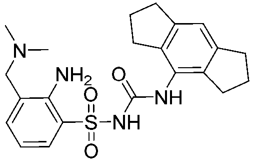 | 0.3 | 2.96 |

The above results indicate that the typical examples in the study have good safety regarding hERG inhibition-related toxicity.

Although the embodiments disclosed in the present application are as above, the contents described are only the embodiments adopted for the convenience of understanding the present application, and are not used to limit the present application. Without departing from the spirit and scope disclosed in the present application, a person skilled in the art to which the present application pertains can make any modification and change to the implementation form and details, but the protection scope of the present application shall still be subjected to the scope defined in the appended claims.

## Claims

1. A sulfonylurea derivative or a pharmaceutically acceptable salt thereof, wherein the sulfonylurea derivative has a structure shown in Formula I, 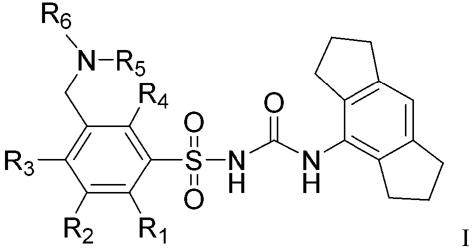 wherein:
R₁ is selected from H, halogen, nitro, amino, hydroxyl, C₁₋₆ alkoxy, C₂₋₆ alkenyl, C₂₋₆ alkynyl, C₁₋₆ alkylamino, aryl, aryloxy, heteroaryl, carboxyl, and heteroalkyl;
R₂ is selected from H, halogen, nitro, amino, hydroxyl, C₁₋₆ alkoxy, C₂₋₆ alkenyl, C₂₋₆ alkynyl, C₂₋₆ alkylamino, aryl, aryloxy, heteroaryl, carboxyl, and heteroalkyl;
R₃ is selected from H, halogen, nitro, amino, hydroxyl, C₁₋₆ alkoxy, halo C₁₋₆ alkoxy, C₂₋₆ alkenyl, C₂₋₆ alkynyl, C₁₋₆ alkylamino, aryl, aryloxy, aryl C₁₋₆ alkoxy, heteroaryl, carboxyl, and heteroalkyl;
R₄ is selected from H, halogen, nitro, amino, hydroxyl, C₁₋₆ alkoxy, C₁₋₆ alkyl, C₂₋₆ alkenyl, C₂₋₆ alkynyl, C₂₋₆ alkylamino, aryl, aryloxy, heteroaryl, carboxyl, and heteroalkyl;
wherein the four substituents R₁, R₂, R₃, and R₄ are not simultaneously H;
R₅ is C₁₋₆ alkyl;
R₆ is C₁₋₆ alkyl;
when R₄ is C₁₋₆ alkyl, R₆ together with R₄ forms a heterocyclic hydrocarbon group;
or when R₃ is C₁₋₆ alkyl, R₆ together with R₃ forms a heterocyclic hydrocarbon group;
or when R₄ is C₁₋₆ alkoxy, methylene linked to an N atom, together with R₄, forms a heterocyclic hydrocarbon group.

2. The sulfonylurea derivative or a pharmaceutically acceptable salt thereof according to claim 1, wherein the halogen is F, Cl, Br, or I, preferably F or Cl.

3. The sulfonylurea derivative or a pharmaceutically acceptable salt thereof according to claim 1, wherein said R₁ is selected from H, halogen, nitro, or amino, preferably H, F, Cl, -NO₂, or -NH₂.

4. The sulfonylurea derivative or a pharmaceutically acceptable salt thereof according to claim 1, wherein said R₂ is H or halogen, preferably H or F.

5. The sulfonylurea derivative or a pharmaceutically acceptable salt thereof according to claim 1, wherein said R₃ is selected from H, C₁₋₃ alkoxy, halo C₁₋₃ alkoxy, aryloxy, aryl C₁₋₃ alkoxy, hydroxyl, halogen, or amino, preferably H, -OCH₃, -OBn, F, Cl, -NH₂, -OCF₃, -OCH₂CH₃, -OCH(CH₃)₂, or -OH.

6. The sulfonylurea derivative or a pharmaceutically acceptable salt thereof according to claim 1, wherein said R₄ is selected from H, halogen, C₁₋₃ alkyl, C₁₋₃ alkoxy, amino, hydroxyl, or nitro, preferably H, -NO₂, -OCH₂CH₃, -NH₂, F, Cl, -CH₃, or -OH; when R₄ is - OCH₂CH₃, methylene linked to an N atom, together with R₄, forms a six-membered heterocyclic hydrocarbon group.

7. The sulfonylurea derivative or a pharmaceutically acceptable salt thereof according to claim 1, wherein said R₅ and R₆ are each independently methyl or ethyl, preferably said R₅ and R₆ are simultaneously methyl or simultaneously ethyl; R₆ together with R₄ forms a six-membered heterocyclic hydrocarbon group; or R₆ together with R₃ forms a six-membered heterocyclic hydrocarbon group.

8. The sulfonylurea derivative or a pharmaceutically acceptable salt thereof according to claim 1, wherein the sulfonylurea derivative is selected from:

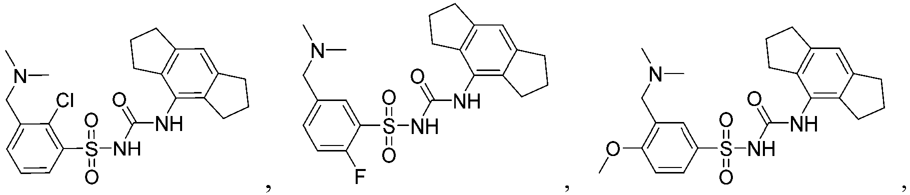

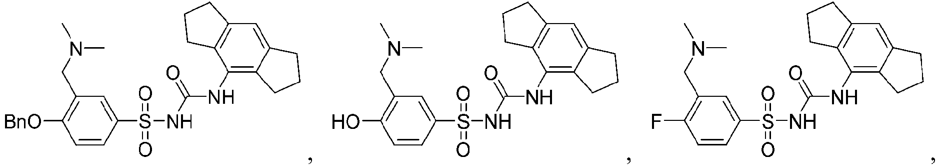

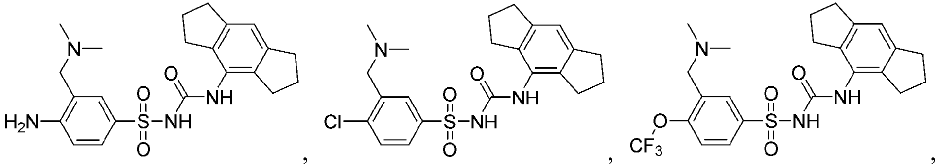

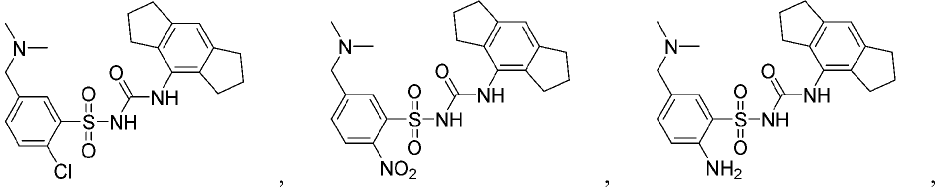

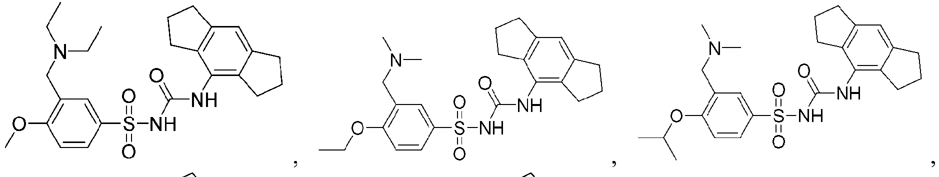

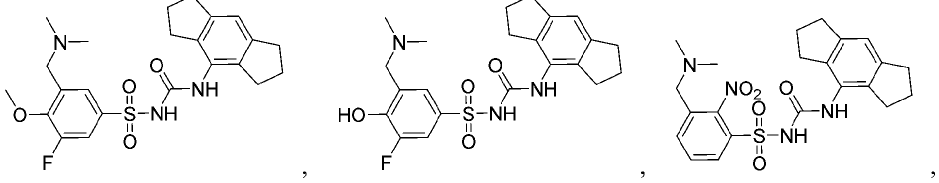

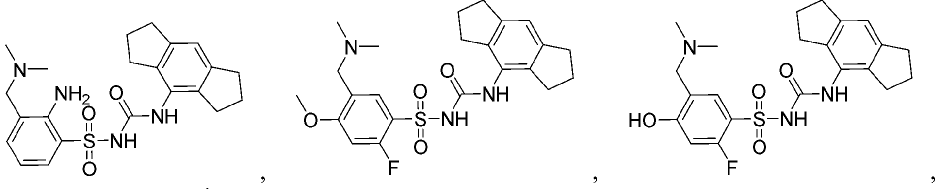

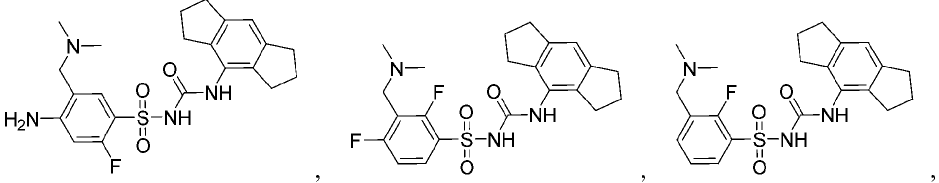

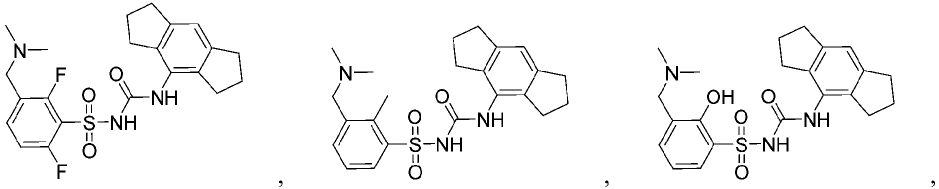

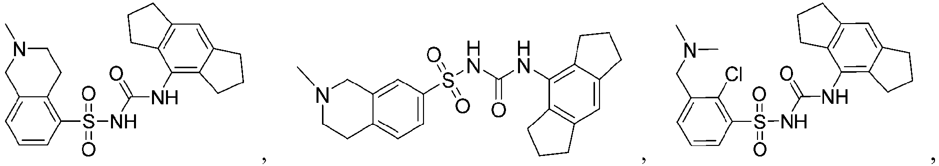

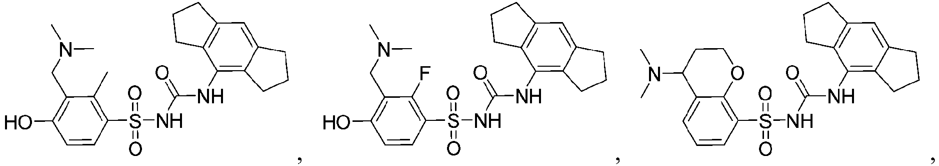

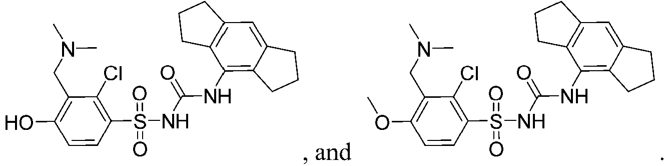

9. The sulfonylurea derivative or a pharmaceutically acceptable salt thereof according to claim 8, wherein the sulfonylurea derivative is selected from: 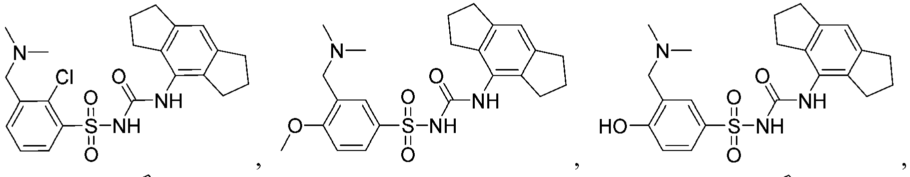 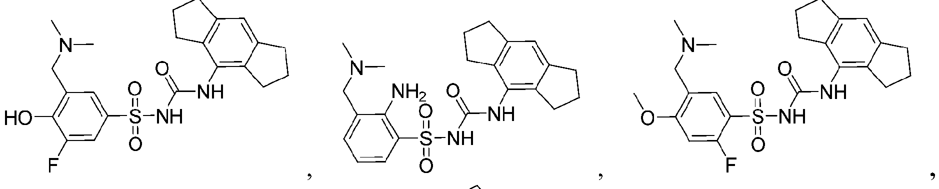 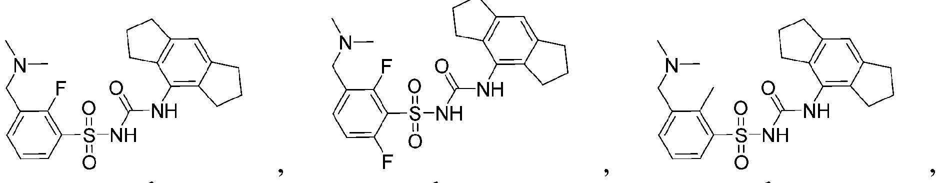 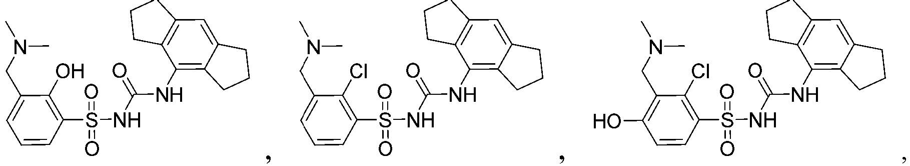 and

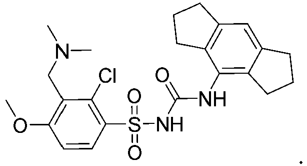

10. The sulfonylurea derivative or a pharmaceutically acceptable salt thereof according to any one of claims 1-9 for use as:
(i) an NLRP3 inflammasome inhibitor; and/or
(ii) a modulator for one or more of IL-1β, IL-17, IL-18, IL-1α, IL-37, IL-33, and Th17 cells.

11. A composition comprising the sulfonylurea derivative or the pharmaceutically acceptable salt thereof according to any one of claims 1-9, and a pharmaceutically acceptable excipient.

12. The sulfonylurea derivative or the pharmaceutically acceptable salt thereof according to any one of claims 1-10 or the composition according to claim 11 for use in the treatment or prevention of a disease, disorder, or condition, wherein the disease, disorder, or condition is:
(i) an immune system disease, disorder, or condition;
(ii) an inflammatory disease, disorder, or condition, or an autoimmune disease, disorder, or condition;
(iii) a skin disease, disorder, or condition;
(iv) a cardiovascular system disease, disorder, or condition;
(v) tumor;
(vi) a renal system disease, disorder, or condition;
(vii) a gastrointestinal disease, disorder, or condition;
(viii) a respiratory system disease, disorder, or condition;
(ix) an endocrine system disease, disorder, or condition;
(x) a central nervous system (CNS) disease, disorder, or condition; and/or
(xi) local or systemic infection.

13. Use of the sulfonylurea derivative or the pharmaceutically acceptable salt thereof according to any one of claims 1-10 or the composition according to claim 11 in the preparation of a medicament for treating or preventing a disease, disorder, or condition, wherein the disease, disorder, or condition is:
(i) an immune system disease, disorder, or condition;
(ii) an inflammatory disease, disorder, or condition, or an autoimmune disease, disorder, or condition;
(iii) a skin disease, disorder, or condition;
(iv) a cardiovascular system disease, disorder, or condition;
(v) tumor;
(vi) a renal system disease, disorder, or condition;
(vii) a gastrointestinal disease, disorder, or condition;
(viii) a respiratory system disease, disorder, or condition;
(ix) an endocrine system disease, disorder, or condition;
(x) a central nervous system (CNS) disease, disorder, or condition; and/or
(xi) local or systemic infection.

14. The sulfonylurea derivative or the pharmaceutically acceptable salt thereof or the composition for use according to claim 12 or the use according to claim 13, wherein the disease, disorder, or condition is selected from the group consisting of: constitutive inflammation, comprising cryopyrin associated periodic syndrome (CAPS), Muckle-Wells syndrome (MWS), familial cold autoinflammatory syndrome (FCAS) and neonatal-onset multisystem inflammatory disease (NOMID), autoinflammatory disease, familial Mediterranean fever (FMF), TNF receptor-associated periodic syndrome (TRAPS), mevalonate kinase deficiency (MKD), hyperimmunoglobulin D with periodic fever syndrome (HIDS), deficiency of interleukin-1-receptor antagonist (DIRA), Majeed syndrome, pyogenic arthritis, pyoderma gangrenosum and acne syndrome (PAPA), haploinsufficiency of A20 (HA20), pediatric granulomatous arthritis (PGA), PLCG2-associated antibody deficiency and immune dysregulation (PLAID), autoinflammation and PLCG2-associated antibody deficiency and immune dysregulation (APLAID), and sideroblastic anemia with B-cell immunodeficiency, periodic fevers, and developmental delay (SIFD); autoimmune diseases, comprising multiple sclerosis (MS), type 1 diabetes, psoriasis, rheumatoid arthritis, Behçet's disease, Sjögren's syndrome, and Schnitzler syndrome; macrophage activation syndrome; Blau syndrome; respiratory system diseases, comprising chronic obstructive pulmonary disease (COPD), asthma such as allergic asthma and steroid-resistant asthma, asbestosis, silicosis, and cystic fibrosis; dermatitis, comprising contact dermatitis; central nervous system diseases, comprising Parkinson's disease, Alzheimer's disease, motor neuron disease, Huntington's disease, cerebral malaria and pneumococcal meningitis-induced brain injuries; metabolic diseases, comprising type 2 diabetes, atherosclerosis, obesity, gout, pseudogout; eye diseases, comprising those diseases of ocular epithelium, age-related macular degeneration (AMD), uveitis, corneal infections, and dry eye syndrome; kidney diseases, comprising chronic kidney disease, oxalate nephropathy, nephrocalcinosis, and diabetic kidney disease; liver diseases, comprising nonalcoholic steatohepatitis (NASH) and alcoholic liver disease; skin inflammatory responses, comprising contact hypersensitivity and sunburn; joint inflammatory responses, comprising osteoarthritis, systemic juvenile idiopathic arthritis, adult-onset Still's disease, and relapsing polychondritis; viral infections, comprising alphavirus (Chikungunya virus, Ross River virus) and flavivirus (dengue virus, Zika virus), influenza virus, and HIV; hidradenitis suppurativa (HS) and other skin diseases causing cysts; cancers, comprising lung cancer metastasis, pancreatic cancer, gastric cancer, myelodysplastic syndromes, and leukemia; polymyositis; stroke, comprising ischemic stroke; myocardial infarction, comprising recurrent myocardial infarction; congestive heart failure; embolism; cardiovascular diseases; graft-versus-host disease; hypertension; colitis; helminth infection; bacterial infection; sepsis; septic shock; abdominal aortic aneurysm; wound healing; depression, psychological stress; ischemia-reperfusion injury; and any disease in which an individual has been determined to carry a germline or somatic non-silent mutation in NLRP3.

15. The sulfonylurea derivative or the pharmaceutically acceptable salt thereof or the composition for use according to claim 12 or the use according to claim 13, wherein the disease, disorder, or condition is:
(i) an autoinflammatory disease such as cryopyrin associated periodic syndrome (CAPS), Muckle-Wells syndrome (MWS), familial cold autoinflammatory syndrome (FCAS), familial Mediterranean fever (FMF), neonatal-onset multisystem inflammatory disease (NOMID), tumor necrosis factor (TNF) receptor-associated periodic syndrome (TRAPS), hyperimmunoglobulin D with periodic fever syndrome (HIDS), deficiency of interleukin-1-receptor antagonist (DIRA), Majeed syndrome, or pyogenic arthritis, pyoderma gangrenosum and acne (PAPA);
(ii) Parkinson's disease or Huntington's disease;
(iii) gout or juvenile idiopathic arthritis;
(iv) nonalcoholic steatohepatitis (NASH);
(v) oxalate nephropathy or nephrocalcinosis;
(vi) uveitis;
(vii) hidradenitis suppurativa (HS);
(viii) myelodysplastic syndrome, macrophage activation syndrome, Schnitzler syndrome, adult-onset Still's disease, or Behçet's disease; or
(ix) sepsis or septic shock.

16. A method for treating or preventing a disease, disorder, or condition, comprising administering the sulfonylurea derivative or the pharmaceutically acceptable salt thereof according to any one of claims 1-10 or the composition according to claim 11 to a subject in need thereof, wherein the disease, disorder, or condition is:
(i) an immune system disease, disorder, or condition;
(ii) an inflammatory disease, disorder, or condition, or an autoimmune disease, disorder, or condition;
(iii) a skin disease, disorder, or condition;
(iv) a cardiovascular system disease, disorder, or condition;
(v) tumor;
(vi) a renal system disease, disorder, or condition;
(vii) a gastrointestinal disease, disorder, or condition;
(viii) a respiratory system disease, disorder, or condition;
(ix) an endocrine system disease, disorder, or condition;
(x) a central nervous system (CNS) disease, disorder, or condition; and/or
(xi) local or systemic infection.

17. The method according to claim 16, wherein the disease, disorder, or condition is selected from the group consisting of: constitutive inflammation, comprising cryopyrin associated periodic syndrome (CAPS), Muckle-Wells syndrome (MWS), familial cold autoinflammatory syndrome (FCAS) and neonatal-onset multisystem inflammatory disease (NOMID), autoinflammatory disease, familial Mediterranean fever (FMF), TNF receptor-associated periodic syndrome (TRAPS), mevalonate kinase deficiency (MKD), hyperimmunoglobulin D with periodic fever syndrome (HIDS), deficiency of interleukin-1-receptor antagonist (DIRA), Majeed syndrome, pyogenic arthritis, pyoderma gangrenosum and acne syndrome (PAPA), haploinsufficiency of A20 (HA20), pediatric granulomatous arthritis (PGA), PLCG2-associated antibody deficiency and immune dysregulation (PLAID), autoinflammation and PLCG2-associated antibody deficiency and immune dysregulation (APLAID), and sideroblastic anemia with B-cell immunodeficiency, periodic fevers, and developmental delay (SIFD); autoimmune diseases, comprising multiple sclerosis (MS), type 1 diabetes, psoriasis, rheumatoid arthritis, Behçet's disease, Sjögren's syndrome, and Schnitzler syndrome; macrophage activation syndrome; Blau syndrome; respiratory system diseases, comprising chronic obstructive pulmonary disease (COPD), asthma such as allergic asthma and steroid-resistant asthma, asbestosis, silicosis, and cystic fibrosis; dermatitis, comprising contact dermatitis; central nervous system diseases, comprising Parkinson's disease, Alzheimer's disease, motor neuron disease, Huntington's disease, cerebral malaria and pneumococcal meningitis-induced brain injuries; metabolic diseases, comprising type 2 diabetes, atherosclerosis, obesity, gout, pseudogout; eye diseases, comprising those diseases of ocular epithelium, age-related macular degeneration (AMD), uveitis, corneal infections, and dry eye syndrome; kidney diseases, comprising chronic kidney disease, oxalate nephropathy, nephrocalcinosis, and diabetic kidney disease; liver diseases, comprising nonalcoholic steatohepatitis (NASH) and alcoholic liver disease; skin inflammatory responses, comprising contact hypersensitivity and sunburn; joint inflammatory responses, comprising osteoarthritis, systemic juvenile idiopathic arthritis, adult-onset Still's disease, and relapsing polychondritis; viral infections, comprising alphavirus (Chikungunya virus, Ross River virus) and flavivirus (dengue virus, Zika virus), influenza virus, and HIV; hidradenitis suppurativa (HS) and other skin diseases causing cysts; cancers, comprising lung cancer metastasis, pancreatic cancer, gastric cancer, myelodysplastic syndromes, and leukemia; polymyositis; stroke, comprising ischemic stroke; myocardial infarction, comprising recurrent myocardial infarction; congestive heart failure; embolism; cardiovascular diseases; graft-versus-host disease; hypertension; colitis; helminth infection; bacterial infection; sepsis; septic shock; abdominal aortic aneurysm; wound healing; depression, psychological stress; ischemia-reperfusion injury; and any disease in which an individual has been determined to carry a germline or somatic non-silent mutation in NLRP3.

18. The method according to claim 16 or claim 17, wherein the disease, disorder, or condition is:
(i) an autoinflammatory disease such as cryopyrin associated periodic syndrome (CAPS), Muckle-Wells syndrome (MWS), familial cold autoinflammatory syndrome (FCAS), familial Mediterranean fever (FMF), neonatal-onset multisystem inflammatory disease (NOMID), tumor necrosis factor (TNF) receptor-associated periodic syndrome (TRAPS), hyperimmunoglobulin D with periodic fever syndrome (HIDS), deficiency of interleukin-1-receptor antagonist (DIRA), Majeed syndrome, or pyogenic arthritis, pyoderma gangrenosum and acne (PAPA);
(ii) Parkinson's disease or Huntington's disease;
(iii) gout or juvenile idiopathic arthritis;
(iv) nonalcoholic steatohepatitis (NASH);
(v) oxalate nephropathy or nephrocalcinosis;
(vi) uveitis;
(vii) hidradenitis suppurativa (HS);
(viii) myelodysplastic syndrome, macrophage activation syndrome, Schnitzler syndrome, adult-onset Still's disease, or Behçet's disease; or
(ix) sepsis or septic shock.
